# EUROPÄISCHE PATENTANMELDUNG

(11) **EP 4 447 617 A1**
(43) Veröffentlichungstag der Anmeldung: **16.10.2024**
(21) Anmeldenummer: 24166249.3
(22) Anmeldetag: 26.03.2024
(51) Int. Cl.: H05B 47/13, H05B 47/155, A61B 90/30, F21V 21/14, H05B 47/115, F21V 23/04, F21W 131/205

(54) **BELEUCHTUNGSVORRICHTUNG UND BELEUCHTUNGSVERFAHREN MIT AUTOMATISCHER ABSCHATTUNGS-KOMPENSATION**

(30) Priorität: 05.04.2023 DE 102023108682
(71) Anmelder: Drägerwerk AG & Co. KGaA, 23558 Lübeck (DE)
(72) Erfinder: Kretschmann, Hanno, 23558 Lübeck (DE); Spielberger, Georg, 23558 Lübeck (DE); Tanler, Peter, 23558 Lübeck (DE)

(57) **Zusammenfassung**

Die Erfindung betrifft eine Beleuchtungsvorrichtung (100) und ein Beleuchtungsverfahren, welche eine Oberfläche zu beleuchten vermögen. Ein Steuergerät (10) erfasst eine Beleuchtungsstärken-Vorgabe und steuert die Lichtquellen (a.1, b.1, c.1) der Beleuchtungsvorrichtung (100) so an, dass die auf der Oberfläche erzielte maximale Beleuchtungsstärke gleich der Beleuchtungsstärken-Vorgabe ist. Jede Lichtquelle (a.1, b.1, c.1) lässt sich unabhängig von jeder anderen Lichtquelle ansteuern. Mehrere Abstandsmesser (dm, dm.2, ..., dm.6) messen den jeweiligen Abstand zwischen sich und der beleuchteten Oberfläche. Das Steuergerät (10) detektiert das Ereignis, dass ein Objekt (AO) einen Bereich der Oberfläche abschattet, und bewirkt, dass diese Abschattung wenigstens teilweise kompensiert wird. Hierfür vergrößert das Steuergerät (10) die maximale Beleuchtungsstärke von nicht abgeschatteten Lichtquellen (a.1, b.1, c.1). Bevorzugt wird die maximale Beleuchtungsstärke von solchen Lichtquellen vergrößert, die zu abgeschatteten Lichtquellen redundant oder teil-redundant sind und selber nicht abgeschattet sind.

## Beschreibung

Die Erfindung betrifft eine Beleuchtungsvorrichtung und ein Beleuchtungsverfahren mit einer automatischen Kompensation einer veränderten Helligkeit, wobei die Veränderung der Helligkeit durch eine Abschattung bewirkt wird. Die Abschattung wird durch ein Objekt im Raum zwischen der Beleuchtungsvorrichtung und einer von der Beleuchtungsvorrichtung beleuchteten Oberfläche hervorgerufen.

Eine solche Beleuchtungsvorrichtung wird beispielsweise verwendet, um einen Operationstisch und damit einen Patienten, der auf diesem Operationstisch liegt, zu beleuchten. Möglich ist, dass als Objekt ein Körperteil eines Menschen, der den Patienten behandelt, oder ein verwendetes Instrument zwischen die Beleuchtungsvorrichtung und den Patienten auf dem Operationstisch gerät und dadurch eine Abschattung des beleuchteten Patienten hervorruft. Wird die Abschattung nicht detektiert oder zwar detektiert, aber nicht ausreichend kompensiert, so kann dies zur Folge haben, dass ein Bereich des Patienten nicht mehr ausreichend hell beleuchtet wird.

In der Regel umfasst die Beleuchtungsvorrichtung mehrere Lichtquellen, die auf den Operationstisch gerichtet sind. In aller Regel bewirkt das Objekt zwischen der Beleuchtungsvorrichtung und dem Operationstisch daher keinen vollständigen Schatten ("Schlagschatten"), sondern lediglich, dass einzelne Bereiche der beleuchteten Oberfläche, also des Patienten, weniger stark beleuchtet werden als gewünscht. Auch dies soll unter dem Begriff "Abschattung" verstanden werden.

Der Erfindung liegt die Aufgabe zugrunde, eine Beleuchtungsvorrichtung und ein Beleuchtungsverfahren bereitzustellen, welche eine Abschattung durch einen Körperteil eines Menschen und / oder einen Gegenstand besser automatisch zu kompensieren vermögen als bekannte Beleuchtungsvorrichtungen und Beleuchtungsverfahren.

Die Aufgabe wird durch eine Beleuchtungsvorrichtung mit den Merkmalen des Anspruchs 1 und durch ein Beleuchtungsverfahren mit den Merkmalen des Anspruchs 14 gelöst. Vorteilhafte Ausgestaltungen sind in den Unteransprüchen angegeben. Vorteilhafte Ausgestaltungen der erfindungsgemäßen Beleuchtungsvorrichtung sind, soweit sinnvoll, auch vorteilhafte Ausgestaltungen des erfindungsgemäßen Beleuchtungsverfahrens und umgekehrt.

Die erfindungsgemäße Beleuchtungsvorrichtung und das erfindungsgemäße Beleuchtungsverfahren vermögen eine Oberfläche zu beleuchten. Die Oberfläche ist beispielsweise ein Operationstisch oder auch ein Patient, der auf dem Operationstisch liegt.

Im Folgenden werden zunächst einige Begriffe definiert, die eine Beleuchtungsvorrichtung betreffen und nachfolgend für die Beschreibung der Erfindung verwendet werden.

Die erfindungsgemäße Beleuchtungsvorrichtung sowie viele aus dem Stand der Technik bekannte Beleuchtungsvorrichtungen für medizinische Zwecke umfassen mehrere einzelne Lichtquellen. Nachfolgend wird der Begriff "Beleuchtungseinheit" verwendet. Der Begriff "Beleuchtungseinheit" ist ein Oberbegriff für die gesamte Beleuchtungsvorrichtung sowie für jede einzelne Lichtquelle und für eine Gruppe von Lichtquellen.

Die "Beleuchtungsstärke" (engl. Illuminance", Ev) einer Beleuchtungseinheit beschreibt den Lichtstrom pro Flächeneinheit, wobei der Lichtstrom von der Beleuchtungseinheit ausgeht und auf eine Fläche trifft. Die SI-Einheit ist Lux = Lumen/m^2. In einer Ebene, die senkrecht auf der optischen Mittelachse der Beleuchtungseinheit steht, nimmt die Beleuchtungsstärke der Beleuchtungseinheit typischerweise den maximalen Wert im Schnittpunkt der optischen Mittelachse mit dieser Ebene an. Insbesondere bei einer Beleuchtungseinheit mit mehreren einzelnen Lichtquellen ist es möglich, dass der maximale Wert der Beleuchtungsstärke außerhalb des Schnittpunkts angenommen wird, beispielsweise auf einem Kreis um den Schnittpunkt. Weil die maximale Beleuchtungsstärke in der Regel vom Abstand zwischen der Beleuchtungseinheit und dem beleuchteten Objekt abhängt, wird die maximale Beleuchtungsstärke häufig auf einen vorgegebenen Referenzabstand bezogen. Dieser beträgt in medizinischen Anwendungen häufig 1m.

Zur Unterscheidung wird für die Beleuchtungsstärke, den die Beleuchtungsvorrichtung insgesamt erzielt, der Begriff "Gesamt-Beleuchtungsstärke" verwendet, und für die Beleuchtungsstärke, den eine einzelne Lichtquelle erzeugt, der Begriff "Einzel-Beleuchtungsstärke". Entsprechend werden die Begriffe "maximale Gesamt-Beleuchtungsstärke" der Beleuchtungsvorrichtung und "maximale Einzel-Beleuchtungsstärke" einer einzelnen Lichtquelle verwendet. Die (lokale) Beleuchtungsstärke sowie die maximale Beleuchtungsstärke sind veränderbare Größen, die zu einem Zeitpunkt jeweils einen Wert annehmen. Die maximal mögliche Einzel-Beleuchtungsstärke eine Lichtquelle ist hingegen ein Wert, der durch die Konstruktion der Lichtquelle vorgegeben ist und sich - wenn überhaupt - nur durch Alterung allmählich verringert. Die maximale Einzel-Beleuchtungsstärke liegt zwischen Null und der maximal möglichen Einzel-Beleuchtungsstärke (einschließlich).

Eine Beleuchtungseinheit weist eine optische Mittelachse auf und erzeugt auf einer beleuchteten Oberfläche ein Lichtfeld. Dieses Lichtfeld weist eine Beleuchtungsstärke auf, die über die beleuchtete Oberfläche variiert und in einem Punkt oder einem Bereich der Oberfläche ein Maximum annimmt. Dieser Bereich wird nachfolgend als "Maximal-Bereich" bezeichnet. Der Maximal-Bereich kann ein einzelner Punkt sein oder auch idealisiert ein Kreisring, wobei dieser Kreisring bevorzugt als Mittelpunkt den Schnittpunkt zwischen der optischen Achse und der beleuchteten Oberfläche hat.

Das Lichtfeld einer Beleuchtungseinheit lässt sich durch eine dreidimensionale Darstellung beschreiben, wobei die als Ebene angenommene beleuchtete Oberfläche sich in der x-y-Ebene dieser Darstellung erstreckt und die Beleuchtungsstärke, welche die Beleuchtungseinheit in einem Punkt (x, y) auf der Oberfläche erzielt, auf der z-Achse aufgetragen ist. In vielen Fällen hat das Lichtfeld einer Beleuchtungseinheit idealisiert die Form einer Glockenkurve.

Diese Glockenkurve nimmt ihr Maximum im Schnittpunkt der optischen Mittelachse mit der beleuchteten Oberfläche an.

Falls die beleuchtete Oberfläche eben ist und senkrecht auf der optischen Mittelachse der Beleuchtungseinheit steht, so ist der Bereich auf der Oberfläche, in dem die über den Bereich gemittelte Beleuchtungsstärke x % der maximalen Beleuchtungsstärke beträgt, typischerweise ein Kreis. Als durchschnittliche Beleuchtungsstärke in diesem Bereich mit x % Beleuchtungsstärke wird bevorzugt ein Mittelwert über die jeweilige Beleuchtungsstärke in mehreren Punkten auf diesem Kreis verwendet, insbesondere in mindestens vier gleichmäßig auf dem Kreis verteilten Punkten. Der Standard IEC 60601-2-41 für Operationsleuchten definiert sogar acht gleichmäßig über einen Kreis verteilte Punkte. Der Durchmesser dieses Kreises wird oft auch als Lichtfeld-Durchmesser dₓ bezeichnet und hängt von dem Abstand zwischen der Beleuchtungseinheit und der beleuchteten Oberfläche ab. Daher wird der Lichtfeld-Durchmesser dₓ oft auf den vorgegebenen Referenzabstand zwischen der Beleuchtungseinheit und der beleuchteten Oberfläche bezogen, beispielsweise auf einen Referenzabstand von 1 m. Bei einer medizinischen Beleuchtungseinheit ist häufig x = 10 %, manchmal auch x = 50%, und der Lichtfeld-Durchmesser ist der Durchmesser, bei dem die Beleuchtungsstärke ein Zehntel bzw. die Hälfte der maximalen Beleuchtungsstärke auf der Oberfläche Ob beträgt. Bei einer Beleuchtungseinheit für einen Operationstisch liegt d₁₀ typischerweise zwischen 13 cm und 35 cm. In vielen Ausgestaltungen kann ein Benutzer einen gewünschten Wert für d₁₀ vorgeben.

Der gerade definierte Kreis, der den Lichtfeld-Durchmesser dₓ aufweist, wird nachfolgend auch als "dₓ-Kreis" bezeichnet. Der Mittelpunkt des dₓ-Kreises ist der Schnittpunkt zwischen der optischen Mittelachse und der beleuchteten Oberfläche. Der Bereich auf der Oberfläche, der vom dₓ-Kreis umgeben wird und die Form eines Vollkreises hat, wird nachfolgend als "dₓ-Bereich" bezeichnet.

Die erfindungsgemäße Beleuchtungsvorrichtung und das erfindungsgemäße Beleuchtungsverfahren vermögen eine Oberfläche zu beleuchten. Die Oberfläche ist beispielsweise ein medizinischer Operationstisch sowie die zur Beleuchtungsvorrichtung hin zeigende Oberfläche eines Patienten, der auf dem Operationstisch liegt. Das erfindungsgemäße Beleuchtungsverfahren wird unter Verwendung der erfindungsgemäßen Beleuchtungsvorrichtung durchgeführt.

Die erfindungsgemäße Beleuchtungsvorrichtung weist eine optische Mittelachse auf und umfasst eine Lichtquellen-Menge mit mehreren Lichtquellen. Bevorzugt umfasst die Lichtquellen-Menge mindestens 10 Lichtquellen, besonders bevorzugt mindestens 30 Lichtquellen, insbesondere mindestens 60 Lichtquellen. Möglich ist, dass die Beleuchtungsvorrichtung mindestens eine weitere Lichtquelle umfasst, die nicht zur Lichtquellen-Menge gehört und daher nicht notwendigerweise alle Eigenschaften aufweist, welche die Lichtquellen der Lichtquellen-Menge erfindungsgemäß oder bevorzugt aufweisen und welche nachfolgend beschrieben werden.

Die Lichtquellen der Lichtquellen-Menge lassen sich von außen ansteuern und können alle baugleich sein. Möglich ist auch, dass sich mindestens zwei Lichtquellen der Lichtquellen-Menge voneinander unterscheiden.

Jede Lichtquelle der Lichtquellen-Menge weist jeweils eine optische Lichtachse auf. Die optischen Lichtachsen von zwei verschiedenen Lichtquellen stimmen nicht miteinander überein, denn die beiden Lichtquellen haben einen Abstand zueinander. Jede Lichtquelle vermag auf der beleuchteten Oberfläche jeweils ein Lichtfeld zu erzeugen. Bevorzugt ist das Lichtfeld einer Lichtquelle rotationsymmetrisch zur optischen Lichtachse dieser Lichtquelle. Eine durch die Konstruktion der Lichtquelle vorgegebene Eigenschaft ist die maximal mögliche Einzel-Beleuchtungsstärke des erzeugten Lichtfelds, die sich bevorzugt auf den Referenzabstand bezieht. Möglich ist, dass alle Lichtquellen der Lichtquellen-Menge dieselbe maximal mögliche Einzel-Beleuchtungsstärke aufweisen. Möglich ist auch, dass mindestens zwei Lichtquellen der Lichtquellen-Menge sich hinsichtlich ihrer maximal möglichen Einzel-Beleuchtungsstärken voneinander unterscheiden.

Zu jedem Zeitpunkt nimmt die maximale Einzel-Beleuchtungsstärke einer Lichtquelle einen Wert an, der zwischen Null und der maximal möglichen Einzel-Beleuchtungsstärke dieser Lichtquelle (einschl.) liegt. Der aktuelle Wert der maximalen Einzel-Beleuchtungsstärke einer Lichtquelle der Lichtquellen-Menge lässt sich durch eine Ansteuerung von außen festlegen und verändern, und zwar unabhängig von jeder anderen Lichtquelle der Lichtquellen-Menge und damit auch unabhängig vom jeweiligen aktuellen Wert der maximalen Einzel-Beleuchtungsstärke jeder anderen Lichtquelle.

Wie oben bereits erwähnt, erzielt die Beleuchtungsvorrichtung in einem Maximal-Bereich eine maximale Gesamt-Beleuchtungsstärke. In vielen Fällen ist der Maximal-Bereich rotationssymmetrisch zum Schnittpunkt der optischen Mittelachse der Beleuchtungsvorrichtung mit der beleuchteten Oberfläche. In der Regel wandert der Maximal-Bereich über die beleuchtete Oberfläche, wenn die Beleuchtungsvorrichtung relativ zur beleuchteten Oberfläche seitlich oder schräg verschoben wird. Welche maximale Gesamt-Beleuchtungsstärke die Beleuchtungsvorrichtung auf der Oberfläche erzielt und wo der Maximal-Bereich relativ zur optischen Mittelachse angeordnet ist, hängt einerseits von der jeweiligen maximalen Einzel-Beleuchtungsstärke jeder Lichtquelle und andererseits von der jeweiligen Orientierung der Lichtachse der Lichtquelle relativ zur optischen Mittelachse ab.

Weiterhin umfasst die Beleuchtungsvorrichtung eine Abstandsmess-Anordnung. Die Abstandsmess-Anordnung umfasst mehrere, d.h. mindestens zwei, Abstandsmesser, die voneinander beabstandet sind. Jeder Abstandsmesser der Abstandsmess-Anordnung weist jeweils eine Messrichtung auf, die zur beleuchteten Oberfläche hin zeigt. Jeder Abstandsmesser vermag jeweils ein Maß für den Abstand in der Messrichtung zwischen sich selbst und einem Licht streuenden Objekt zu messen. Dieses Licht streuende Objekt kann die beleuchtete Oberfläche oder ein Objekt auf der Oberfläche sein oder auch ein abschattendes Objekt, also ein Objekt, welches in den Bereich zwischen der Beleuchtungsvorrichtung und der beleuchteten Oberfläche gerät und einen Abstand zur beleuchteten Oberfläche aufweist.

Die Beleuchtungsvorrichtung umfasst weiterhin ein signalverarbeitendes Steuergerät (control unit). Das Steuergerät kann in einem Lichtquellen-Träger der Beleuchtungsvorrichtung integriert sein oder räumlich von einem solchen Träger getrennt sein. Zwischen dem Steuergerät und jeder Lichtquelle der Lichtquellen-Menge besteht dauerhaft oder jeweils wenigstens zeitweise eine Datenverbindung vom Steuergerät zur Lichtquelle, die per Kabel und / oder per elektromagnetischer Funkwellen realisiert sein kann. Dauerhaft oder wenigstens zeitweise führt eine weitere Datenverbindung von der Abstandsmess-Anordnung zum Steuergerät.

Dieses Steuergerät vermag ein Signal der Abstandsmess-Anordnung sowie Vorgaben zu empfangen und automatisch zu verarbeiten. Eine solche Vorgabe kann von einem Benutzer kommen oder von einer übergeordneten Steuerung oder Regelung. Erfindungsgemäß vermag das Steuergerät als eine Vorgabe eine Beleuchtungsstärken-Vorgabe zu erfassen. Diese Beleuchtungsstärken-Vorgabe gibt einen Sollwert für die maximale Gesamt-Beleuchtungsstärke vor, wobei die Beleuchtungsvorrichtung eine maximale Gesamt-Beleuchtungsstärke mit diesem Wert erzielen soll. Bevorzugt bezieht sich die Beleuchtungsstärken-Vorgabe auf den Referenzabstand.

Abhängig von dieser Verarbeitung vermag das Steuergerät die Lichtquellen der Lichtquellen-Menge anzusteuern, und zwar jede Lichtquelle unabhängig von jeder anderen Lichtquelle. Außerdem vermag das Steuergerät für jede Lichtquelle der Lichtquellen-Menge jeweils vorgegebene Informationen zu erfassen. Diese Informationen können in einem Datenspeicher abgespeichert sein, auf den das Steuergerät wenigstens zeitweise Lesezugriff aufweist. Diese Informationen können auch als Parameter eines abgespeicherten Programms vorgegeben sein, wobei das Steuergerät bei der Beleuchtung der Oberfläche dieses Programm ausführt. In der Regel ist dieses Programm in einem Datenspeicher des Steuergeräts abgespeichert und läuft auf einem Prozessor des Steuergeräts ab. Möglich ist auch, dass ein Teil der Informationen im Datenspeicher abgespeichert ist und ein anderer Teil als Parameter des Programms vorgegeben ist.

Die Informationen über eine Lichtquelle der Lichtquellen-Menge umfassen einerseits ein Maß dafür, welche maximale Einzel-Beleuchtungsstärke diese Lichtquelle zu erzeugen vermag, also eine durch die Konstruktion vorgegebene maximal mögliche Einzel-Beleuchtungsstärke. Bevorzugt bezieht sich diese maximal mögliche Einzel-Beleuchtungsstärke auf den oben erwähnten Referenzabstand. Das Maß ist beispielsweise die maximale elektrische Spannung, die an der Lichtquelle anliegen kann, oder die maximale elektrische Stromstärke oder maximale elektrische Leistung für die Lichtquelle.

Andererseits umfassen die Informationen für jede Lichtquelle der Lichtquellen-Menge jeweils eine Information, wie die optische Lichtachse dieser Lichtquelle relativ zur optischen Mittelachse der Beleuchtungsvorrichtung positioniert und orientiert ist. Die Lichtachse einer Lichtquelle kann mit der optischen Mittelachse der Beleuchtungsvorrichtung übereinstimmen oder mit einem Abstand parallel zur optischen Mittelachse sein oder die optische Mittelachse schneiden oder auch windschief zur optischen Mittelachse angeordnet sein. Zwei Geraden im Raum sind windschief zueinander, wenn sie weder übereinstimmen noch zueinander parallel sind noch sich schneiden. Die vom Steuergerät erfassten Informationen legen insbesondere fest, wie die Lichtachsen der Lichtquellen relativ zueinander positioniert sind.

Das Steuergerät vermag jede Lichtquelle der Lichtquellen-Menge einzeln anzusteuern, also unabhängig von jeder anderen Lichtquelle der Lichtquellen-Menge. Durch eine entsprechende Ansteuerung vermag das Steuergerät die jeweilige maximale Einzel-Beleuchtungsstärke einer Lichtquelle auf einen Wert einzustellen, der zwischen Null und der maximal möglichen Einzel-Beleuchtungsstärke dieser Lichtquelle (einschl.) liegt. In einer Realisierungsform vermag das Steuergerät die erzielte maximale Beleuchtungsstärke stufenlos zu verändern, in einer anderen Regierungsformen gestuft. Dank dieser Ansteuerung lässt sich die jeweilige maximale Einzel-Beleuchtungsstärke einer Lichtquelle der Lichtquellen-Menge unabhängig von jeder anderen Lichtquelle verändern. Anmerkung: Natürlich lässt sich in der Regel die maximale Einzel-Beleuchtungsstärke einer Lichtquelle nicht exakt auf einen gewünschten Wert festlegen, sondern nur mit einem gewissen Fehler.

Anmerkung: Möglich ist, dass mindestens zwei einzelne Lichtquellen der Beleuchtungsvorrichtung zu einem Lichtquellen-Modul zusammengefasst sind und sich die einzelnen Lichtquellen eines Lichtquellen-Moduls nur gemeinsam ansteuern lassen. In diesem Falle wird unter dem Begriff "Lichtquelle der Lichtquellen-Menge" ein solches Lichtquellen-Modul mit mindestens zwei einzelnen Lichtquellen verstanden. Die erfindungsgemäße Beleuchtungsvorrichtung umfasst in diesem Fall mindestens zwei solcher Lichtquellen-Module. Das Lichtquellen-Modul ist beispielsweise eine Baugruppe mit mindestens zwei einzelnen Lichtquellen, wobei diese Baugruppe sich separat vom Rest der Beleuchtungsvorrichtung austauschen lässt.

Erfindungsgemäß vermag das Steuergerät ein Signal der Abstandsmess-Anordnung zu empfangen und zu verarbeiten. Durch diese Verarbeitung vermag das Steuergerät automatisch zu prüfen, ob ein Abschattungs-Ereignis aufgetreten ist und / oder aktuell vorliegt, und insbesondere ein Abschattungs-Ereignis zu detektieren. Unter einem "Abschattungs-Ereignis" wird folgendes Ereignis verstanden: Mindestens ein Objekt befindet sich aktuell zwischen der Beleuchtungsvorrichtung - genauer gesagt: zwischen mindestens einer Lichtquelle der Lichtquellen-Menge - und der beleuchteten Oberfläche. Indem das Steuergerät das Signal der Abstandsmess-Anordnung verarbeitet, vermag das Steuergerät ein abschattendes Objekt mindestens dann zu detektieren, wenn dieses Objekt den jeweiligen Abstand verändert, den der oder mindestens ein Abstandsmesser der Abstandsmess-Anordnung misst. In aller Regel bewirkt das Objekt, dass mindestens eine Lichtquelle der Lichtquellen-Menge aktuell vollständig oder wenigstens teilweise abgeschattet ist und mindestens einen gemessenen Abstand verändert. Daher wird im Folgenden auch von "dem abschattenden Objekt" gesprochen. Mit diesem Begriff können auch mehrere Objekte gemeint sein, beispielsweise ein Körperteil eines behandelnden Arztes und ein vom Arzt verwendetes Instrument. In der Regel variiert die Position des abschattenden Objekts relativ zur Beleuchtungsvorrichtung, und damit variiert die Abschattung über die Zeit.

Ohne eine Gegenmaßnahme verändert die Abschattung durch das abschattende Objekt das Lichtfeld, welches die Beleuchtungsvorrichtung auf der beleuchteten Oberfläche erzielt. Unter einer "Abschattungs-Kompensation" wird eine Maßnahme verstanden, die das Steuergerät automatisch mit dem Ziel auslöst, die Abschattung wenigstens teilweise zu kompensieren. Idealerweise bleibt das Lichtfeld, das die Beleuchtungsvorrichtung auf der beleuchteten Oberfläche erzielt, dank der Abschattungs-Kompensation unverändert. In der Praxis lässt sich dieses Ziel meistens nur annähernd erreichen.

Erfindungsgemäß wird dieses Ziel wie folgt realisiert: Das oberste Ziel bei der Abschattungs-Kompensation ist, dass sowohl die maximale Gesamt-Beleuchtungsstärke, die die Beleuchtungsvorrichtung erzielt, als auch die Position des Maximal-Bereichs relativ zur optischen Mittelachse der Beleuchtungsvorrichtung unverändert bleiben und daher trotz der Abschattung die erzielte maximale Gesamt-Beleuchtungsstärke gleich einer Beleuchtungsstärken-Vorgabe ist. Der Maximal-Bereich soll seine Position relativ zur optischen Mittelachse beibehalten. Mit anderen Worten: Die Position des Maximal-Bereichs relativ zur optischen Mittelachse soll durch das Abschattungs-Ereignis nicht verändert werden. Im Falle einer medizinischen Beleuchtungsvorrichtung befindet sich nämlich oft um diesen Maximal-Bereich herum ein Bereich des Körpers eines Patienten, an dem eine Operation stattfindet.

Ein optionales weiteres Ziel ist, dass zusätzlich der Gesamt-Lichtfeld-Durchmesser, den die Beleuchtungsvorrichtung erzielt, unverändert bleibt. Möglich ist auch, dass der dₓ-Kreis seine Position relativ zur optischen Mittelachse nicht ändern soll. Ein optionales drittes Ziel ist, dass auch die korrelierte Gesamt-Farbtemperatur der Beleuchtungsvorrichtung unverändert bleibt. Abhängig vom Ausmaß der Abschattung lassen sich diese Ziele vollständig oder nur teilweise erreichen. Möglich ist, dass das Steuergerät diese drei Ziele mit absteigender Priorität automatisch zu erreichen versucht. Möglich ist auch, dass das Steuergerät einen Kompromiss zwischen mindestens zwei dieser Ziele bewirkt.

Vereinfacht gesagt umfasst die oder jede Maßnahme zur Abschattungs-Kompensation den Schritt, dass die maximale Einzel-Beleuchtungsstärke mindestens einer nicht abgeschatteten Lichtquelle vergrößert wird. Zwar prinzipiell möglich, aber dank der Erfindung nicht erforderlich ist, dass sich der Lichtfeld-Durchmesser und / oder die korrelierte Farbtemperatur einer einzelnen Lichtquelle verändern lassen. Die Erfindung erfordert in der Regel auch nicht, dass ein Stellantrieb eine Lichtquelle relativ zu einem Träger der Beleuchtungsvorrichtung und damit relativ zu einer anderen Lichtquelle zu bewegen vermag. Vielmehr können alle Lichtquellen starr im Träger montiert sein und sich nur zusammen mit dem Träger bewegen lassen. Der Verzicht auf einen Stellantrieb spart Platz im Träger ein und erspart eine Ansteuerung eines Stellantriebs.

Das Steuergerät ist weiterhin wie folgt ausgestaltet: Wenn ein Abschattungs-Ereignis, also ein abschattendes Objekt, detektiert ist, so ermittelt das Steuergerät wenigstens näherungsweise einen Bereich auf der beleuchteten Oberfläche, der durch das Objekt zwischen der Beleuchtungsvorrichtung und der beleuchteten Oberfläche vollständig oder wenigstens teilweise abgeschattet ist. In der Regel erzeugt ein abschattendes Objekt keinen Schlagschatten, weil die Beleuchtungsvorrichtung mehrere Lichtquellen umfasst. "Wenigstens teilweise abgeschattet" bedeutet: In diesem Bereich weicht die Beleuchtungsstärke nach der Abschattung von der Beleuchtungsstärke vor der Abschattung um mehr als eine vorgegebene Schranke nach unten ab. beispielsweise wird durch die Abschattung die Beleuchtungsstärke um mindestens 30% oder sogar um mindestens 50% reduziert.

Um den abgeschatteten Bereich zu ermitteln, verwendet das Steuergerät für jede Lichtquelle die vorgegebene und erfasste Information, wie die Lichtachse dieser Lichtquelle relativ zur optischen Mittelachse der Beleuchtungsvorrichtung orientiert und / oder positioniert ist. Außerdem verwendet das Steuergerät das Signal der Abstandsmess-Anordnung. Bevorzugt verwendet das Steuergerät bei der Ermittlung des Bereichs die beiden vereinfachenden Annahmen, dass die beleuchtete Oberfläche senkrecht auf der optischen Mittelachse der Beleuchtungsvorrichtung steht und dass der Abstand zur Beleuchtungsvorrichtung gleich dem Referenzabstand ist. In vielen Fällen sind die Abweichungen zwischen der Realität und diesen beiden Annahmen praktisch unerheblich.

Bevorzugt ermittelt das Steuergerät die Kontur des abschattenden Objekts in einer Ebene, die senkrecht auf der optischen Mittelachse der Beleuchtungsvorrichtung steht. Um den Abschattungs-Bereich zu ermitteln, verwendet das Steuergerät die ermittelte Kontur sowie den Abstand entlang der optischen Mittelachse zwischen der Beleuchtungsvorrichtung und der beleuchteten Oberfläche sowie den Abstand zwischen der Beleuchtungsvorrichtung und dem abschattenden Objekt und / oder zwischen dem abschattenden Objekt und der beleuchteten Oberfläche.

Das Steuergerät sucht nach mindestens einer Lichtquelle der Lichtquellen-Menge, die zur Abschattungs-Kompensation geeignet ist - genauer gesagt: aktuell dafür geeignet ist, die detektierte Abschattung wenigstens teilweise zu kompensieren, in der Regel zusammen mit mindestens einer anderen Lichtquelle der Lichtquellen-Menge. Für diese Suche verwendet das Steuergerät eine Information über den ermittelten abgeschatteten Bereich der beleuchteten Oberfläche. Bevorzugt ermittelt das Steuergerät mindestens eine Lichtquelle der Lichtquellen-Menge mit folgender Eigenschaft: Die jeweilige Lichtachse der oder jeder ermittelten Lichtquelle schneidet die Oberfläche im abgeschatteten Bereich.

Eine Lichtquelle ist gemäß der Erfindung zur Abschattungs-Kompensation geeignet, wenn folgende Bedingungen kumulativ eingetreten sind, sind, wobei sich automatisch feststellen lässt, ob diese Bedingungen erfüllt sind oder nicht:
- Die Lichtquelle ist aktuell überhaupt nicht oder wenigstens nicht vollständig abgeschattet.
- Die Lichtachse der Lichtquelle schneidet die beleuchtete Oberfläche im ermittelten Abschattungs-Bereich.
- Die Lichtquelle wird aktuell mit einem Wert für ihre maximale Einzel-Beleuchtungsstärke betrieben, der kleiner ist als die maximal mögliche Einzel-Beleuchtungsstärke dieser Lichtquelle. Die Lichtquelle kann aktuell auch ausgeschaltet sein. Daher lässt sich der aktuelle Wert der maximalen Einzel-Beleuchtungsstärke dieser Lichtquelle vergrößern.

Weiter oben wurde eine Ausgestaltung beschrieben, bei welcher das Steuergerät die Kontur des abschattenden Objekts in einer Ebene, die senkrecht auf der optischen Mittelachse steht, ermittelt. In einer Ausgestaltung ist die Lichtquelle dann nicht oder wenigstens nicht vollständig abgeschattet, wenn die Lichtachse der Lichtquelle nicht durch die ermittelte Kontur verläuft, sondern einen Abstand zur Kontur aufweist.

Das Steuergerät verwendet das Signal der Abstandsmess-Anordnung sowie die oben beschriebenen erfassten Informationen über die Lichtquellen, um nach einer solchen geeigneten Lichtquelle zu suchen. In der Regel hängt es von mindestens einer Abmessung des abschattenden Objekts sowie der Position des Objekts relativ zur Beleuchtungsvorrichtung und / oder zur beleuchteten Oberfläche ab, welche Lichtquellen aktuell abgeschattet sind und welche nicht. Insbesondere hängt es von der Kontur des abschattenden Objekts in einer Ebene, die senkrecht auf der optischen Mittelachse steht, sowie vom Abstand zwischen dem abschattenden Objekt und der beleuchteten Oberfläche ab, welche Lichtquellen geeignet sind und welche nicht.

Möglich ist, dass das Steuergerät keine geeignete Lichtquelle findet. Dies ist insbesondere dann der Fall, wenn alle nicht abgeschatteten Lichtquelle bereits mit der maximal möglichen Einzel-Beleuchtungsstärke betrieben werden.

Das Steuergerät ist weiterhin wie folgt ausgestaltet: Falls es mindestens eine geeignete Lichtquelle gefunden hat, so ermittelt es eine Teilmenge der Lichtquellen-Menge, wobei diese Teilmenge mindestens eine Lichtquelle umfasst und nur aus aktuell zur Abschattungs-Kompensation geeigneten Lichtquellen besteht. Die Teilmenge kann aus allen aktuell geeigneten Lichtquellen oder aus einem Teil der geeigneten Lichtquellen bestehen. Sie kann auch nur aus einer einzigen geeigneten Lichtquelle bestehen, auch wenn es mehrere geeignete Lichtquellen gibt. Vorteilhafte Ausgestaltungen spezifizieren unterschiedliche Realisierungsformen, wie diese Teilmenge ermittelt werden kann.

Das Steuergerät vermag zu bewirken, dass die jeweilige maximale Einzel-Beleuchtungsstärke der oder jeder Lichtquelle der ermittelten Teilmenge vergrößert wird. Zu diesem Zweck vermag das Steuergerät für jede Lichtquelle der ermittelten Teilmenge jeweils einen Sollwert zu berechnen oder auf andere Weise festzulegen. Dieser Sollwert legt die zu erzielende maximale Einzel-Beleuchtungsstärke dieser Lichtquelle fest. Der berechnete Sollwert für eine geeignete Lichtquelle ist größer als der aktuelle Wert der maximalen Einzel-Beleuchtungsstärke und ist in einer Ausgestaltung gleich der vorgegebenen maximal möglichen Einzel-Beleuchtungsstärke dieser Lichtquelle, in einer anderen Ausgestaltung kleiner als die maximal mögliche Einzel-Beleuchtungsstärke. Die maximal mögliche Einzel-Beleuchtungsstärke erfasst das Steuergerät aus den oben beschriebenen Informationen über die Lichtquellen.

Um die Teilmenge zu ermitteln und den jeweiligen Sollwert für die maximale Einzel-Beleuchtungsstärke zu berechnen, verwendet das Steuergerät die erfassten Informationen über die Lichtquellen der Lichtquellen-Menge und optional das Signal der Abstandsmess-Anordnung. Das Steuergerät führt die Berechnung der Sollwerte für die Lichtquellen der Teilmenge mit dem Ziel durch, dass der tatsächliche Wert der maximalen Gesamt-Beleuchtungsstärke, den die Beleuchtungsvorrichtung aktuell erzielt, gleich der erfassten Beleuchtungsstärken-Vorgabe ist.

Das Steuergerät steuert die oder jede Lichtquelle der ermittelten Teilmenge mit dem Ziel an, dass der tatsächliche Wert der maximalen Einzel-Beleuchtungsstärke, den die Lichtquelle aktuell erzielt, gleich den berechneten Sollwert ist.

Das Ziel ist also, die Lichtquellen der ermittelten Teilmenge so anzusteuern, dass die ermittelte Teilmenge die Abschattung vollständig oder wenigstens teilweise kompensieren kann. Erfindungsgemäß bewirkt das Steuergerät, dass der Wert der maximalen Einzel-Beleuchtungsstärke mindestens einer Lichtquelle, bevorzugt jeder Lichtquelle der ermittelten Teilmenge vergrößert wird. Natürlich ist es möglich, dass die Abschattung nur teilweise kompensiert werden kann, obwohl mindestens eine geeignete Lichtquelle gefunden wurde. Zwei mögliche Gründe hierfür sind, dass nicht genügend geeignete Lichtquellen gefunden werden oder die noch erzielbare Vergrößerung der maximalen Einzel-Beleuchtungsstärken der gefundenen geeigneten Lichtquellen nicht ausreicht.

Das erfindungsgemäße Verfahren wird unter Verwendung einer erfindungsgemäßen Beleuchtungsvorrichtung durchgeführt und umfasst die folgenden Schritte:
- Das Steuergerät erfasst für jede Lichtquelle der Lichtquellen-Menge die jeweils vorgegebenen Informationen über diese Lichtquelle.
- Das Steuergerät erfasst eine Beleuchtungsstärken-Vorgabe.
- Das Steuergerät stellt die jeweilige aktuelle maximalen Einzel-Beleuchtungsstärke jeder Lichtquelle der Lichtquellen-Menge auf einen Wert ein, und zwar mindestens einmal und bei Bedarf erneut.
- Die Beleuchtungsvorrichtung beleuchtet die Oberfläche und erzielt eine maximale Gesamt-Beleuchtungsstärke .
- Der oder jeder Abstandsmesser der Abstandsmess-Anordnung misst ein Maß für den jeweiligen Abstand zwischen sich selbst und einem Licht streuenden Objekt. Der Abstandsmesser misst das Maß mindestens einmal, bevorzugt mehrmals, besonders bevorzugt mit einer festen Abtastfrequenz.
- Das Steuergerät prüft, ob ein Abschattungs-Ereignis aufgetreten ist. Für diese Prüfung verwendet das Steuergerät ein Signal der Abstandsmess-Anordnung. Diese Prüfung führt das Steuergerät mindestens einmal durch, bevorzugt wiederholt, besonders bevorzugt mit einer festen Abtastfrequenz. Die Abtastfrequenz des Steuergeräts kann die gleiche sein wie die Abtastfrequenz der Abstandsmess-Anordnung oder auch kleiner oder grösser sein.

Die Detektion eines Abschattungs-Ereignisses löst mindestens die folgenden weiteren Schritte aus:
Das Steuergerät sucht nach mindestens einer Lichtquelle der Lichtquellen-Menge, die aktuell zur Abschattungs-Kompensation geeignet ist.

Falls mindestens eine zur Abschattungs-Kompensation geeignete Lichtquelle gefunden ist, so führt das Steuergerät die folgenden Schritte durch:
- Das Steuergerät ermittelt eine Teilmenge mit mindestens einer geeigneten Lichtquelle. Die Teilmenge kann auch aus allen geeigneten Lichtquellen bestehen.
- Für jede Lichtquelle der ausgewählten Teilmenge berechnet das Steuergerät jeweils einen Sollwert. Dieser Sollwert legt fest, welche maximale Einzel-Beleuchtungsstärke diese Lichtquelle erzeugen soll. Der berechnete Sollwert ist größer als der aktuelle Wert der maximalen Einzel-Beleuchtungsstärke und ist kleiner oder gleich der maximal möglichen Einzel-Beleuchtungsstärke.
- Das Steuergerät steuert die oder eine, bevorzugt jede Lichtquelle der ermittelten Teilmenge an und bewirkt durch die Ansteuerung, dass die angesteuerte Lichtquelle den berechneten Sollwert für die maximale Einzel-Beleuchtungsstärke tatsächlich erzielt.
- Das Steuergerät führt die Berechnung der Sollwerte für die Lichtquellen der Teilmenge mit dem Ziel durch, dass der Maximal-Bereich seine Position relativ zur optischen Mittelachse beibehält und die Gesamt-Beleuchtungsstärke einen Wert annimmt, der gleich der erfassten Beleuchtungsstärken-Vorgabe ist.

Die Erfindung ermöglicht es in vielen Fällen, eine Abschattung relativ gut zu kompensieren. Insbesondere wird in vielen Fällen bewirkt, dass die maximale Gesamt-Beleuchtungsstärke relativ wenig verkleinert wird und daher die Oberfläche trotz der Abschattung noch ausreichend beleuchtet wird. Insbesondere bleibt häufig der Maximal-Bereich ausreichend stark beleuchtet. Oft wird sogar bewirkt, dass das Lichtfeld, welches die Beleuchtungsvorrichtung auf der beleuchteten Oberfläche erzielt, sich trotz der Abschattung dank der erfindungsgemäßen Kompensation nur relativ wenig verändert.

Die Erfindung erfordert nicht, dass die Beleuchtungsvorrichtung einen Stellantrieb umfasst, wobei dieser Stellantrieb mindestens eine Lichtquelle relativ zu mindestens einer anderen Lichtquelle zu verschwenken oder anderweitig zu bewegen vermag.

Die Erfindung lässt sich für eine Beleuchtungsvorrichtung anwenden, deren Lichtquellen unterschiedliches Licht ausstrahlen, beispielsweise Licht mit unterschiedlichen korrelierten Farbtemperaturen und / oder unterschiedlichen Amplituden, um anhand dieser Unterschiede eine Abschattung zu detektieren. Die Erfindung erfordert aber nicht eine Beleuchtungsvorrichtung mit derartigen Lichtquellen. Alle Lichtquellen der Beleuchtungsvorrichtung können auch Licht mit gleicher Amplitude ausstrahlen.

Erfindungsgemäß ermittelt das Steuergerät den Abschattungs-Bereich, ermittelt also, welcher Bereich auf der beleuchteten Oberfläche durch das Objekt abgeschattet wird. Um diese Abschattung wenigstens näherungsweise zu kompensieren, ermittelt das Steuergerät mindestens eine Lichtquelle, deren Lichtachse die beleuchtete Oberfläche im Abschattungs-Bereich schneidet. Die maximale Einzel-Beleuchtungsstärke einer solchen Lichtquelle wird vergrößert. Dieses Merkmal führt in vielen Fällen dazu, dass die Abschattung besser kompensiert wird als bei bekannten Verfahren. Dieser Effekt wird insbesondere deshalb erzielt, weil der Abschattungs-Bereich auf der Oberfläche ermittelt wird und nicht oder nicht nur die Größe des abschattenden Objekts. Insbesondere wird oft die Abschattung auch dann relativ gut kompensiert, wenn nicht alle Lichtquellen konzentrisch um die optische Mittelachse herum angeordnet sind. Außerdem wird die Abschätzung durch die Erfindung besser kompensiert, als wenn lediglich ermittelt werden würde, welche Lichtquellen aktuell nicht abgeschattet sind. Möglich ist nämlich, dass eine Lichtquelle zwar nicht abgeschattet ist, ihre Lichtachse aber die beleuchtete Oberfläche außerhalb des Abschattungs-Bereichs schneidet. In vielen Fällen wäre es nicht sinnvoll, die maximale Einzel-Beleuchtungsstärke dieser Lichtquelle zu vergrößern.

Bevorzugt erfasst das Steuergerät zusätzlich die Information, wie die jeweilige Messrichtung jedes Abstandsmessers relativ zur optischen Mittelachse der Beleuchtungsvorrichtung angeordnet ist. Bevorzugt vermag das Steuergerät automatisch festzustellen, ob und wenn ja welche Lichtquellen der Lichtquellen-Menge aktuell abgeschattet sind. In einer bevorzugten Ausgestaltung hat das Steuergerät die Abschattung einer Lichtquelle dann detektiert, wenn detektiert ist, dass sich ein Licht streuendes Objekt in demjenigen Abschnitt der Lichtachse dieser Lichtquelle befindet, der von der Lichtquelle zur beleuchteten Oberfläche reicht. In einer Ausgestaltung vermag das Steuergerät festzustellen, welche Abstandsmesser der Abstandsmess-Anordnung jeweils einen Abstand detektiert haben, der signifikant kleiner ist als der Abstand zwischen dem Abstandsmesser und der beleuchteten Oberfläche, beispielsweise außerhalb eines vorgegebenen Toleranzbands liegt. Natürlich ist es möglich, dass es nur einen solchen Abstandsmesser gibt.

Bevorzugt vermag das Steuergerät wiederholt, besonders bevorzugt mit einer vorgegebenen Abtastfrequenz, zu prüfen, ob aktuell ein abschattendes Ereignis vorliegt oder nicht. Das Steuergerät ist bevorzugt so ausgestaltet, die gerade beschriebenen Maßnahmen zur Abschattungs-Kompensation mit dieser Abtastfrequenz durchzuführen. Durch eine wiederholte Durchführung wird in vielen Fällen die Abschattung rasch detektiert und wenigstens teilweise kompensiert. Die Abtastfrequenz wird in der Regel durch die Abtastfrequenz, die die Abstandsmess-Anordnung zu erzielen vermag, und / oder durch die Rechenkapazität des Steuergeräts begrenzt. In manchen Fällen kann auch die Zeitspanne, die benötigt wird, um die maximale Einzel-Beleuchtungsstärke einer Lichtquelle zu verändern, ein limitierender Faktor sein.

Erfindungsgemäß vermag das Steuergerät eine Beleuchtungsstärken-Vorgabe zu erfassen. In einer Ausgestaltung vermag das Steuergerät zusätzlich eine Lichtfeld-Durchmesser-Vorgabe zu erfassen. Auch diese Lichtfeld-Durchmesser-Vorgabe kann von einem Benutzer oder von einer übergeordneten Steuerung oder Regelung stammen. Die Lichtfeld-Durchmesser-Vorgabe gibt einen Sollwert für den Gesamt-Lichtfeld-Durchmesser vor, das ist der Lichtfeld-Durchmesser, den das Lichtfeld aufweisen soll, welches die Beleuchtungsvorrichtung insgesamt erzeugt. Auch dieser Gesamt-Lichtfeld-Durchmesser bezieht sich bevorzugt auf den oben erwähnten Referenzabstand erwähnten Referenzabstand und auf eine beleuchtete Oberfläche, die senkrecht auf der optischen Mittelachse der Beleuchtungsvorrichtung steht.

Erfindungsgemäß ermittelt das Steuergerät eine Teilmenge von geeigneten Lichtquellen und berechnet für jede Lichtquelle der ermittelten Teilmenge jeweils einen Sollwert für die maximale Einzel-Beleuchtungsstärke. Ein Ziel bei der Berechnung der Sollwerte ist erfindungsgemäß, dass die Gesamt-Beleuchtungsstärke mit der Beleuchtungsstärken-Vorgabe übereinstimmt. Gemäß einer weiter oben beschriebenen Ausgestaltung ist ein weiteres (ein zusätzliches) Ziel bei der Berechnung der Sollwerte, dass der Wert des Gesamt-Lichtfeld-Durchmessers mit der Lichtfeld-Durchmesser-Vorgabe übereinstimmt. Dank dieser Ausgestaltung wird die Abschattung also noch besser kompensiert. In vielen Fällen wird erreicht, dass das Lichtfeld, welches die Beleuchtungsvorrichtung nach der Kompensation der Abschattung auf der beleuchteten Oberfläche erzielt, relativ wenig vom Lichtfeld, welches die Beleuchtungsvorrichtung vor der Abschattung erzielt, abweicht, idealerweise nicht in einer von einem Menschen wahrnehmbaren Weise abweicht. Auch der Gesamt-Lichtfeld-Durchmesser ändert sich in vielen Fällen trotz der Abschattung relativ wenig, weil die gerade beschriebene Ausgestaltung der Abschattungs-Kompensation angewendet wird. Selbstverständlich ist es möglich, dass auch das optionale weitere Ziel nicht vollständig erreicht werden kann.

Die weiter oben beschriebene Ausgestaltung mit dem Gesamt-Lichtfeld-Durchmesser lässt sich mit der bereits beschriebenen Ausgestaltung kombinieren, dass Lichtquellen ausgeschaltet werden, die vollständig oder wenigstens teilweise abgeschattet sind.

Erfindungsgemäß erfasst das Steuergerät eine Beleuchtungsstärken-Vorgabe und optional eine Lichtfeld-Durchmesser-Vorgabe. Das Steuergerät detektiert ein Abschattungs-Ereignis. In einer Ausgestaltung prüft das Steuergerät, ob der Maximal-Bereich und / oder der dₓ-Bereich im Abschattungs-Bereich liegt oder nicht. Falls weder der Maximal-Bereich noch der dₓ-Bereich innerhalb des Kreises im Abschattungs-Bereich liegen, so ist es in manchen Fällen nicht erforderlich, das Abschattungs-Ereignis zu kompensieren.

Das Steuergerät ist bevorzugt dazu ausgestaltet, mindestens einmal den Gesamt-Lichtfeld-Durchmesser rechnerisch vorherzusagen. Das erzeugte Gesamt-Lichtfeld und damit der Gesamt-Lichtfeld-Durchmesser hängen einerseits von dem jeweiligen aktuellen Wert der maximalen Einzel-Beleuchtungsstärke jeder nicht ausgeschalteten Lichtquelle ab und andererseits von der jeweiligen Position der Lichtachse der Lichtquelle relativ zur optischen Mittelachse der Beleuchtungsvorrichtung. Bevorzugt verwendet das Steuergerät einen vorgegebenen Standardwert für den jeweiligen Einzel-Lichtfeld-Durchmesser einer Lichtquelle. Bevorzugt bezieht der Gesamt-Lichtfeld-Durchmesser sich wiederum auf den Referenzabstand und auf eine Oberfläche, die im Referenzabstand senkrecht auf der optischen Mittelachse steht. Das Steuergerät berechnet die Sollwerte für die maximalen Einzel-Beleuchtungsstärken so, dass der vorhergesagte Gesamt-Lichtfeld-Durchmesser der Lichtfeld-Durchmesser-Vorgabe möglichst nahe kommt, beispielsweise um nicht mehr als eine vorgegebene Toleranz abweicht.

Erfindungsgemäß sucht das Steuergerät nach mindestens einer Lichtquelle, die aktuell zur Abschattungs-Kompensation geeignet ist. Möglich ist, dass bei dieser Suche auch Lichtquellen einbezogen werden, die teilweise abgeschattet sind, aber nicht vollständig. Gemäß einer Ausgestaltung ist das Steuergerät hingegen so ausgestaltet, dass es bei dieser Suche nur unter denjenigen Lichtquellen sucht, die überhaupt nicht abgeschattet sind. Bei der Suche werden also teilweise abgeschattete Lichtquellen nicht berücksichtigt. Diese Ausgestaltung spart in vielen Fällen Rechenzeit und / oder Rechenkapazität ein.

Bevorzugt bewirkt das Steuergerät, dass jede Lichtquelle, die vollständig oder wenigstens teilweise abgeschattet ist, ausgeschaltet wird. Diese Ausgestaltung senkt in vielen Fällen den Energieverbrauch. Außerdem wird in vielen Fällen die Erwärmung des abschattenden Objekts reduziert, wenn Lichtquellen, die vollständig oder wenigstens teilweise abgeschattet sind, ausgeschaltet werden. Eine solche Erwärmung ist insbesondere dann häufig unerwünscht, wenn das abschattende Objekt ein Körperteil eines behandelnden Arztes ist. Das abschattende Objekt weist einen geringeren Abstand zur Beleuchtungsvorrichtung auf und ist daher häufig einem stärkeren Eintrag von Wärmeenergie pro Flächeneinheit ausgesetzt als die beleuchtete Oberfläche. Bevorzugt überprüft das Steuergerät mindestens einmal, bevorzugt wiederholt, ob diese Lichtquelle immer noch abgeschattet ist. Wenn die Lichtquelle nicht mehr abgeschaltet ist, ist es möglich, die Lichtquelle wieder einzuschalten.

In der Regel weist jede Lichtquelle eine korrelierte Farbtemperatur auf. Typischerweise ist diese korrelierte Farbtemperatur durch die Konstruktion der Lichtquelle vorgegeben und lässt sich nicht verändern. Möglich ist, dass alle Lichtquellen der Lichtquellen-Menge dieselbe korrelierte Farbtemperatur aufweisen. In einer bevorzugten Ausgestaltung weisen die Lichtquellen der Lichtquellen-Menge hingegen insgesamt mindestens zwei verschiedene korrelierte Farbtemperaturen auf. Die Lichtquellen einer ersten Teilmenge weisen also eine erste korrelierte Farbtemperatur auf, die Lichtquellen einer zweiten Teilmenge eine zweite korrelierte Farbtemperatur, die sich von der ersten korrelierten Farbtemperatur unterscheidet. Beispielsweise sind die Lichtquellen der ersten Teilmenge warmweiß und die Lichtquellen der zweiten Teilmenge kaltweiß. In einer Realisierungsform haben die Lichtquellen der ersten Teilmenge eine Farbtemperatur von 2700 Kelvin (warmweiß), die Lichtquellen der zweiten Teilmenge eine Farbtemperatur von 6500 Kelvin (kaltweiß). Möglich sind auch mehr als zwei verschiedene korrelierte Farbtemperaturen.

Die Beleuchtung, welche die Beleuchtungsvorrichtung insgesamt erzielt, weist eine korrelierte Gesamt-Farbtemperatur auf. Diese korrelierte Gesamt-Farbtemperatur setzt sich additiv aus den korrelierten Farbtemperaturen der aktuell eingeschalteten und nicht vollständig abgeschatteten Lichtquellen zusammen und hängt in der Regel zusätzlich von den jeweiligen maximalen Einzel-Beleuchtungsstärken dieser Lichtquellen ab.

In einer Ausgestaltung vermag das Steuergerät eine Farbtemperatur-Vorgabe zu erfassen. Diese Vorgabe kann wiederum von einem Benutzer oder von einer übergeordneten Steuerung oder Regelung stammen. Wie gerade dargelegt, berechnet das Steuergerät für jede Lichtquelle der Lichtquellen-Menge jeweils eine maximale Einzel-Beleuchtungsstärke. Diese maximale Einzel-Beleuchtungsstärke hängt von der Beleuchtungsstärken-Vorgabe, optional von der Lichtfeld-Durchmesser-Vorgabe und zusätzlich von der optionalen Farbtemperatur-Vorgabe ab. In der Regel vermag das Steuergerät durch eine Ansteuerung den Wert der jeweiligen maximalen Einzel-Beleuchtungsstärke einer Lichtquelle zu verändern, und zwar zwischen Null und der jeweiligen maximal möglichen Einzel-Beleuchtungsstärke, während der jeweilige Lichtfeld-Durchmesser und die jeweilige korrelierte Farbtemperatur jeder Lichtquelle bevorzugt konstant sind und sich nicht verändern lassen.

Sobald das Steuergerät ein Abschattungs-Ereignis detektiert hat, ermittelt das Steuergerät erfindungsgemäß eine Teilmenge mit Lichtquellen, die zur Abschattungs-Kompensation geeignet sind, und berechnet für jede Lichtquelle der ermittelten Teilmenge jeweils einen Sollwert für die maximale Einzel-Beleuchtungsstärke oder legt diesen fest - natürlich nur, wenn mindestens eine geeignete Lichtquelle gefunden ist. Das oberstes Ziel bei der Berechnung dieser Sollwerte ist, dass die Abweichung zwischen der Beleuchtungsstärken-Vorgabe und dem erzielten oder erwarteten Wert der maximalen Gesamt-Beleuchtungsstärke möglichst gering ist. Optionale weitere Ziele sind, und zwar bevorzugt in dieser Reihenfolge, dass der erzielte Gesamt-Lichtfeld-Durchmesser möglichst wenig von einem erfassten Lichtfeld-Durchmesser-Vorgabe abweicht und dass die erzielte korrigierte Gesamt-Farbtemperatur möglichst wenig von einer erfassten Farbtemperatur-Vorgabe.

Erfindungsgemäß vermag das Steuergerät eine Beleuchtungsstärken-Vorgabe zu erfassen, optional zusätzlich eine Lichtfeld-Durchmesser-Vorgabe und / oder eine Farbtemperatur-Vorgabe. Bevorzugt ist das Steuergerät wie folgt ausgestaltet: Wenn es eine Vorgabe erfasst hat, so berechnet es für jede Lichtquelle der Lichtquellen-Menge jeweils einen initialen Sollwert. Dieser initiale Sollwert legt die maximale Einzel-Beleuchtungsstärke fest, den die Lichtquelle erzeugen soll. Das Ziel bei der Berechnung der initialen Sollwerte ist, dass die erzielte Gesamt-Beleuchtungsstärke gleich der Beleuchtungsstärken-Vorgabe ist und optional der erzielte Gesamt-Lichtfeld-Durchmesser gleich der Lichtfeld-Durchmesser-Vorgabe ist.

In einer Realisierungsform wertet das Steuergerät automatisch eine Tabelle ("look-up table") aus, die für mehrere mögliche Beleuchtungsstärken-Vorgaben und optional mehrere mögliche Lichtfeld-Durchmesser-Vorgaben und / oder Farbtemperatur-Vorgaben für jede Lichtquelle der Lichtquellen-Menge jeweils einen dann einzustellenden initialen Sollwert vorgibt. Die initialen Sollwerte gelten für eine Situation ohne Abschattung und bleiben mindestens so lange gültig, weg das Steuergerät keine abweichende Vorgabe erfasst hat und auch kein abschattendes Ereignis detektiert hat. Die Ausgestaltung mit der Tabelle erfordert in vielen Fällen relativ wenig Rechenkapazität und / oder Rechenzeit.

Das Steuergerät ist weiterhin wie folgt ausgestaltet: Wenn ein Abschattungs-Ereignis detektiert ist und eine zur Abschattungs-Kompensation geeignete Lichtquelle gefunden ist, so führt das Steuergerät mindestens einmal eine Vergrößerungs-Abfolge durch. Die oder jede Vergrößerungs-Abfolge umfasst folgende Schritte:
- Das Steuergerät ermittelt eine Teilmenge. Diese Teilmenge besteht aus mindestens einer aktuell zur Abschattungs-Kompensation geeigneten Lichtquelle der Lichtquellen-Menge. Welche Kriterien eine aktuell geeignete Lichtquelle erfüllt, wurde weiter oben erläutert.
- Für die oder jede Lichtquelle der ermittelten Teilmenge berechnet das Steuergerät jeweils einen Sollwert. Dieser Sollwert legt die maximale Einzel-Beleuchtungsstärke fest, den die Lichtquelle erzielen soll. Ein Ziel bei der Berechnung der Sollwerte wurde weiter oben erläutert, nämlich dass die erzielte Gesamt-Beleuchtungsstärke gleich der erfassten Beleuchtungsstärken-Vorgabe ist. In der Regel ändert sich die Beleuchtungsstärken-Vorgabe durch das abschattende Ereignisse nicht. Der oder mindestens ein Sollwert kann die maximal mögliche Einzel-Beleuchtungsstärke der Lichtquelle sein.
- Das Steuergerät steuert die oder jede Lichtquelle der ermittelten Teilmenge an. Durch die Ansteuerung bewirkt das Steuergerät, dass die jeweilige aktuelle maximale Einzel-Beleuchtungsstärke jeder Lichtquelle auf den jeweils berechneten Sollwert vergrößert wird.

In einer Fortbildung dieser Ausgestaltung ist das Steuergerät weiterhin wie folgt ausgestaltet:
- Jede Vergrößerungs-Abfolge umfasst die Schritte, eine Teilmenge zu ermitteln und für jede Lichtquelle der ermittelten Teilmenge jeweils einen Sollwert für deren maximale Einzel-Beleuchtungsstärke zu berechnen. Gemäß der Fortbildung prüft das Steuergerät rechnerisch, ob der dann erzielte tatsächliche Wert der maximalen Gesamt-Beleuchtungsstärke der Beleuchtungsvorrichtung ausreichend genau mit der erfassten Beleuchtungsstärken-Vorgabe übereinstimmt oder übereinstimmen wird. Für diese rechnerische Prüfung verwendet das Steuergerät den jeweiligen Wert der maximalen Einzel-Beleuchtungsstärke jeder nicht abgeschatteten Lichtquelle der Lichtquellen-Menge sowie die jeweiligen Informationen über jede nicht abgeschattete Lichtquelle. Das Steuergerät führt bevorzugt eine rechnerische Vorhersage durch, bevor sie tatsächlich geeignete Lichtquellen ansteuert. Bei dieser Vorhersage sagt das Steuergerät vorher, welche maximale Gesamt-Beleuchtungsstärke bei diesen Einstellungen die Beleuchtungsvorrichtung erzielen wird.
- Falls die rechnerisch vorhergesagte maximale Gesamt-Beleuchtungsstärke nicht ausreichend genau mit der Beleuchtungsstärken-Vorgabe übereinstimmt, so prüft das Steuergerät, ob mindestens eine maximale Einzel-Beleuchtungsstärke vergrößert oder weiter vergrößert werden kann.
- Falls ja, so führt das Steuergerät bevorzugt erneut die Schritte durch, eine Teilmenge zu ermitteln und für jede Lichtquelle der bei der erneuten Durchführung ermittelten Teilmenge jeweils eine maximale Einzel-Beleuchtungsstärke zu berechnen. Die Teilmenge, die bei der weiteren Ermittlung ermittelt wird, kann die gleiche sein wie die zuvor ermittelte Teilmenge sein oder sich von dieser unterscheiden.
- Alternativ stellt das Steuergerät fest, dass die Abweichung zwischen der Beleuchtungsstärken-Vorgabe und der aktuellen maximalen Gesamt-Beleuchtungsstärke nicht weiter verringert werden kann, und führt daher keine weitere Vergrößerungs-Abfolge durch.

Dieses schrittweise Vorgehen erfordert in manchen Fällen weniger Rechenzeit als ein anderes mögliches Vorgehen.

Erfindungsgemäß ist das Steuergerät wie folgt ausgestaltet: Falls es mindestens eine zur Abschattungs-Kompensation geeignete Lichtquelle gefunden hat, so ermittelt das Steuergerät eine Teilmenge mit mindestens einer geeigneten Lichtquelle, optional mit mehreren geeigneten Lichtquellen.

Bevorzugt löst der Vorgang, dass ein Abschattungs-Ereignisse detektiert ist, folgende Schritte aus:
- Das Steuergerät detektiert mindestens eine Lichtquelle, die vollständig oder wenigstens teilweise abgeschattet ist, bevorzugt jede abgeschattete Lichtquelle.
- Das Steuergerät sucht nach einer Lichtquelle, die bezüglich der oder mindestens einer abgeschatteten Lichtquelle ein vorgegebenes Kompensierungs-Kriterium erfüllt.
- Dann wenn mindestens eine solche Lichtquelle gefunden ist, prüft das Steuergerät, welche der gefundenen Lichtquellen zur Abschattungs-Kompensation geeignet sind.
- Dann, wenn mindestens eine Lichtquelle gefunden ist, die das Kompensierungs-Kriterium erfüllt und die geeignet ist, ermittelt das Steuergerät als Teilmenge eine solche Menge von Lichtquellen, die aus erfüllenden und geeigneten Lichtquellen besteht. Das Steuergerät verwendet diese ermittelte Teilmenge, um die Abschattung wenigstens teilweise zu kompensieren.

Bevorzugt verwendet das Steuergerät erfassbare Informationen über die Lichtquellen, um zu prüfen, ob eine Lichtquelle das oder jedes vorgegebene Kompensierungs-Kriterium erfüllt. Diese erfassbaren Informationen sind vorab festgelegt und hängen nicht vom aktuellen Betrieb oder von einer Abschattung ab. Daher lassen diese Informationen sich rasch erfassen, beispielsweise durch einen Lesezugriff auf einen Datenspeicher. Daher sucht das Steuergerät bevorzugt zunächst nach weiteren Lichtquellen, die das oder jedes Kompensierungs-Kriterium erfüllen, und prüft dann, ob diese weiteren Lichtquellen einzeln oder in Kombination auch zur Kompensierung der Abschattung geeignet sind.

Die weiter oben beschriebene Ausgestaltung, dass mindestens eine Vergrößerungs-Abfolge durchgeführt wird, lässt sich mit der beschriebenen Ausgestaltung, dass mindestens ein Kompensierungs-Kriterium verwendet wird, kombinieren. bevorzugt umfasst die Kombination folgende Schritte:
- Eine erste Vergrößerungs-Abfolge wird durchgeführt. Bei dieser ersten Vergrößerungs-Abfolge wird nach Lichtquellen gesucht, die das oder jedes Kompensierungs-Kriterium erfüllen und zur Abschattungs-Kompensation geeignet sind. Die bei der ersten Vergrößerungs-Abfolge verwendete Teilmenge besteht aus solchen Lichtquellen, die das oder jedes Kompensierungs-Kriterium erfüllen und die zur Abschattungs-Kompensation geeignet sind.
- Vorhergesagt oder geprüft wird, ob die erste Vergrößerungs-Abfolge ausreicht, um die Abschattung zu kompensieren.
- Falls dies nicht der Fall ist, wird eine zweite Vergrößerungs-Abfolge durchgeführt. Bei dieser zweiten Vergrößerungs-Abfolge wird nach Lichtquellen gesucht, die nicht jedes Kompensierungs-Kriterium erfüllen, optional kein Kompensierungs-Kriterium erfüllen, und die bei der ersten Vergrößerungs-Abfolge nicht verwendet wurden und die ebenfalls zur Abschattungs-Kompensation geeignet sind.

Möglich ist auch, mehr als zwei Vergrößerungs-Abfolgen durchzuführen, wobei nach jeder Vergrößerungs-Abfolge, optional außer nach der letzten Vergrößerungs-Abfolge, geprüft wird, ob die Abschattung nunmehr ausreichend kompensiert ist, und wobei in jeder Vergrößerungs-Abfolge schwächere Bedingungen an die verwendeten Lichtquellen gestellt werden als bei der vorhergehenden Vergrößerungs-Abfolge.

Nachfolgend wird eine bevorzugte Ausgestaltung beschrieben, wie diese Teilmenge ermittelt wird. Diese bevorzugte Ausgestaltung spezifiziert das oder ein Kompensierungs-Kriterium. Besonders bevorzugt braucht bei dieser bevorzugten Ausgestaltung keine Lichtquelle der Lichtquellen-Menge ihre Position und Orientierung relativ zu einer anderen Lichtquelle zu verändern.

Gemäß dieser bevorzugten Ausgestaltung umfasst die Beleuchtungsvorrichtung mindestens eine erste Lichtquelle und mindestens eine andere Lichtquelle, die zur ersten Lichtquelle redundant ist. Eine Lichtquelle B ist zu einer Lichtquelle A redundant,
- wenn die Schnittpunkte der Lichtachsen der beiden Lichtquellen A und B mit einer beleuchteten ebenen Oberfläche um nicht mehr als eine vorgegebene Toleranz voneinander beabstandet sind und
- wenn außerdem die Lichtfelder, welche die beiden Lichtquellen A und B erzielen, um nicht mehr als eine vorgegebene Toleranz voneinander abweichen.

Bevorzugt wird folgende Definition verwendet: Zwei Lichtfelder weichen um nicht mehr als eine vorgegebene Toleranz voneinander ab, wenn in jedem Punkt der beleuchteten Oberfläche die beiden Beleuchtungsstärken, die die beiden Lichtquellen in diesem Punkt erzielen, um höchstens diese Toleranz voneinander abweichen. Die beiden Schnittpunkte und Lichtfelder beziehen sich auf eine Situation ohne Abschattung und bevorzugt wieder auf den oben erwähnten Referenzabstand und auf einer Ebene, die im Referenzabstand senkrecht auf der optischen Mittelachse steht. Zumindest dann, wenn keine Lichtquelle der Beleuchtungsvorrichtung relativ zu einer anderen Lichtquelle bewegt werden kann, ist durch die Konstruktion der Beleuchtungsvorrichtung vorgegeben, welche Lichtquellen zu welchen anderen Lichtquellen redundanter sind. Diese Redundanz verändert sich nicht während eines Einsatzes der Beleuchtungsvorrichtung.

Möglich ist, auch nur das erste Kriterium (Schnittpunkte dicht beieinander) zu verwenden, um festzulegen, wann eine Lichtquelle B zu einer Lichtquelle A redundant ist. In vielen Fällen ist das erste Kriterium dann erfüllt, wenn das zweite Kriterium (fast übereinstimmende Lichtfelder) erfüllt ist.

Gemäß der bevorzugten Ausgestaltung ist für jede Lichtquelle der Lichtquellen-Menge zusätzlich folgende Informationen abgespeichert: Welche andere Lichtquelle oder welche anderen Lichtquellen der Lichtquellen-Menge sind zur ersten Lichtquelle redundant? Die Information, welche andere Lichtquelle(n) zu einer ersten Lichtquelle redundant ist (sind), ist durch die Konstruktion der Beleuchtungsvorrichtung vorgegeben. Natürlich ist es möglich, dass es für eine Lichtquelle keine redundante Lichtquelle gibt.

Das Steuergerät ist wie folgt ausgestaltet:
- Falls es ein Abschattungs-Ereignis detektiert hat, so ermittelt das Steuergerät mindestens eine Lichtquelle, bevorzugt jede Lichtquelle der Lichtquelle-Menge, die aktuell vollständig oder wenigstens teilweise abgeschattet ist. In aller Regel bewirkt ein Abschattungs-Ereignis, dass mindestens eine Lichtquelle abgeschattet ist.
- Das Steuergerät sucht nach einer Lichtquelle der Lichtquellen-Menge, die zu der oder einer abgeschatteten Lichtquelle redundant ist, bevorzugt nach jeder solchen redundanten Lichtquelle. Hierfür verwendet das Steuergerät die gerade beschriebenen Informationen über redundante Lichtquellen.
- Falls das Steuergerät mindestens eine solche redundante Lichtquelle gefunden hat, so prüft es, welche der gefundenen redundanten Lichtquellen zur Abschattungs-Kompensation geeignet sind.

Möglich ist, dass keine geeignete redundante Lichtquelle gefunden wird. Falls mindestens eine redundante und geeignete Lichtquelle gefunden ist, so ermittelt und verwendet das Steuergerät als Teilmenge eine Menge von Lichtquellen, die aus mindestens einer redundanten geeigneten Lichtquelle besteht, bevorzugt aus allen redundanten geeigneten Lichtquellen.

Das Steuergerät bewirkt, dass die jeweilige maximale Einzel-Beleuchtungsstärke jeder redundanten und geeigneten Lichtquelle der ermittelten Teilmenge vergrößert wird. Das oder wenigstens ein erfindungsgemäßes Ziel ist, dass die erzielte Gesamt-Beleuchtungsstärke gleich der Beleuchtungsstärken-Vorgabe ist.

Bevorzugt sucht das Steuergerät für jede abgeschattete Lichtquelle jeweils genau eine redundante geeignete Lichtquelle. Bei n abgeschatteten Lichtquellen ermittelt das Steuergerät bis zu n verschiedene redundante geeignete Lichtquellen.

Die Ausgestaltung mit den redundanten Lichtquellen führt in vielen Fällen zu einer raschen und oft sogar vollständigen Kompensation der Abschattung - vorausgesetzt genügend redundante und geeignete Lichtquellen werden gefunden. Denn die Informationen, welche Lichtquellen zu welchen anderen Lichtquellen redundant sind, sind durch die Konstruktion vorgegeben, und das Steuergerät vermag diese Informationen durch einen Lesezugriff zu ermitteln. Außerdem erfordert diese Ausgestaltung häufig relativ wenig Rechenkapazität und daher Rechenzeit.

In einer weiteren bevorzugten Ausgestaltung umfasst die Beleuchtungsvorrichtung mindestens eine zweite Lichtquelle und mindestens eine andere Lichtquelle, die zur zweiten Lichtquelle teil-redundant ist. Diese zweite Lichtquelle kann die erste Lichtquelle sein, die weiter oben mit Bezug auf redundante Lichtquellen beschrieben worden ist, oder eine andere Lichtquelle sein. Eine Lichtquelle B ist zu einer Lichtquelle A teil-redundant, wenn mindestens eine der folgenden Bedingungen erfüllt ist:
- Die beiden Schnittpunkte der beiden Lichtachsen der beiden Lichtquellen sind um nicht mehr als die vorgegebene Toleranz voneinander entfernt.
- Der Schnittpunkt der Lichtachse der Lichtquelle B liegt im dₓ-Kreis um den Schnittpunkt der Lichtachse der Lichtquelle A und umgekehrt. Mit anderen Worten: Mindestens dann, wenn beide Lichtquellen mit der maximal möglichen Einzel-Beleuchtungsstärke betrieben werden und keine Abschattung vorliegt, so gilt folgendes: Im Schnittpunkt der Lichtachse der Lichtquelle B mit der beleuchteten Oberfläche erzielt die Lichtquelle A eine Einzel-Beleuchtungsstärke, die mindestens x% der maximal möglichen Einzel-Beleuchtungsstärke der Lichtquelle A ist, und umgekehrt.
- Die Lichtfelder, welche diese beiden Lichtquellen A und B erzielen, weichen um mehr als die vorgegebene Toleranz voneinander ab.

In einer Realisierungsform sind zwei Lichtquellen zueinander immer auch teil-redundant, wenn sie zueinander redundant sind. In einer anderen Realisierungsform sind zwei Lichtquellen nur dann zueinander teil-redundant, wenn die oben genannten Kriterien erfüllt sind und die Lichtquellen nicht zueinander redundanter sind. Möglich ist auch, nur die Ausgestaltung mit den redundanten Lichtquellen oder nur die Ausgestaltung mit den teil-redundanten Lichtquellen zu verwenden.

Gemäß der Ausgestaltung mit den teil-redundanten Lichtquellen ist für jede Lichtquelle der Lichtquellen-Menge zusätzlich abgespeichert, welche anderen Lichtquellen zu dieser Lichtquelle teil-redundant sind. Das Steuergerät ist dazu ausgestaltet, für jede abgeschattete Lichtquelle nach einer weitere Lichtquelle zu suchen, die zur abgeschatteten Lichtquelle teil-redundant und zur Abschattungs-Kompensation geeignet ist. Die weiteren Schritte entsprechen denjenigen, die gerade mit Bezug auf redundante Lichtquellen beschrieben wurden.

Auch die Information über teil-redundante Abschattungen ist durch die Konstruktion der Beleuchtungsvorrichtung vorgegeben, und das Steuergerät kann diese Information durch eine Lesezugriff ermitteln. Die Ausgestaltung mit den redundanten und / oder teil-redundanten Lichtquellen schränkt den Suchraum bei der Suche nach Lichtquellen, die aktuell zur Abschattungs-Kompensation geeignet sind, ein. In vielen Fällen braucht das Steuergerät daher weniger Berechnungen durchzuführen, was Rechenzeit einspart und daher in vielen Fällen zu einer schnelleren Kompensation der detektierten Abschattung führt, verglichen mit einer Ausgestaltung, bei der der Suchraum aus allen nicht abgeschatteten Lichtquellen der Lichtquellen-Menge besteht.

Die Ausgestaltung mit den redundanten Lichtquellen und die Ausgestaltung mit den teil-redundanten Lichtquellen lassen sich auf verschiedene Weisen miteinander kombinieren. In einer Realisierungsform sucht das Steuergerät für jede abgeschattete Lichtquelle in einem Arbeitsschritt sowohl nach redundanten als auch nach teil-redundanten Lichtquellen, die zur Abschattungs-Kompensation geeignet sind, und verwendet als Teilmenge die Menge aller geeigneten redundanten und teil-redundanten Lichtquelle.

Die nachfolgend beschriebene abweichende Realisierungsform einer Kombination spezifiziert hingegen ein schrittweises Vorgehen. Die Lichtquellen-Menge der Beleuchtungsvorrichtung ist gemäß dieser abweichenden Realisierungsform wie folgt ausgestaltet: Für mindestens eine erste Lichtquelle gibt es mindestens eine redundante Lichtquelle, für mindestens eine zweite Lichtquelle mindestens eine teil-redundante Lichtquelle. Das Steuergerät vermag eine erste Vergrößerungs-Abfolge und bei Bedarf eine zweite Vergrößerungs-Abfolge durchzuführen. Das Steuergerät versucht bei der ersten Vergrößerungs-Abfolge, die Abschattung mit Hilfe von mindestens einer redundanten und / oder teil-redundanten Lichtquelle zu kompensieren. Die oder jede bei der ersten Vergrößerungs-Abfolge verwendete Lichtquelle erfüllt zusätzlich mindestens ein vorgegebenes Zusatz-Kriterium. Nach der ersten Vergrößerungs-Abfolge ermittelt oder berechnet das Steuergerät, welche Gesamt-Beleuchtungsstärke die Beleuchtungsvorrichtung nunmehr erzielt oder erzielen wird, und vergleicht diese mit der erfassten Beleuchtungsstärken-Vorgabe.

Falls die aktuelle Gesamt-Beleuchtungsstärke um mehr als eine vorgegebene Toleranz von der erfassten Beleuchtungsstärken-Vorgabe abweicht, führt das Steuergerät eine nachfolgende zweite Vergrößerungs-Abfolge durch. Bei der zweiten Vergrößerungs-Abfolge versucht das Steuergerät, die Abschattung zusätzlich mit Hilfe von mindestens einer redundanten und / oder teil-redundanten Lichtquelle zu kompensieren. Die oder jede bei der zweiten Vergrößerungs-Abfolge verwendete Lichtquelle erfüllt nicht das oder jedes vorgegebene Zusatz-Kriterium. In vielen Fällen führt die Durchführung der zweiten Vergrößerungsabfolge dazu, dass die Abschattung zwar relativ gut kompensiert werden kann, jedoch eine Beleuchtungsstärken-Vorgabe und / und eine optionale Lichtfeld-Durchmesser-Vorgabe weniger gut eingehalten werden kann.

Gemäß der gerade beschriebenen Realisierungsform wird eine erste Vergrößerungs-Abfolge durchgeführt, bei der nach redundanten und / oder teil-redundanten Lichtquellen gesucht wird, die zusätzlich das oder jedes vorgegebene Zusatz-Kriterium erfüllen. Das oder jedes Zusatz-Kriterium schränkt den Suchraum weiter ein. Falls die erste Vergrößerungs-Abfolge allein nicht dazu führt, die Abschattung ausreichend zu kompensieren, so wird eine nachfolgende zweite Vergrößerungs-Abfolge durchgeführt, bei der ebenfalls nach redundanten und / oder teil-redundanten Lichtquellen gesucht wird, aber kein oder wenigstens nicht jedes vorgegebene Zusatz-Kriterium verwendet wird. Die bei der zweiten Vergrößerungs-Abfolge gefundenen Lichtquellen brauchen also nicht notwendigerweise das oder jedes Zusatz-Kriterium zu erfüllen.

Unterschiedliche Realisierungsformen sind möglich, wie das oder ein Zusatz-Kriterium für die erste Vergrößerungs-Abfolge ausgestaltet sein kann.

In einer Ausgestaltung ist das oder ein Zusatz-Kriterium für die erste Vergrößerungs-Abfolge das, dass die weitere Lichtquelle zur ersten Lichtquelle redundant ist. In der ersten Vergrößerungs-Abfolge wird also nur nach solchen Lichtquellen der Lichtquellen Menge gesucht, die zu mindestens einer abgeschatteten Lichtquelle redundant ist. Teil-redundante Lichtquellen werden in der ersten Vergrößerungs-Abfolge nicht berücksichtigt, bevorzugt aber in der zweiten Vergrößerungs-Abfolge. Mit anderen Worten: In der ersten Vergrößerungs-Abfolge wird nur nach redundanten Lichtquellen gesucht, in der zweiten Vergrößerungs-Abfolge nach teil-redundanten Lichtquellen, und zwar nach solchen, die nicht bereits in der ersten Vergrößerungs-Abfolge gefunden wurden, in der Regel also nach teil-redundanten, aber nicht redundanten Lichtquellen. In der Regel lässt sich die Abschattung einer Lichtquelle besonders gut durch eine nicht abgeschaltete und zur abgeschatteten Lichtquelle redundanten weiteren Lichtquelle kompensieren. In manchen Fällen reichen redundante und nicht abgeschaltete Lichtquellen aber nicht aus, um die Abschattung ausreichend zu kompensieren.

Weiter oben wurden drei Kriterien aufgelistet, wann eine Lichtquelle B teil-redundant zu einer Lichtquelle A ist. In einer Realisierungsform sucht das Steuergerät in einem Schritt nach jeder Lichtquelle, die zur Abschattungs-Kompensation geeignet ist und mindestens eines dieser drei Kriterien erfüllt. Bevorzugt wird diese Suche auf diejenigen geeigneten Lichtquellen beschränkt, die nicht zu einer abgeschalteten Lichtquelle redundant sind und daher nicht bereits bei der ersten Vergrößerungs-Abfolge gefunden sind. In einer anderen Ausgestaltung wird eine absteigende Reihenfolge unter diesen Kriterien vorgegeben. Zunächst sucht das Steuergerät nach den geeigneten Lichtquellen, welche das am höchsten bewertete Kriterium für Teil-Redundanz erfüllen, danach nach den geeigneten Lichtquellen, die das zweithöchste Kriterium erfüllen und so fort.

Erfindungsgemäß ist das oberste Ziel, dass durch die Kompensation der Abschattung der Wert der maximalen Gesamt-Beleuchtungsstärke, den die Beleuchtungsvorrichtung aktuell erzielt, der erfassten Beleuchtungsstärken-Vorgabe nahekommt, idealerweise gleich ist. Ein optionales weiteres Ziel ist, dass zusätzlich eine Lichtfeld-Durchmesser-Vorgabe eingehalten wird, ein optionales drittes Ziel, dass zusätzlich eine Farbtemperatur-Vorgabe eingehalten wird. Die Lichtfeld-Durchmesser-Vorgabe spezifiziert einen Lichtfeld-Durchmesser, die Farbtemperatur-Vorgabe eine korrelierte Farbtemperatur, welche die Beleuchtungsvorrichtung erzielen soll.

Möglich ist, dass das oberste Ziel und / oder ein optionales weiteres Ziel allein mit Hilfe von geeigneten redundanten und teil-redundanten geeigneten Lichtquellen nicht erreicht wird. In einer Ausgestaltung wird die Abweichung hingenommen. In einer anderen Ausgestaltung sucht das Steuergerät in dieser Situation nach weiteren geeigneten Lichtquellen, also nach Lichtquellen, die zur Abschattungs-Kompensation geeignet sind und zu den abgeschatteten Lichtquellen weder redundant noch teil-redundant sind.

Wie bereits dargelegt, ist das oder mindestens ein vorgegebenes Kompensierungs-Kriterium, dass eine weitere Lichtquelle zu einer abgeschatteten Lichtquelle redundant und / oder teil-redundant ist. Eine weitere Ausgestaltung des oder eines Kompensierungs-Kriteriums ist möglich, wobei diese weitere Ausgestaltung sich auf Lichtquellen-Gruppen bezieht. Das Kompensierungs-Kriterium mit redundanten und / oder Teil redundanten Lichtquellen lässt sich mit dem Kompensierungs-Kriterium mit den Lichtquellen-Gruppen kombinieren.

Gemäß der Ausgestaltung mit den Lichtquellen-Gruppen sind die Lichtquellen der Lichtquellen-Menge auf mindestens zwei Lichtquellen-Gruppen aufgeteilt. Jede Lichtquelle der Lichtquellen-Gruppe gehört zu einer und nur zu einer Lichtquellen-Gruppe.

Die Lichtquellen einer Lichtquellen-Gruppe weisen folgende Eigenschaft auf: Die Lichtquellen der Lichtquellen-Gruppe erzeugen zusammen auf der beleuchteten Oberfläche ein Lichtfeld mit einer maximalen Beleuchtungsstärke. In einer Alternative tritt diese maximale Beleuchtungsstärke im Schnittpunkt der optischen Mittelachse der Beleuchtungsvorrichtung mit der beleuchteten Oberfläche auf. In einer anderen Alternative tritt diese maximale Beleuchtungsstärke in einem Kreis um diesen Schnittpunkt heraus auf. Diese Eigenschaft gilt mindestens dann, wenn
- die beleuchtete Oberfläche im Referenzabstand senkrecht auf der optischen Mittelachse steht,
- alle Lichtquellen der Lichtquellen-Gruppe mit derselben maximalen Einzel-Beleuchtungsstärke betrieben werden und
- kein abschattendes Ereignis eingetreten ist.

Das oder ein Kompensierungs-Kriterium ist das folgende: Die weitere Lichtquelle und die oder einer abgeschattete Lichtquelle gehören zu derselben Lichtquellen-Gruppe. Diese Ausgestaltung führt in vielen Fällen dazu oder ermöglicht es wenigstens in vielen Fällen, dass trotz der Abschattung der Lichtfeld-Durchmesser, den die Beleuchtungsvorrichtung insgesamt auf der beleuchteten Oberfläche erzielt, annähernd gleich bleibt.

Andere oder weitere Kompensierungs-Kriterien sind möglich. Ein mögliches weiteres Kompensierungs-Kriterium ist die korrelierte Farbtemperatur. In der Regel weist jede Lichtquelle eine Beleuchtungsvorrichtung jeweils eine korrelierte Farbtemperatur auf. Bevorzugt weisen die Lichtquellen der Lichtquellen-Menge mindestens zwei verschiedene korrelierte Farbtemperaturen auf, beispielsweise kaltweiß und warmweiß. Die gesamte korrelierte Farbtemperatur der Beleuchtungsvorrichtung wird durch eine Überlagerung der korrelierten Farbtemperaturen der eingeschalteten Lichtquellen bewirkt. Ein mögliches weiteres Kompensierungs-Kriterium ist, dass die weitere Lichtquelle die gleiche korrelierte Farbtemperatur wie die abgeschattete Lichtquelle aufweist. Dieses weitere Kompensierungs-Kriterium bewirkt in vielen Fällen, dass die Farbtemperatur der Beleuchtungsvorrichtung trotz der Abschattung relativ wenig verändert wird.

Ein mögliches weiteres Kompensierungs-Kriterium ist, dass die weitere Lichtquelle aktuell mit einer relativ geringen maximalen Einzel-Beleuchtungsstärke betrieben wird, beispielsweise mit höchstens der Hälfte oder sogar nur höchstens einem Viertel der maximal möglichen Einzel-Beleuchtungsstärke. Falls die maximale Einzel-Beleuchtungsstärke dieser Lichtquelle vergrößert wird, so trägt diese Vergrößerung häufig besonders viel zur Kompensierung der Abschattung bei.

Erfindungsgemäß umfasst die Beleuchtungsvorrichtung eine Abstandsmess-Anordnung. Das Steuergerät ermittelt einen Abschattungs-Bereich, das ist ein Bereich auf der beleuchteten Oberfläche, der vom Objekt zwischen der Beleuchtungsvorrichtung und der beleuchteten Oberfläche abgeschattet wird. Mit "Abschattung" ist gemeint, dass die Gesamt-Beleuchtungsstärke auf der Oberfläche im Abschattungs-Bereich durch die Abschattung um mindestens eine vorgegebene absolute oder relative Schranke verringert wird. Das Steuergerät vermag hierfür ein Signal der Abstandsmess-Anordnung zu empfangen und auszuwerten. Bevorzugt ermittelt das Steuergerät die Kontur des abschattenden Objekts in einer Ebene, die senkrecht auf der optischen Mittelachse steht, sowie den Abstand zwischen der Beleuchtungsvorrichtung und der Kontur und / oder der Kontur und der beleuchteten Oberfläche. In einer Ausgestaltung vermag das Steuergerät ein topologisches Profil eines Bereichs zwischen der Lichtquellen Menge und der beleuchteten Oberfläche zu messen. Um das topologische Profil zu messen, verwendet das Steuergerät ein Signal das Signal der Abstandsmess-Anordnung. Falls sich kein abschattendes Objekt im Bereich zwischen der Beleuchtungsvorrichtung und der beleuchteten Oberfläche befindet, so ist dieses Profil gleich dem Profil der beleuchteten Oberfläche und umfasst beispielsweise das Profil eines beleuchteten Patienten auf einem Operationstisch Als der beleuchteten Oberfläche. Ein abschattendes Objekt verändert dieses topologische Profil. Das Steuergerät vermag dieses ermittelte topologische Profil auszuwerten, bevorzugt mit der oben erwähnten Abtastfrequenz. Das Steuergerät vermag ein Abschattungs-Ereignis abhängig von der Auswertung des topologischen Profils zu detektieren. In vielen Fällen verändert ein abschattendes Objekt das topologische Profil schlagartig, und das Steuergerät detektiert eine schlagartige Veränderung. Bevorzugt verwendet das Steuergerät außerdem das topologische Profil, um den Abschattungs-Bereich auf der beleuchteten Oberfläche zu ermitteln.

Erfindungsgemäß erfasst und verwendet das Steuergerät Informationen über die Lichtquellen der Lichtquellen-Menge, insbesondere die jeweilige maximal mögliche Einzel-Beleuchtungsstärke und die Position und / oder Orientierung der Lichtachse relativ zur optischen Mittelachse der Beleuchtungsvorrichtung. In einer Ausgestaltung sind diese Informationen in einem Datenspeicher abgespeichert, optional außerdem die jeweilige korrelierte Farbtemperatur jeder Lichtquelle. Das Steuergerät hat wenigstens zeitweise Lesezugriff auf den Datenspeicher und erfasst die Informationen durch einen Lesezugriff auf den Datenspeicher.

Die Ausgestaltung mit dem Datenspeicher ermöglicht es, das gleiche Programm zur Abschattungs-Kompensation für unterschiedliche Beleuchtungsvorrichtungen zu verwenden. Die Konstruktion einer bestimmten Beleuchtungsvorrichtung wird durch entsprechende Informationen in den Datenspeicher berücksichtigt, und das Programm kann unverändert bleiben. Wie bereits oben erwähnt, führt bevorzugt das Steuergerät auf einem Prozessor dieses Programm aus, während es die Beleuchtungsvorrichtung ansteuert und bei Bedarf eine Abschattungs-Kompensation durchführt.

Erfindungsgemäß vermag das Steuergerät ein Abschattungs-Ereignis zu detektieren und die detektierte Abschattung wenigstens teilweise zu kompensieren. Bei den bislang beschriebenen Ausgestaltungen wird das Abschattungs-Ereignis durch mindestens ein Objekt verursacht, welches in den Bereich zwischen der Beleuchtungsvorrichtung und der beleuchteten Oberfläche gelangt. In einer Ausgestaltung werden die gerade beschriebenen Ausgestaltungen, um eine Abschattung zu kompensieren, in gleicher Weise verwendet, um den Ausfall mindestens einer Lichtquelle zu kompensieren. Im Gegensatz zu den gerade beschriebenen Ausgestaltungen verwendet das Steuergerät in der Regel nicht ein Signal von der Abstandsmess-Anordnung, um den Ausfall einer Lichtquelle zu detektieren, sondern wertet Signale von den Lichtquellen aus, beispielsweise das Ereignis, dass eine Lichtquelle einen elektrischen Widerstand oberhalb einer vorgegebenen Schranke aufweist. Die Schritte, um den Ausfall mindestens eine Lichtquelle zu kompensieren, sind die gleichen wie die Schritte, um eine Abschattung zu kompensieren. Insbesondere ermittelt das Steuergerät, welcher Bereich auf der beleuchteten Oberfläche vom Ausfall einer Lichtquelle aktuell beeinflusst wird. Diese Ausgestaltung, um den Ausfall mindestens einer Lichtquelle zu kompensieren, ermöglicht es in vielen Fällen, die Beleuchtungsvorrichtung weiterzuverwenden, bis die oder jede ausgefallene Lichtquelle ausgetauscht ist.

Im Folgenden wird die Erfindung anhand eines Ausführungsbeispiels beschrieben. Hierbei zeigt
- Figur 1: die erfindungsgemäße Beleuchtungsvorrichtung des Ausführungsbeispiels, wobei die optische Mittelachse senkrecht auf der Zeichenebene steht und ein Betrachter auf die Lichtquellen der Beleuchtungsvorrichtung schaut;
- Figur 2: die Lichtachsen und Lichtkegel der Lichtquellen der beiden Positionsgruppen Pos.1 und Pos.4;
- Figur 3: mittels eines Diagramms in der x-z-Ebene die erzielten Lichtfelder der beiden Positionsgruppen von Figur 2;
- Figur 4: die Lichtachsen und Lichtkegel der Lichtquellen der beiden Positionsgruppen Pos.2 und Pos.5;
- Figur 5: durch eine Gegenüberstellung eine erste Realisierungsform, wie die Lichtfelder der Lichtquellen der beiden Positionsgruppen von Figur 4 relativ zueinander positioniert sind;
- Figur 6: durch eine Gegenüberstellung eine zweite Realisierungsform, wie die Lichtfelder der Lichtquellen der beiden Positionsgruppen von Figur 4 relativ zueinander positioniert sind;
- Figur 7: durch eine Gegenüberstellung eine dritte Realisierungsform, wie die Lichtfelder der Lichtquellen der beiden Positionsgruppen von Figur 4 relativ zueinander positioniert sind;
- Figur 8: mittels eines Diagramms in der x-z-Ebene die erzielten Lichtfelder der beiden Positionsgruppen von Figur 4;
- Figur 9: die Lichtachsen und Lichtkegel der Lichtquellen der beiden Positionsgruppen Pos.3 und Pos.6;
- Figur 10: mittels eines Diagramms in der x-z-Ebene die erzielten Lichtfelder der beiden Positionsgruppen von Figur 9;
- Figur 11: ein erstes Beispiel für ein abschattendes Objekt;
- Figur 12: ein zweites Beispiel für ein abschattendes Objekt;
- Figur 13: ein drittes Beispiel für ein abschattendes Objekt.

Im Ausführungsbeispiel wird die Erfindung in einer Beleuchtungsvorrichtung für einen Operationssaal verwendet, also in einer OP-Leuchte. Die Beleuchtungsvorrichtung beleuchtet einen Operationstisch, auf dem ein zu behandelnder Patient liegt. Die Beleuchtungsvorrichtung ist rotationssymmetrisch zu einer Mittelachse. Die Beleuchtungsvorrichtung ist mithilfe einer Halterung an der Decke befestigt und lässt sich frei im Raum positionieren und orientieren.

Die Beleuchtungsvorrichtung erzeugt ein Lichtfeld auf einer beleuchteten Oberfläche. Diese beleuchtete Oberfläche ist ein Operationstisch oder auch diejenige Oberfläche eines Patienten auf dem Operationstisch, die zur Beleuchtungsvorrichtung hin zeigt. In mehreren Figuren und der nachfolgenden Beschreibung wird vereinfacht eine ebene beleuchtete Oberfläche eines Objekts gezeigt und entsprechend beschrieben. Das Objekt wird mit Obj bezeichnet, die zur Beleuchtungsvorrichtung hin zeugende beleuchtete ebene Oberfläche mit Ob. Die Erfindung setzt nicht voraus, dass die beleuchtete Oberfläche eine Ebene ist.

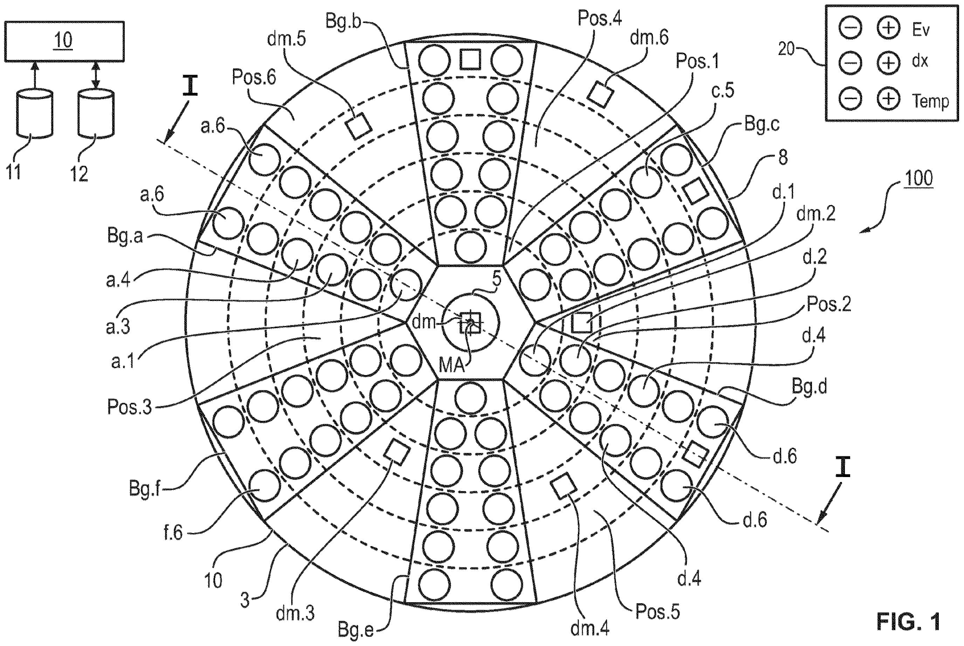

Figur 1 zeigt schematisch und beispielhaft die Beleuchtungsvorrichtung 100 des Ausführungsbeispiels, wobei die optische Mittelachse MA der Beleuchtungsvorrichtung 100 senkrecht auf der Zeichenebene von Figur 1 steht. Zu sehen ist eine Vielzahl von einzelnen Lichtquellen a.1, ..., a.6, b.1, ..., b.6, ..., die durch jeweils einen Kreis (einen Ring) dargestellt sind und an einem Träger 8 mit kreisförmigen Querschnitt befestigt sind. Diese Kreise erstrecken sich in der Zeichenebene von Figur 1. Der Betrachter von Figur 1 schaut parallel zur Mittelachse MA zu diesen Lichtquellen a.1, ..., a.6, b.1, ..., b.6, ... hin. An der zum Betrachter hin zeigenden Oberfläche des Trägers 8 ist ein schematisch gezeigter Griff 5 befestigt, mit dessen Hilfe sich die Beleuchtungsvorrichtung 100 im Raum bewegen lässt. Im Ausführungsbeispiel hat der Griff 5 die Form eines Kegelstumpfs, er kann auch z.B. die Form eines Zylinders oder eines Prismas oder Prismenstumpfs haben.

Im Ausführungsbeispiel ist jede Lichtquelle a.1, ..., a.6, b.1, ..., b.6, ... fest am Träger 8 befestigt. Daher kann sich nicht eine Lichtquelle relativ zu einer anderen Lichtquelle der Beleuchtungsvorrichtung 100 bewegen. Diese Ausgestaltung spart einen Stellantrieb für eine Lichtquelle ein. Möglich ist aber auch, dass einzelne Lichtquellen oder mindestens eine Gruppe von Lichtquellen mithilfe jeweils eine Stellantriebs (nicht gezeigt) relativ zum Träger 8 und bevorzugt auch relativ zu den übrigen Lichtquellen bewegt werden können. Der Stellantrieb ermöglicht es insbesondere, das von einer Lichtquelle erzeugte Lichtfeld relativ zur beleuchteten Oberfläche Ob zu bewegen, ohne dass der Träger 8 bewegt wird.

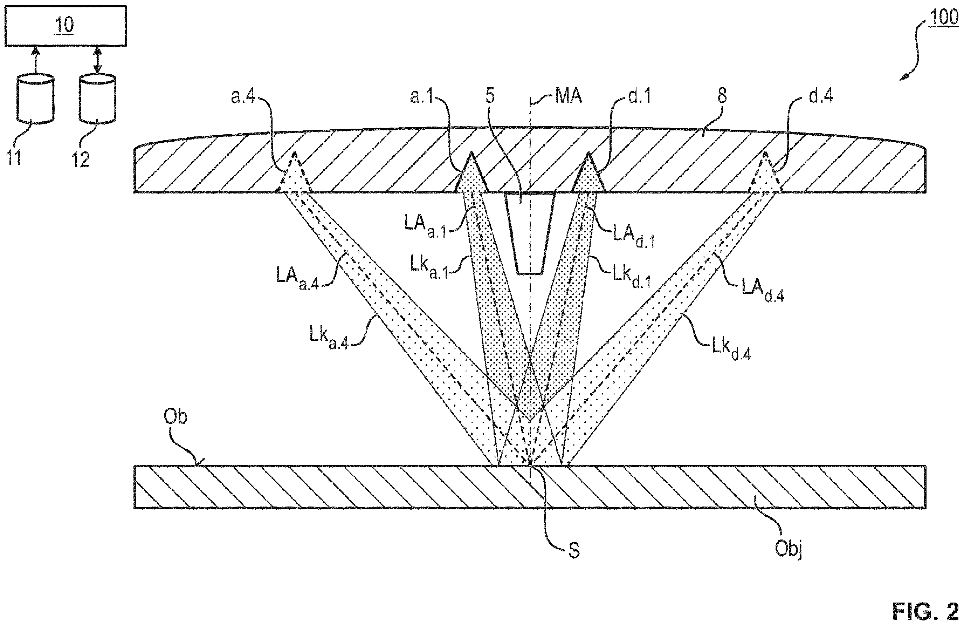

Jede einzelne Lichtquelle a.1, ..., a.6, b.1, ..., b.6, ... weist eine optische Mittelachse auf, die zur Unterscheidung von der optischen Mittelachse MA der Beleuchtungsvorrichtung 100 nachfolgend als "Lichtachse" bezeichnet wird. Die Lichtstrahlen bilden zusammen einen Kegelstumpf, dessen Durchmesser sich von der Lichtquelle a.1, ..., a.6, b.1, ..., b.6, ... vergrößert, manchmal einen Zylinder oder sogar einen Kegelstumpf, dessen Durchmesser sich von der Lichtquelle a.1, ..., a.6, b.1, ..., b.6, ... zunächst verkleinert und dann wieder vergrößert. Nachfolgend wird vereinfacht von einem "Lichtkegel" mit einem "Lichtdivergenzwinkel" gesprochen. Auch der Begriff "Lichtbündel" wäre passend. Jede einzelne Lichtquelle a.1, ..., a.6, b.1, ..., b.6, ... erzeugt also ein Lichtkegel. Beispielsweise bezeichnen LA_{a.4} die Lichtachse und Lk_{a.4} den Lichtkegel der Lichtquelle a.4. In der Mantelfläche des gezeigten Lichtkegels Lk_{a.4} beträgt die Einzel-Beleuchtungsstärke 10 % der maximalen Einzel-Beleuchtungsstärke, die entlang der Lichtachse LA_{a.4} erzielt wird. Figur 2, Figur 4 und Figur 9 zeigen jeweils einen Querschnitt in der Linie I - I von Figur 1. Die Mittelachse MA liegt in den Zeichenebenen von Figur 2, Figur 4 und Figur 9. Die Figuren sind nicht notwendigerweise maßstabgerecht.

Im Ausführungsbeispiel umfasst die Beleuchtungsvorrichtung 100 66 einzelne Lichtquellen. Selbstverständlich kann die Erfindung auch mit jeder anderen sinnvollen Anzahl von Lichtquellen realisiert werden. Die 66 Lichtquellen sind einerseits auf Baugruppen und andererseits auf Positionsgruppen aufgeteilt. Dies wird nachfolgend mit Bezug auf Figur 1 erläutert.

Im gezeigten Beispiel umfasst die Beleuchtungsvorrichtung 100 sechs Baugruppen Bg.a, ..., Bg.e, die konzentrisch um die Mittelachse MA der Beleuchtungsvorrichtung 100 angeordnet, strahlenförmig von der Mittelachse MA ausgehen und am Träger 8 befestigt sind. Die Baugruppen Bg.a, ..., Bg.e können auch anders als konzentrisch um die Mittelachse MA herum angeordnet sein. Jede Baugruppe Bg.a, ..., Bg.e umfasst in der gezeigten Realisierungsform jeweils elf Lichtquellen. Die Baugruppe Bg.a umfasst die elf Lichtquellen a.1, ..., a.6, die Baugruppe Bg.b die elf Lichtquellen b.1, ..., b.6 usw.

Die Anordnung und Ausgestaltung der Baugruppen Bg.a, ..., Bg.e ist nur beispielhaft zu verstehen. Selbstverständlich sind auch eine andere Anzahl von Baugruppen und / oder eine andere Anzahl von Lichtquellen möglich. Möglich ist auch, dass Baugruppen mit insgesamt mindestens zwei unterschiedlichen Anzahlen von Lichtquellen a.1, ..., a.6, b.1, ..., b.6, ... verwendet werden.

Bevorzugt lässt sich jede einzelne Baugruppe unabhängig von jeder anderen Baugruppe austauschen, d.h. eine alte Baugruppe lässt sich vom Träger 8 nehmen, und eine neue Baugruppe lässt sich einsetzen.

Im Ausführungsbeispiel sind die sechs Baugruppen Bg.a, ..., Bg.e fest auf dem kreisförmigen Träger 8 der Beleuchtungsvorrichtung 100 angeordnet, sodass nicht eine Baugruppe Bg.a, ..., Bg.e relativ zu einer anderen Baugruppe bewegt werden kann. Die jeweils elf Lichtquellen einer Baugruppe Bg.a, ..., Bg.e sind fest auf der Baugruppe Bg.a, ..., Bg.e befestigt, sodass keine Lichtquelle relativ zu einer anderen Lichtquelle derselben Baugruppe bewegt werden kann.

Die 66 Lichtquellen der Beleuchtungsvorrichtung 100 sind im Ausführungsbeispiel außerdem auf sechs verschiedene Positionsgruppen Pos.1, ..., Pos.6 aufgeteilt. Auch diese Anzahl ist nur beispielhaft zu verstehen. Jede Lichtquelle gehört einerseits zu genau einer (einer und nur einer) Baugruppe Bg.a, ..., Bg.e und andererseits zu genau einer Positionsgruppe Pos.1, ..., Pos.6.

Die Lichtquellen einer Positionsgruppe Pos.1, ..., Pos.6 haben im Ausführungsbeispiel alle denselben Abstand von der Mittelachse MA der Beleuchtungsvorrichtung 100. Die sechs Positionsgruppen Pos.1, ..., Pos.6 sind konzentrisch um die Mittelachse MA angeordnet. Die innerste Positionsgruppe Pos.1 bildet einen Kreisring um den Griff 5, die übrigen Positionsgruppen Pos.2, ..., Pos.6 konzentrische Kreisringe um die innerste Positionsgruppe Pos.1 herum. Jede Lichtquelle gehört zu genau einer Positionsgruppe, sodass die innerste Positionsgruppe Pos.1 sechs Lichtquellen und die übrigen fünf Positionsgruppen Pos.2, ..., Pos.6 jeweils zwölf Lichtquellen umfassen.

Die Positionsgruppen Pos.1, ..., Pos.6 des Ausführungsbeispiels bilden die Lichtquellen-Gruppen im Sinne von Patentansprüchen. Die Lichtachsen der Lichtquellen eine Positionsgruppe sind bevorzugt parallel zueinander angeordnet.

Die Nummerierung der Lichtquellen zeigt an, zu welcher Baugruppe und zu welcher Positionsgruppe eine Lichtquelle jeweils gehört: Die Lichtquellen a.x, b.x, ..., f.x gehören zur Positionsgruppe Pos.x (x=1,..., 6) und zur Baugruppe a, b, ..., f.

Wie in Figur 1 zu sehen ist, gehören im Ausführungsbeispiel jeweils zwei einzelne Lichtquellen zu derselben Baugruppe und zu derselben Positionsgruppe - mit Ausnahme der Lichtquellen der Inneren Positionsgruppe Pos.1. Die Lichtachsen dieser beiden Lichtquellen sind idealerweise dergestalt angeordnet, dass sie sich im Referenzabstand schneiden. Auf einer Ebene im Referenzabstand erzielen die beiden Lichtquellen idealerweise zwei Lichtfelder, die bis auf einen Versatz senkrecht zur Lichtachse und optional bis auf eine Verdrehung um die Lichtachse übereinstimmen. Diese beiden Lichtquellen werden daher nachfolgend als eine einzige Lichtquelle behandelt und haben daher auch die gleiche Bezeichnung. Optional senden die beiden Lichtquellen Licht in unterschiedlichen korrelierten Farbtemperaturen aus, und diese Farbtemperaturen überlagern sich zu einer Gesamt-Farbtemperatur dieser beiden Lichtquellen.

Bevorzugt tritt zwischen den Lichtachsen der Lichtquellen einer Positionsgruppe Pos.1, ..., Pos.6 und der optischen Mittelachse MA der Beleuchtungsvorrichtung 100 stets der gleiche Winkel auf. Die Lichtachsen der oder wenigstens einiger Lichtquellen können auch windschief zur optischen Mittelachse MA angeordnet sein. Wenn der Winkel zwischen der Lichtachse und der Mittelachse MA stets der gleiche ist, sind der Lichtkegel und das Lichtfeld, welches die Lichtquellen einer Positionsgruppe zusammen erzeugen, idealisiert rotationssymmetrisch zur optischen Mittelachse MA.

Zur Verdeutlichung werden die Lichtkegel von zwei verschiedenen Positionsgruppen in Figur 2, Figur 4 und Figur 9 unterschiedlich schraffiert dargestellt. Außerdem wird ein Schnittpunkt S gezeigt. In diesem Schnittpunkt S schneidet die optische Mittelachse MA der Beleuchtungsvorrichtung 100 die beleuchtete Oberfläche Ob.

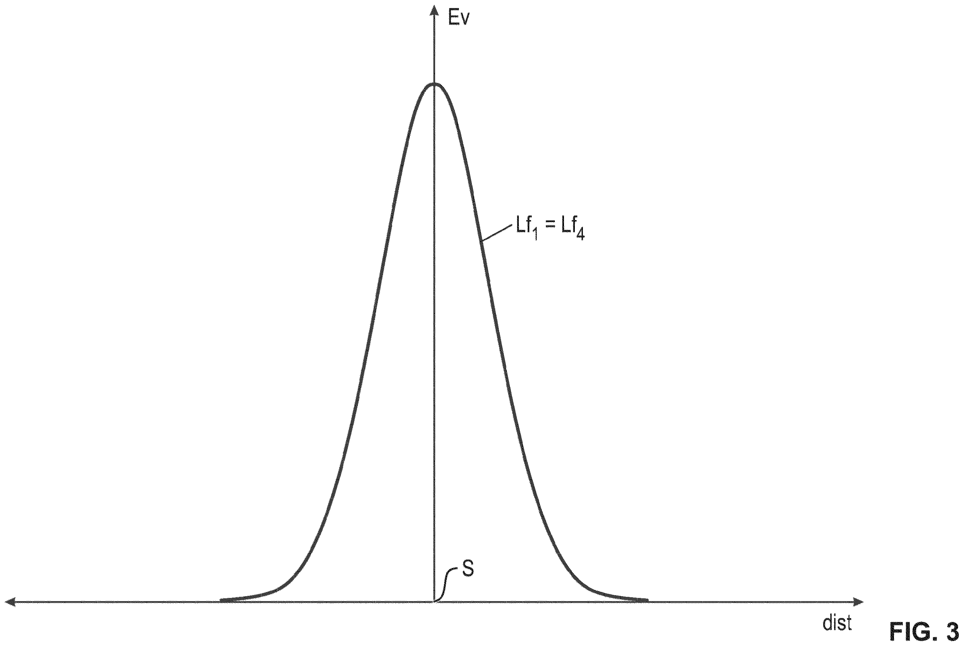

Figur 3, Figur 8 und Figur 10 veranschaulichen jeweils ein erzeugtes Lichtfeld, was nachfolgend näher beschrieben wird. Die Darstellung veranschaulicht jeweils ein Lichtfeld in der x-z-Ebene. Der Schnittpunkt S liegt im Ursprung des Koordinatensystems. Die y-Achse steht senkrecht auf der Zeichenebene. Auf der z-Achse ist die jeweils erzielte Beleuchtungsstärke Ev aufgetragen.

Im Ausführungsbeispiel sind die Lichtquellen a.1, ..., a.6, b.1, ..., b.6, ... so am Träger 8 montiert, dass die Lichtachsen und die Lichtkegel der Lichtquellen einer Positionsgruppe Pos.2, ..., Pos.6 rotationssymmetrisch um die Mittelachse MA der Beleuchtungsvorrichtung 100 angeordnet sind. Daher gelten die Darstellungen von Figur 2, Figur 4 und Figur 9 entsprechend auch für andere Schnittdarstellungen, bei denen die optische Mittelachse MA in der Zeichenebene liegt, beispielsweise eine Schnittdarstellung durch die Baugruppen Bg.c und Bg.f.

Bevorzugt strahlt jede Lichtquelle a.1, ..., a.6, b.1, ..., b.6, ... Licht entlang jeweils einer Lichtachse LA_{a.1}, ..., LA_{a.6}, LA_{b.1}, ..., LA_{b.6}, ... aus. Der Lichtkegel des von einer Lichtquelle a.1, ..., a.6, b.1, ..., b.6, ... ausgestrahlten Lichts ist rotationssymmetrisch zur jeweiligen Lichtachse LA_{a.1}, ..., LA_{a.6}, LA_{b.1}, ..., LA_{b.6}, ... und liegt bevorzugt unter 20 Grad, besonders bevorzugt unter 15 Grad, insbesondere zwischen 5 und 12,5 Grad.

Jede Lichtquelle a.1, ..., a.6, b.1, ..., b.6, ... erzielt jeweils eine aktuelle maximale Einzel-Beleuchtungsstärke, die in der Regel auf ihrer Lichtachse LA_{a.1}, ..., LA_{a.6}, LA_{b.1}, ..., LA_{b.6}, .... auftritt. Möglich ist auch, dass auf der Lichtachse ein lokales Minimum der Einzel-Beleuchtungsstärke erzielt wird. Idealerweise lässt sich diese aktuelle maximale Einzel-Beleuchtungsstärke stufenlos zwischen Null und einer maximal möglichen Einzel-Beleuchtungsstärke der Lichtquelle verändern, und zwar unabhängig von der jeweiligen aktuellen maximalen Einzel-Beleuchtungsstärke jeder anderen Lichtquelle. Möglich ist auch, dass sich die jeweilige maximale Einzel-Beleuchtungsstärke jeder Lichtquelle in mehreren Stufen verändern lässt, und zwar wiederum unabhängig von der jeweiligen maximalen Einzel-Beleuchtungsstärke jeder anderen Lichtquelle. Beispielsweise sind jeweils 8 Bit = 256 verschiedene Stufen möglich. Mindestens aber lässt sich jede Lichtquelle unabhängig von jeder anderen Lichtquelle einschalten und ausschalten.

In einer Ausgestaltung sind alle Lichtquellen a.1, ..., a.6, b.1, ..., b.6, ... baugleich. In einer bevorzugten Ausgestaltung werden hingegen zwei unterschiedliche Typen von Lichtquellen verwendet, wobei Lichtquellen eines ersten Typs warmweißes Licht und Lichtquellen eines zweiten Typs kaltweißes Licht aussenden. In einer Realisierungsform erzielen alle Lichtquellen dieselbe maximal mögliche Einzel-Beleuchtungsstärke und weisen denselben Lichtdivergenzwinkel auf. Möglich ist auch, dass sich Lichtquellen auch hinsichtlich der maximal mögliche Einzel-Beleuchtungsstärke und / oder des Lichtdivergenzwinkels unterscheiden.

Ein nur schematisch gezeigtes signalverarbeitendes Steuergerät 10 (control unit) vermag jede einzelne Lichtquelle a.1, ..., a.6, b.1, ..., b.6, ... der Beleuchtungsvorrichtung 100 anzusteuern und dadurch insbesondere zu bewirken, dass die aktuelle maximale Einzel-Beleuchtungsstärke, welche eine Lichtquelle a.1, ..., a.6, b.1, ..., b.6, ... auf der beleuchteten Oberfläche Ob erzeugt, unabhängig von der jeweiligen aktuellen maximalen Einzel-Beleuchtungsstärke jeder anderen Lichtquelle verändert wird.

Bevorzugt werden die Lichtquellen gepulst betrieben. Um die maximale Einzel-Beleuchtungsstärke einer Lichtquelle zu verändern, vermag das Steuergerät 10 die Pulsweite oder auch die Pulsdauer oder Pulsfrequenz der elektrische Impulse zu verändern. Bei einer Pulsweitenmodulation wird das Verhältnis zwischen der Dauer eines elektrischen Pulses und der Dauer einer Pause zwischen zwei elektrischen Pulsen verändert. Möglich ist auch, dass das Steuergerät 10 die Stärke des durch eine Lichtquelle fließenden Stroms oder die anliegende elektrische Spannung verändert.

In einer Realisierungsform hat das Steuergerät 10 wenigstens zeitweise Lesezugriff auf einen Datenspeicher 11. Durch einen Lesezugriff auf diesen Datenspeicher 11 und optional durch eine nachfolgende Berechnung vermag das Steuergerät 10 für jede Lichtquelle a.1, ..., a.6, b.1, ..., b.6, ... jeweils folgende Informationen zu ermitteln:
- die maximal mögliche Einzel-Beleuchtungsstärke,
- wie die aktuelle maximale Einzel-Beleuchtungsstärke von einem veränderbaren elektrischen Parameter abhängt, mit dem die Lichtquelle angesteuert wird, beispielsweise der Pulsweite, der Stromstärke und / oder der Spannung,
- die jeweilige Position auf dem Träger 8, insbesondere der Abstand von der und die Rotationsposition relativ zur optischen Mittelachse MA der Beleuchtungsvorrichtung 100,
- die Orientierung der Lichtachse relativ zur Mittelachse MA,
- der Lichtdivergenzwinkel und
- die korrelierte Farbtemperatur.

Diese Informationen sind durch die Konstruktion der Beleuchtungsvorrichtung 100 vorgegeben oder lassen sich vorab empirisch oder theoretisch ermitteln. In einer Realisierungsform ist in diesem Datenspeicher 11 ein Algorithmus in Form eines Computerprogramms abgespeichert, den das Steuergerät 10 anwendet und der für jede gleichartige Beleuchtungsvorrichtung 100 gültig ist. Die gerade aufgelisteten Informationen über die Lichtquellen sind fest in diesem Algorithmus implementiert. In einer anderen Realisierungsform ist dieser Algorithmus auf eine andere Weise im Steuergerät 10 hinterlegt. Abkürzend wird nachfolgend davon gesprochen, dass das Steuergerät 10 die Informationen über die einzelnen Lichtquellen durch einen Lesezugriff auf den Datenspeicher 11 einliest.

Die Informationen aus dem Datenspeicher 11 werden verwendet, um die Beleuchtungsvorrichtung 100 so einzustellen, dass sie auf der beleuchteten Oberfläche Ob idealerweise ein gewünschtes Gesamt-Lichtfeld erzeugt. Im laufenden Betrieb verändern sich diese Informationen nicht, solange die Beleuchtungsvorrichtung 100 nicht umgebaut wird, und bleiben daher gültig.

Wie bereits dargelegt, vermag das Steuergerät 10 die aktuelle maximale Einzel-Beleuchtungsstärke einer Lichtquelle unabhängig von der jeweiligen aktuellen maximalen Einzel-Beleuchtungsstärke jeder anderen Lichtquelle zu verändern, und zwar bevorzugt stufenlos oder mit mehr als hundert verschiedenen Stufen zwischen der maximal möglichen Einzel-Beleuchtungsstärke und Null. In einer Realisierungsform ist diese jeweilige aktuelle maximale Einzel-Beleuchtungsstärke in einem Datenspeicher 12 abgespeichert, auf den das Steuergerät 10 wenigstens zeitweise Lesezugriff und Schreibzugriff hat. Falls das Steuergerät 10 die aktuelle maximale Einzel-Beleuchtungsstärke einer Lichtquelle verändert, so verändert das Steuergerät 10 auch den entsprechenden Eintrag in diesem Datenspeicher 12.

Die Beleuchtungsvorrichtung 100 umfasst eine Eingabeeinheit 20, beispielsweise einen berührungssensitiven Bildschirm (touch screen) und / oder eine Anordnung mit mehreren Knöpfen. Diese Eingabeeinheit 20 ist beispielsweise am Träger 8, am Griff 5 oder räumlich entfernt vom Träger 8 und vom Griff 5 angeordnet und wird beispielhaft in Figur 1 gezeigt. Mit Hilfe dieser Eingabeeinheit 20 kann ein Benutzer Vorgaben für die Beleuchtung machen, welche die Beleuchtungsvorrichtung 100 auf der beleuchteten Oberfläche Ob erzeugen soll. Insbesondere kann der Benutzer mindestens einem, bevorzugt jedem der folgenden Parameter jeweils einen Wert zuweisen, beispielsweise mithilfe jeweils eines Schiebereglers oder eines Drehknopfs:
- eine Beleuchtungsstärken-Vorgabe, das ist die aktuelle maximale Gesamt-Beleuchtungsstärke, den die Beleuchtungsvorrichtung 100 im Referenzabstand erzielen soll (in Figur 1: Ev),
- ein Lichtfeld-Durchmesser dₓ, wobei der Begriff Lichtfeld-Durchmesser weiter oben definiert wurde und wobei bevorzugt x = 10 % ist (in Figur 1: dx), und
- eine korrelierte Farbtemperatur, die mit dem Lichtspektrum der Beleuchtungsvorrichtung korreliert (in Figur 1: Temp).

Alternativ kann der Benutzer den aktuellen Wert vergrößern oder verkleinern, ohne notwendigerweise einen aktuellen Wert ablesen oder einen gewünschten Wert eingeben zu müssen.

Möglich ist auch, dass zusätzlich oder stattdessen eine übergeordnete Steuerung mindestens einem dieser Parameter einen anderen Wert zuweist. Bevorzugt beziehen sich die Parameter auf den oben genannten Referenzabstand von z.B. 1m sowie auf eine ebene beleuchtete Oberfläche Ob, die senkrecht auf der optischen Mittelachse MA steht.

Das Steuergerät 10 erfasst diese Vorgaben eines Benutzers oder einer übergeordneten Steuerung und steuert die Lichtquellen a.1, ..., a.6, b.1, ..., b.6, ... mit dem Ziel an, dass die Beleuchtungsvorrichtung 100 auf der beleuchteten Oberfläche Ob ein Lichtfeld erzeugt, dessen tatsächliche Werte gleich den Vorgaben sind. In der Regel wird dieses Ziel nur näherungsweise erreicht. Das Lichtfeld und insbesondere der Lichtfeld-Durchmesser und die maximale Gesamt-Beleuchtungsstärke sowie die korrelierte Farbtemperatur der Beleuchtungsvorrichtung 100 entstehen durch eine Überlagerung der Lichtfelder und korrelierten Farbtemperaturen der einzelnen Lichtquellen a.1, ..., a.6, b.1, ..., b.6, ... Um das Lichtfeld der Beleuchtungsvorrichtung 100 einzustellen, steuert das Steuergerät 10 so wie oben beschrieben die einzelnen Lichtquellen a.1, ..., a.6, b.1, ..., b.6, ... an und verändert insbesondere bei Bedarf die jeweilige aktuelle maximale Einzel-Beleuchtungsstärke und optional die korrelierte Farbtemperatur jeder Lichtquelle. Um die korrelierte Farbtemperatur der Beleuchtungsvorrichtung 100 zu verändern, vergrößert oder verringert das Steuergerät 10 bevorzugt die jeweilige maximale Einzel-Beleuchtungsstärke von warmweißen oder kaltweißen Lichtquellen.

Figur 2 zeigt beispielhaft die Lichtachsen LA_{a.1}, LA_{d.1} und die Lichtkegel Lk_{a.1}, Lk_{d.1} von zwei Lichtquellen a.1 und d.1 der Positionsgruppe Pos.1 und die Lichtachsen LA_{a.4}, LA_{d.4} und Lichtkegel Lk_{a.4}, Lk_{d.4} von zwei Lichtquellen a.4 und d.4 der Positionsgruppe Pos.4. Die optische Mittelachse MA der Beleuchtungsvorrichtung 100 und die Lichtachsen dieser vier Lichtquellen liegen in der Zeichenebene von Figur 2.

Die beiden Positionsgruppen Pos.1 und Pos.4 sind so angeordnet, dass jede Lichtachse einer Lichtquelle der Positionsgruppe Pos.1 oder der Positionsgruppe Pos.4 im Schnittpunkt S die beleuchtete Oberfläche Ob schneidet. Dies wird in Figur 2 beispielhaft für die vier dargestellten Lichtquellen a.1, d.1, a.4, d.4 gezeigt.

Figur 3 zeigt die beiden Lichtfelder Lf₁ und Lf₄, welche die Lichtquellen der Positionsgruppen Pos.1 bzw. Pos.4 zusammen erzeugen. Die Mittelachsen MA_{a.1}, ..., MA_{d.4} der vier in Figur 2 gezeigten Lichtquellen a.1, ..., d.4 schneiden sich im Schnittpunkt S zwischen der optischen Mittelachse MA und der beleuchteten Oberfläche Ob des Objekts Obj. Daher ist die Beleuchtungsstärke Ev in diesem Schnittpunkt S am größten und nimmt mit wachsendem Abstand dist nach Form einer Glockenkurve ab. In dem gezeigten Beispiel stimmen die beiden Lichtfelder Lf₁ und Lf₄ einerseits geometrisch überein, haben also insbesondere die gleiche maximale Einzel-Beleuchtungsstärke und den gleichen Lichtfeld-Durchmesser d₁₀ auf der Oberfläche Ob, andererseits stimmen sie auch hinsichtlich der Farbtemperatur überein. Daher sind die beiden Positionsgruppen Pos.1 und Pos.4 (gewollt) redundant, nämlich weil sie auf der Oberfläche Ob das gleiche Lichtfeld Lf₁ bzw. Lf₄ erzeugen.

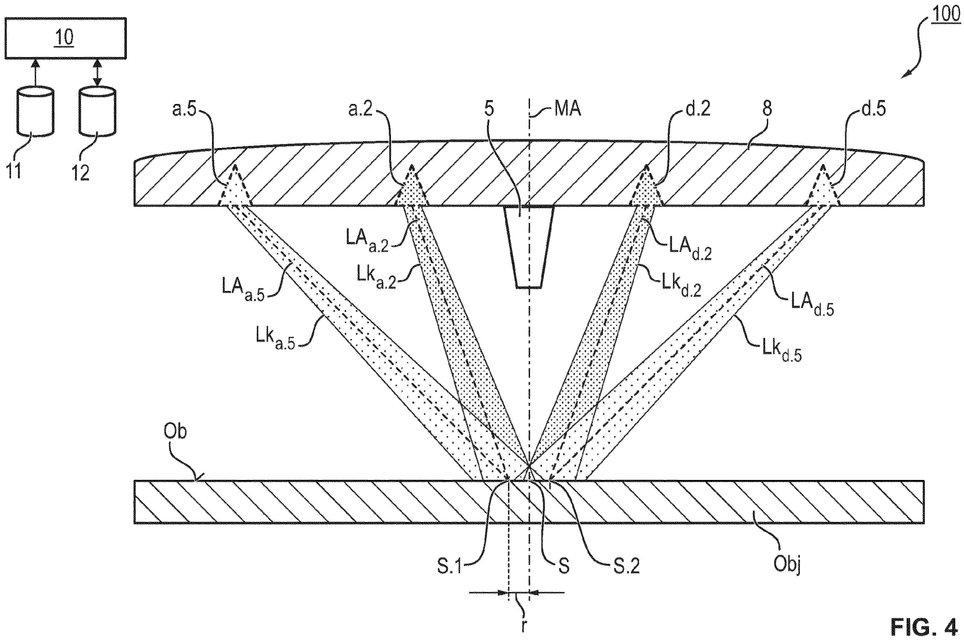

Figur 4 zeigt die vier Lichtachsen LA_{a.2}, LA_{a.5}, LA_{d.2}, LA_{d.5} und die vier Lichtkegel Lk_{a.2}, Lk_{a.5}, Lk_{d.2}, Lk_{d.5} der vier Lichtquellen a.2, a.2, d.5, d.5 der Positionsgruppen Pos.2 und Pos.5. Die Lichtachsen LA_{a.2} und LA_{a.5} der Lichtquellen a.2 und a.5 schneiden sich in demselben Schnittpunkt S.1 mit der beleuchteten Oberfläche Ob des Objekts Obj, und dieser Schnittpunkt S.1 hat einen Abstand r zur Mittelachse MA und daher zum Schnittpunkt S. Auch die Lichtachsen LA_{d.2} und LA_{d.5} der Lichtquellen d.2 und d.5 schneiden sich in demselben Schnittpunkt S.2 mit der beleuchteten Oberfläche Ob des Objekts Obj, und dieser Schnittpunkt S.2 hat denselben Abstand r zur Mittelachse MA und einen Abstand 2*r zu dem Schnittpunkt S.1 der Lichtachsen MA_{a.2} und MA_{a.5}.

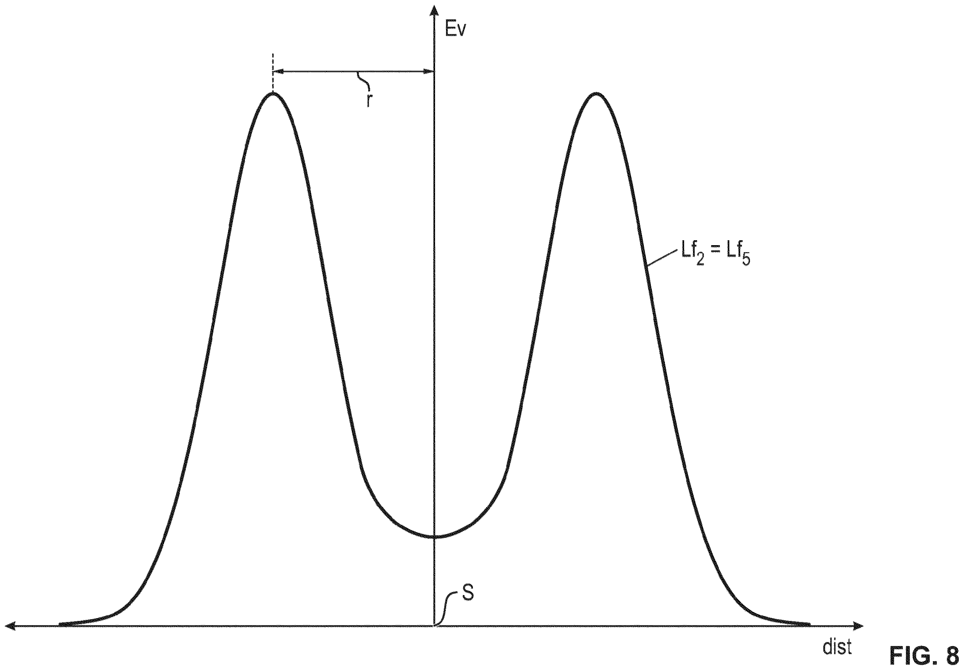

Daher hat jedes Lichtfeld Lf₂ und Lf₅ einer Ebene, in der die Lichtachse liegt, jeweils zwei Maxima, die jeweils einen Abstand r zur Mittelachse MA aufweisen, vgl. Figur 8. Die Maxima bilden einen Kreis mit dem Radius r. Allgemein bilden die Schnittpunkte S.1, S.2 der Lichtachsen der Lichtquellen der Positionsgruppen Pos.2 und Pos.5 einen Kreis um den Schnittpunkt S der optischen Mittelachse MA mit der Oberfläche Ob. Die beiden Positionsgruppen Pos.2 und Pos.5 sind teil-redundant, weil sie zwar zwei Lichtfelder Lf₂, Lf₅ um denselben Kreis herum aufweisen, wobei dieser Kreis den Mittelpunkt S aufweist, jedoch das eine Lichtfeld Lf₂ nicht exakt mit dem anderen Lichtfeld Lf₅ übereinstimmt, insbesondere wenn die Lichtquellen sich hinsichtlich ihrer maximalen Einzel-Beleuchtungsstärken und / oder des Lichtfeld-Durchmessers und / oder der korrelierten Farbtemperaturen voneinander unterscheiden. Beispielsweise ist das Lichtfeld einer Lichtquelle relativ zum Lichtfeld einer anderen Lichtquelle um die optische Mittelachse MA verdreht.

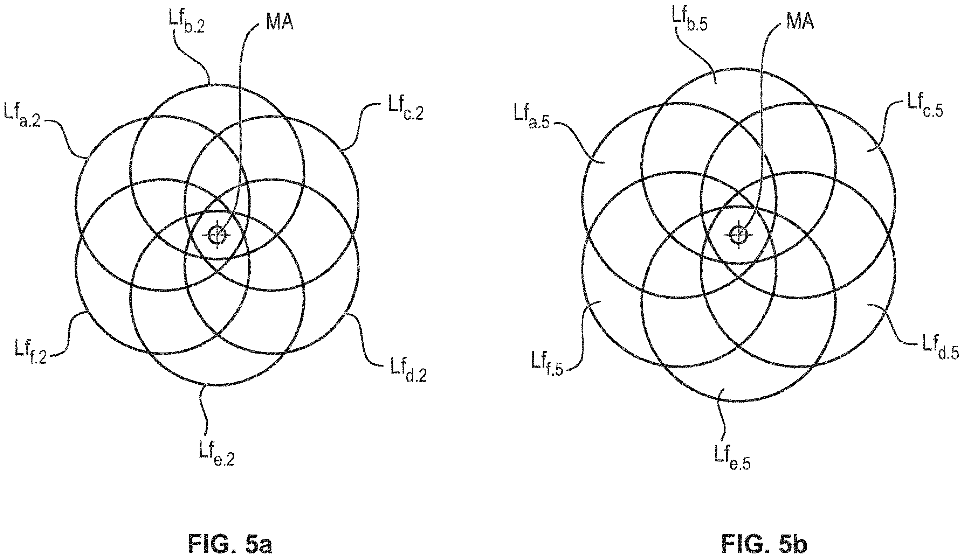

Figur 5, Figur 6 und Figur 7 zeigen beispielhaft drei alternative Realisierungsformen, wie die sechs Lichtfelder der sechs Lichtquellen der Positionsgruppe Pos.5 relativ zu den sechs Lichtfeldern der Lichtquellen der Positionsgruppe Pos.2 positioniert sein können, vgl. Figur 4. Die Mittelachse MA der Beleuchtungsvorrichtung 100 steht senkrecht auf den Zeichenebenen von Figur 5, Figur 6 und Figur 7. Gezeigt werden jeweils die Lichtfeld-Durchmesser d₁₀ der Lichtfelder, welche die sechs Lichtquellen der Positionsgruppe Pos.2 erzeugen (links), und die Lichtfeld-Durchmesser d₁₀ der Lichtfelder, welche die sechs Lichtquellen der Positionsgruppe Pos.5 erzeugen (rechts).

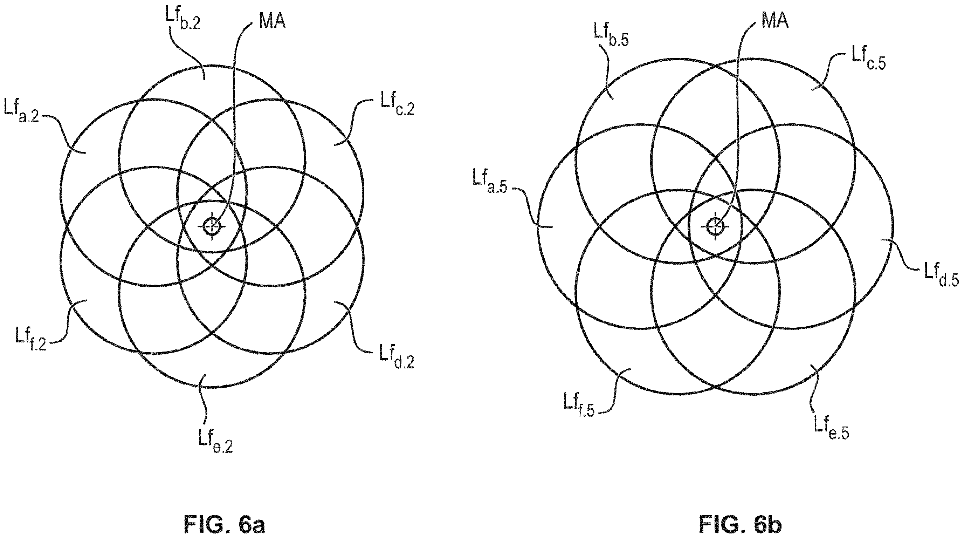

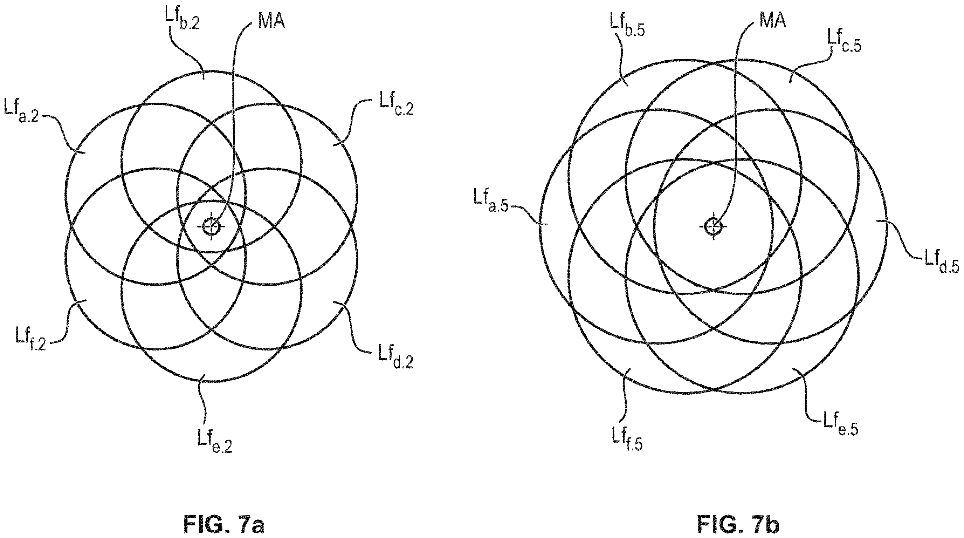

Im Beispiel von Figur 5 stimmen die sechs Lichtfelder paarweise miteinander überein, d.h. jedes Lichtfeld einer Lichtquelle der Positionsgruppe Pos.2 stimmt mit jeweils einem Lichtfeld einer Lichtquelle der Position Pos.5 hinsichtlich Form und Position überein, insbesondere hinsichtlich des Lichtfeld-Durchmessers. Im Beispiel von Figur 6 sind die Lichtfelder der Lichtquellen der Positionsgruppe Pos.5 relativ zu den sechs Lichtfeldern der Lichtquellen der Positionsgruppe Pos.2 um etwa 20 Grad im Uhrzeigersinn gedreht, aber nicht die gleiche Position auf der Oberfläche Ob. Genauer gesagt: Jedes Lichtfeld einer Lichtquelle der Positionsgruppe Pos.5 hat ein Lichtfeld den gleichen Form und insbesondere den gleichen Lichtfeld-Durchmesser wie ein Lichtfeld einer Lichtquelle der Positionsgruppe Pos.6, aber nicht die gleiche Position, sondern ist um die Mittelachse MA gedreht. Im Beispiel von Figur 7 weisen die sechs Lichtfelder der Lichtquellen der Positionsgruppe Pos.5 jeweils einen größeren Lichtfeld-Durchmesser auf als die sechs Lichtfelder der Lichtquellen der Positionsgruppe Pos.2. Außerdem sind die Lichtfelder der Lichtquellen der Positionsgruppe Pos.5 relativ zu den Lichtfeldern der Lichtquellen der Positionsgruppe Pos.2 um etwa 20 Grad verdreht. Selbstverständlich sind auch andere Situationen möglich. Das gemeinsame ist: Die Maxima der Lichtfelder der Lichtquellen der Positionsgruppe Pos.2 und die Maxima der Lichtfelder der Lichtquellen der Positionsgruppe Pos.5 liegen auf demselben Kreis um die optische Mittelachse MA herum.

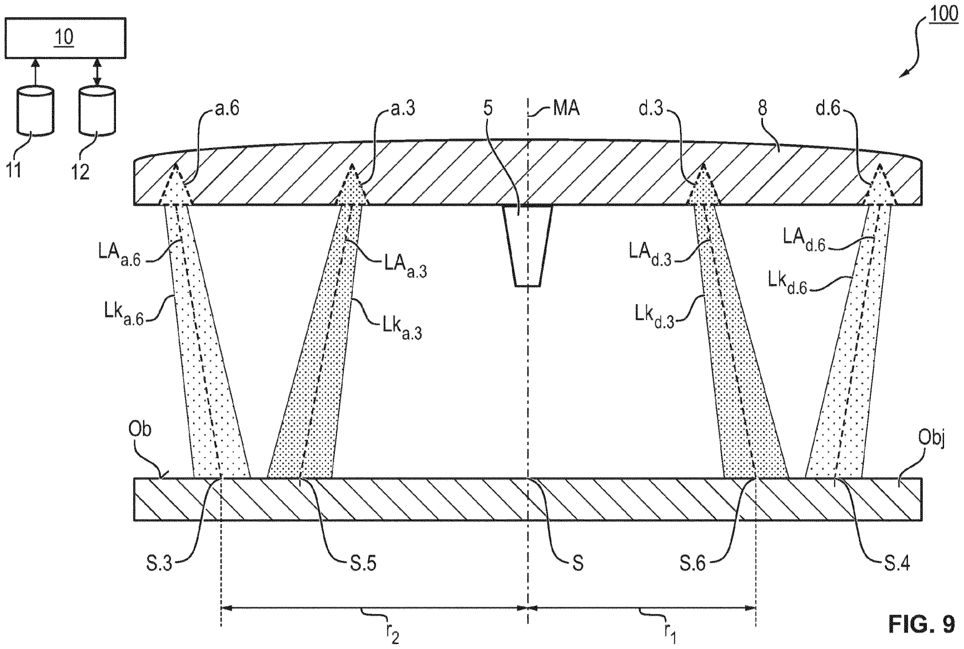

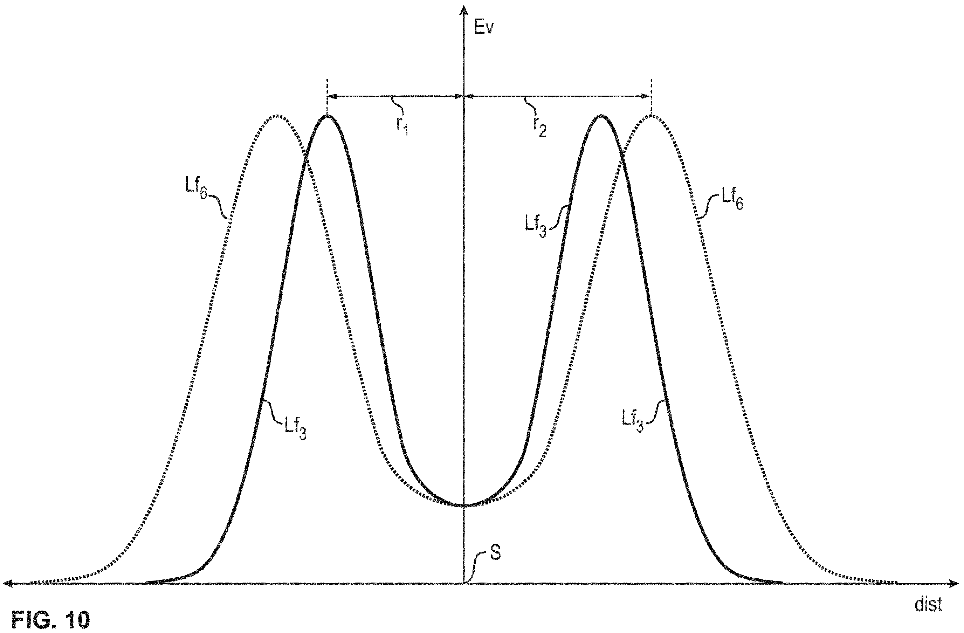

Figur 9 zeigt die vier Lichtachsen LA_{a.3}, LA_{a.6}, LA_{d.3}, LA_{d.6} und die vier Lichtkegel Lk_{a.3}, Lk_{a.6}, Lk_{d.3}, Lk_{d.6} der vier Lichtquellen a.3, a.6, d.3, d.6. Figur 10 zeigt die beiden Lichtfelder Lf₃ und Lf₆, welche die Lichtquellen der Positionsgruppen Pos.3 und Pos.6 zusammen erzeugen. Die Mittelachsen LA_{a.3} und LA_{d.3} schneiden die beleuchteten Oberfläche Ob in zwei Schnittpunkten S.5 und S.6, die von der Mittelachse MA einen Abstand von jeweils r₁ und einen Abstand voneinander von jeweils 2*r₁ aufweisen. Die Mittelachsen MA_{a.6} und MA_{d.6} schneiden die beleuchteten Oberfläche Ob ebenfalls in zwei Schnittpunkten S.3 und S.4, die von der Mittelachse MA einen Abstand von jeweils r₂ und einen Abstand voneinander von jeweils 2*r₂ aufweisen, vgl. Figur 9. Es gilt: r₂ > r₁.

Insgesamt werden in Figur 9 vier verschiedene und voneinander beabstandete Schnittpunkte S.3, ..., S.6 gezeigt.

Das Lichtfeld Lf₃ wird konzentrisch vom Lichtfeld Lf₆ umgeben. Das Maximum des Lichtfelds Lf₃ hat einen Abstand von r₁ zur Mittelachse MA, das Maximum des Lichtfelds Lf₆ einen Abstand von r2. Die beiden Positionsgruppen Pos.3 und Pos.6 sind nicht redundant, weil sie unterschiedliche Lichtfelder Lf₃ bzw. Lf₆ erzeugen. Diese beiden Lichtfelder Lf₃ und Lf₆ werden in Figur 10 gezeigt. Im gezeigten Beispiel sind die Positionsgruppen Pos.3 und Pos.6 auch nicht teil-redundant, weil die Maxima auf unterschiedlichen Kreisringen liegen.

Im Ausführungsbeispiel umfasst die Beleuchtungsvorrichtung 100 mindestens einen, bevorzugt mehrere Abstandsmesser. Der oder jeder Abstandsmesser vermag berührungslos den Abstand zwischen sich selbst und einer Licht streuenden Oberfläche zu messen. Bevorzugt emittiert der oder jeder Abstandsmesser jeweils elektromagnetische Strahlung auf die beleuchtete Oberfläche Ob zu, bevorzugt im Infrarot-Bereich, optional im Ultraviolett-Bereich. Ein Teil der gestreuten Strahlung erreicht den Abstandsmesser, und der Abstandsmesser misst die Laufzeit und leitet hieraus die Entfernung ab. Beispielhaft werden in Figur 1 ein mittiger Abstandsmesser dm sowie jeweils ein Abstandsmesser dm.2, ..., dm.6 für jede Positionsgruppe Pos.2, ..., Pos.6 gezeigt. Auch andere Prinzipien zur berührungslosen Entfernungsmessung sind möglich.

Der mittige Abstandsmesser dm sowie der oder jeder weitere Abstandsmesser dm.2, ..., dm.6 sind im Ausführungsbeispiel fest am Träger 8 befestigt, und daher sind die Position und Orientierung eines Abstandsmessers dm, dm.2, ..., dm.6, ... relativ zum Träger 8 unveränderlich. In dem oben erwähnten Datenspeicher 11 sind die jeweilige Position und Orientierung jedes Abstandsmessers am Träger 8 abgespeichert. Wie bereits erwähnt, hat das Steuergerät 10 Lesezugriff auf diesen Datenspeicher 11.

In einer bevorzugten Ausgestaltung vermag der oder mindestens ein Abstandsmesser der Beleuchtungsvorrichtung 100 ein topologisches Profil der beleuchteten Oberfläche Ob zu erzeugen. Eine Implementierung eines derartigen Abstandsmessers ist unter der Bezeichnung "Time-of-Flight-Sensor" bekannt geworden. Auch Laserscanner und 3D-Kamerasysteme sind in manchen Implementierungen dafür geeignet, jeweils ein topologisches Profil zu erzeugen. Möglich ist auch, dass eine Mehrzahl von Abstandsmessern zusammen Signale erzeugt, aus denen das Steuergerät 10 ein topologisches Profil ermittelt.

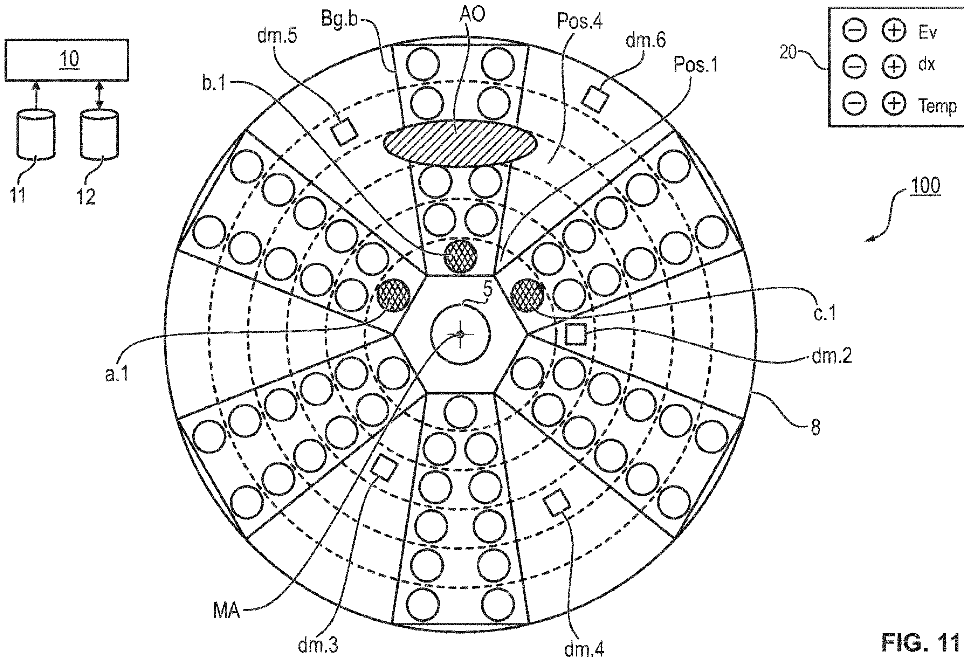

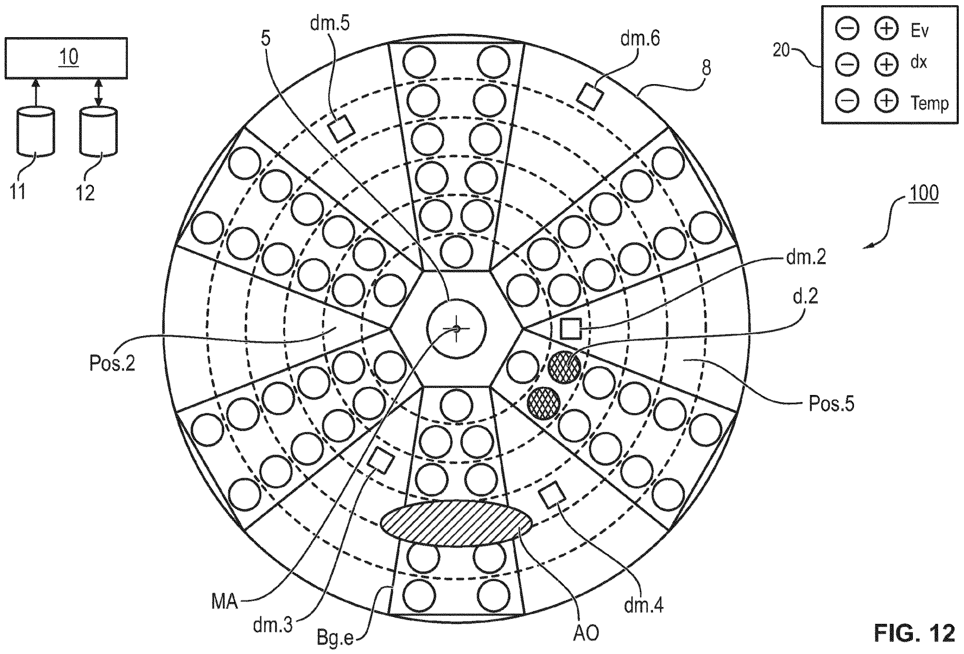

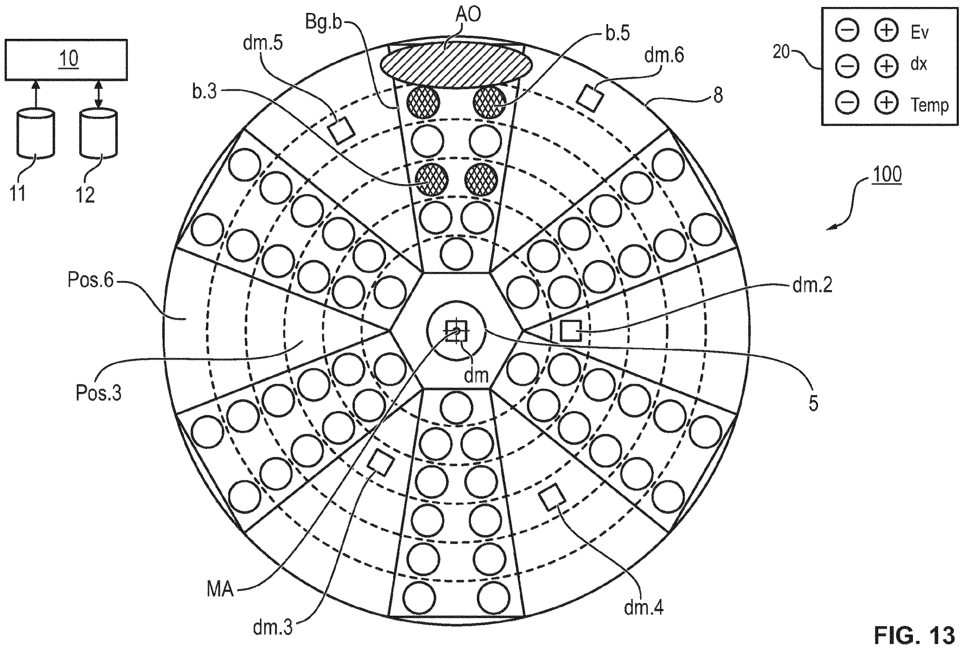

Möglich ist, dass ein Objekt in den Bereich zwischen der Beleuchtungsvorrichtung 100 und der beleuchteten Oberfläche Ob, hier also dem Patienten auf dem Operationstisch, gelangt. Dieses Objekt kann insbesondere ein Körperteil eines behandelnden Arztes oder ein medizinisches Instrument sein. Das Objekt bewirkt eine Abschattung. In der Regel gelangt das abschattende Objekt in den jeweiligen Lichtkegel mindestens einer Lichtquelle, so dass die Gesamt-Beleuchtungsstärke in einigen Bereichen des erzielten Gesamt-Lichtfelds reduziert wird. Figur 11, Figur 12 und Figur 13 zeigen aus der Betrachtungsrichtung von Figur 1 drei Beispiele für jeweils eine Positionierung eines abschattenden Objekts AO. Das abschattenden Objekt AO wird nur schematisch gezeigt. Häufig ist in der Realität ein abschattendes Objekt größer als das hier gezeigte.

Weil die Beleuchtungsvorrichtung 100 viele (hier: 66) verschiedene Lichtquellen aufweist und weil die Lichtachsen dieser Lichtquellen nicht alle parallel zueinander angeordnet sind, bewirkt eine solche Abschattung in der Regel nicht, dass auf der beleuchteten Oberfläche Ob ein scharfer Schlagschatten gebildet wird oder gar ein zuvor beleuchteter Bereich der Oberfläche Ob überhaupt nicht mehr beleuchtet wird. Trotzdem ist die Abschattung unerwünscht. Das Steuergerät 10 bewirkt automatisch, dass die Abschattung wenigstens teilweise kompensiert wird. Wie dieses Ziel erreicht wird, wird nachfolgend beschrieben.

Das Steuergerät 10 empfängt Signale von den Abstandsmessern dm, dm.1, ..., dm.6 und wertet diese Signale aus. Nachfolgend wird abkürzend von "dem Signal der Abstandsmess-Anordnung" gesprochen. Das Steuergerät 10 stellt automatisch fest, welche Lichtquellen der Beleuchtungsvorrichtung 100 abgeschattet sind. Hierfür wird die Annahme verwendet, dass ein abschattendes Objekt AO die auftreffende elektromagnetische Strahlung und damit Licht im sichtbaren Bereich streut und nicht transmittiert. Daher bewirkt das abschattende Objekt AO, dass der gemessene Abstand zwischen mindestens einem Abstandsmesser dm, dm.1, ..., dm.6 und der Licht streuenden Oberfläche sich verringert, in der Regel schlagartig verringert.

In einer Ausgestaltung berücksichtigter Steuergerät 10 nur solche Werte für den Abstand zwischen einem Abstandsmesser und einem Licht streuenden Objekt, die in einem vorgegebenen Wertebereich liegen, wobei dieser Wertebereich ein Teil des Intervalls zwischen 0m und dem Referenzabstand ist. Beispielsweise beträgt der Referenzabstand 1m, und dieser Wertebereich ist der Bereich zwischen 0,2m und 0,8m. Ein abschattendes Objekt hat in der Regel einen Abstand zur Beleuchtungsvorrichtung 100, die in diesem Wertebereich liegt, während in der Regel sowohl der Operationstisch Ob als auch der Patient auf dem Operationstisch Ob einen größeren Abstand aufweisen.

Bevorzugt ermittelt das Steuergerät 10 wiederholt, beispielsweise mit einer vorgegebenen konstanten Abtastfrequenz, welche Lichtquellen aktuell abgeschattet sind. Selbstverständlich ist es möglich, dass aktuell keine Lichtquelle abgeschattet ist.

Bevorzugt ermittelt das Steuergerät 10 wenigstens näherungsweise die Kontur eines abschattenden Objekts AO und die Position dieser Kontur in einer Ebene, die senkrecht auf der optischen Mittelachse MA steht. Diese Ebene hat bevorzugt den Referenzabstand zur Beleuchtungsvorrichtung 100. Die ermittelte Kontur liegt näherungsweise fest, welcher Bereich der beleuchteten Oberfläche durch das Objekt AO abgeschattet wird, also den Abschattungs-Bereich. Möglich ist, dass diese Kontur näherungsweise durch ein Rechteck oder einen Kreis oder eine sonstige geeignete geometrische Figur beschrieben wird, wobei diese geometrische Figur in einer Ebene liegt, die im Referenzabstand senkrecht auf der optischen Mittelachse MA steht. Um die Kontur zu ermitteln, verwendet das Steuergerät 10 das Signal von der Abstandsmess-Anordnung. Wie bereits dargelegt, misst jeder Abstandsmesser mindestens den Abstand zwischen sich selbst und der beleuchteten Oberfläche Ob oder einem Licht streuenden Objekt AO. Optional ermittelt das Steuergerät 10 ein topologisches Profil der beleuchteten Oberfläche. Das abschattende Objekt AO ragt aus der beleuchteten Oberfläche Ob hervor, hat also einen kleineren Abstand zur Beleuchtungsvorrichtung 100 als die beleuchtete Oberfläche Ob.

Wie bereits oben erwähnt, hat im Ausführungsbeispiel das Steuergerät 10 wenigstens zeitweise Lesezugriff auf einen Datenspeicher 11, in dem
- für jede Lichtquelle die jeweilige maximal mögliche Einzel-Beleuchtungsstärke, die Position und Orientierung der Lichtachse relativ zur Mittelachse MA, der Lichtdivergenzwinkel und die korrelierte Farbtemperatur sowie
- für jeden Abstandsmesser dm, dm.2, ..., dm.6 die Position und Orientierung der Betrachtungsrichtung (Messrichtung) dieses Abstandsmessers relativ zur Mittelachse MA
abgespeichert sind. Aus den gemessenen Abständen und den gerade beschriebenen Informationen, die im Datenspeicher 11 abgespeichert sind, leitet das Steuergerät 10 die Informationen her, welche Lichtquellen a.1, ..., a.6, b.1, ..., b.6, ... aktuell abgeschattet sind. In einer Ausgestaltung wird eine Lichtquelle als abgeschattet bewertet, wenn das Objekt AO in die Lichtachse dieser Lichtquelle gelangt, in einer anderen Ausgestaltung, wenn das Objekt AO in den Lichtkegel (Lichtfeld-Durchmesser d₁₀) dieser Lichtquelle gerät, vgl. Figur 2, Figur 4 und Figur 9.

Wie bereits dargelegt, können ein Benutzer oder auch eine übergeordnete Steuerung Vorgaben für die Beleuchtungsvorrichtung 100 geben, insbesondere Vorgaben für die folgenden Parameter:
- die maximale Gesamt-Beleuchtungsstärke,
- den Lichtfeld-Durchmesser dₓ, bevorzugt d₁₀, und
- die korrelierte Farbtemperatur.

Das Steuergerät 10 kompensiert die Abschattung automatisch mit dem Ziel, dass die gerade genannten Parameter nach der Kompensation jeweils den gleichen Wert aufweisen wie vor der Abschattung. In vielen Fällen kann dieses Ziel nicht oder wenigstens nicht ideal erreicht werden. Daher wendet das Steuergerät 10 die folgende Abstufung unter den Zielen an:
- Das oberste Ziel ist, dass die maximale Gesamt-Beleuchtungsstärke der Beleuchtungsvorrichtung 100 gleichbleibt. Außerdem soll der Punkt, in dem diese maximale Gesamt-Beleuchtungsstärke erzielt wird, der gleiche bleiben. In der Regel ist dies der Schnittpunkt S.
- Ein Ziel mittlerer Bedeutung ist, dass zusätzlich der Lichtfeld-Durchmesser dₓ der Beleuchtungsvorrichtung 100 unverändert bleibt. Mindestens ist das Ziel, dass der Lichtfeld-Durchmesser dₓ für x gleich 10 % konstant bleibt, optional zusätzlich für x = 50 % oder einen anderen Wert für x.
- Ein Ziel geringerer Priorität kann sein, dass das Lichtfeld der Beleuchtungsvorrichtung 100 unverändert bleibt.
- Ein Ziel mit noch geringerer Priorität ist, dass zusätzlich die korrelierte Farbtemperatur der Beleuchtungsvorrichtung 100 gleich bleibt oder wenigstens eine Veränderung nicht für einen Menschen wahrnehmbar ist.

Anmerkung: In vielen Fällen weist jede Lichtquelle eine von zwei möglichen korrelierten Farbtemperaturen auf, beispielsweise warmweiß und kaltweiß. Kaltweiße und warmweiße Lichtquellen sind gleichmäßig über den Träger 3 verteilt. Daher bewirkt in vielen Fällen eine Abschattung, dass die korrelierte Farbtemperatur der Beleuchtungsvorrichtung 100 nicht in einer von einem Menschen wahrnehmbaren Weise verändert wird. Daher ist häufig das letzte Ziel von alleine erreicht, wenn die ersten beiden Ziele erreicht sind, ohne dass es einer speziellen Ansteuerung bedarf.

Eine Randbedingung ist, dass die Kompensation der Abschattung wenig Rechenzeit in Anspruch nehmen soll. Genauer gesagt: Die Kompensation der Abschattung soll innerhalb einer Zeitspanne mit einer vorgegebenen maximalen Dauer durchgeführt werden. Bevorzugt soll außerdem die Kompensation relativ wenig Rechenleistung erfordern, damit ein relativ einfacher Chip oder Prozessor verwendet werden kann. Das Steuergerät 10 führt die Kompensation der Abschattung wiederholt durch, beispielsweise mit der gerade erwähnten Abtastfrequenz.

Bevorzugt bewirkt das Steuergerät 10, dass eine abgeschattete Lichtquelle ausgeschaltet wird und so lange ausgeschaltet bleibt, wie die Lichtquelle abgeschattet ist. Diese Ausgestaltung spart einerseits Energie und Wärmeeintrag in ein beleuchtetes Objekt, insbesondere in ein abschattendes Objekt, und andererseits Rechenzeit ein verglichen mit einer Ausgestaltung, bei der einer abgeschattete Lichtquelle mit reduzierter maximaler Einzel-Beleuchtungsstärke eingeschaltet bleibt.

Im Ausführungsbeispiel werden die maximale Gesamt-Beleuchtungsstärke, der Lichtfeld-Durchmesser dₓ und die korrelierte Farbtemperatur der Beleuchtungsvorrichtung 100 nicht gemessen, sondern vom Steuergerät 10 berechnet. Wie bereits erwähnt, entstehen die maximale Gesamt-Beleuchtungsstärke, der Lichtfeld-Durchmesser dₓ und die korrelierte Farbtemperatur der Beleuchtungsvorrichtung 100 durch eine Überlagerung der Lichtfelder der Lichtquellen - genau gesagt: derjenigen Lichtquellen, die aktuell eingeschaltet sind.

Das Steuergerät 10 ermittelt durch einen Lesezugriff auf den oben erwähnten Datenspeicher 11 die jeweilige Position und Orientierung der Lichtachse, die maximal mögliche Einzel-Beleuchtungsstärke; den Lichtfeld-Durchmesser dₓ und die korrelierte Farbtemperatur jeder Lichtquelle, wobei diese Parameter in einer Realisierungsform unveränderlich bleiben, sowie durch eine Lesezugriff auf den Datenspeicher 12 die aktuelle maximale Einzel-Beleuchtungsstärke jeder Lichtquelle. Das Steuergerät 10 vermag mindestens die jeweilige aktuelle maximale Einzel-Beleuchtungsstärke jeder Lichtquelle zu verändern, beispielsweise bis zur maximal möglichen Einzel-Beleuchtungsstärke zu vergrößern. In einer anderen Realisierungsform vermag das Steuergerät 10 zusätzlich einen Stellantrieb für eine Lichtquelle anzusteuern und dadurch die Orientierung der Lichtachse dieser Lichtquelle relativ zur Mittelachse MA zu verändern.

Denkbar wäre es, dass die Steuergerät 10 Lesezugriff auf eine in rechnerauswertbare Form vorliegende Tabelle aufweist. Diese Tabelle enthält jeweils einen Eintrag für jede mögliche Abschattung von Lichtquellen der Beleuchtungsvorrichtung 100 und einen resultierenden Stelleingriff, der diese Abschattung kompensiert und die oben genannten Ziele einhält. Bei N Lichtquellen müsste diese Tabelle aber theoretisch 2^N verschiedene Einträge aufweisen, damit für jede mögliche Abschattung ein passender Eintrag vorliegt. Bei 66 Lichtquellen wären dies 7*10^19 Einträge. Eine so große Tabelle lässt sich in vielen Fällen nicht rasch genug auswerten. Daher wendet das Steuergerät 10 abkürzende Heuristiken an.

Nachfolgend werden die Begriffe "redundant" und "teil-redundant" verwendet, und zwar sowohl für einzelne Lichtquellen als auch für Positionsgruppen.

Zwei Lichtquellen sind zueinander redundant, wenn sie auf einer beleuchteten Oberfläche Ob - bis auf eine Toleranz - dasselbe Lichtfeld erzeugen. Diese Oberfläche Ob steht senkrecht auf der Mittelachse MA der Beleuchtungsvorrichtung 100 und befindet sich im Referenzabstand von der Beleuchtungsvorrichtung 100. Zwei Positionsgruppen sind zueinander redundant, wenn ihre Lichtquellen zusammen dasselbe Lichtfeld erzeugen und es zu jeder Lichtquelle der einen Positionsgruppe jeweils eine redundante Lichtquelle der anderen Positionsgruppe gibt. Im Beispiel von Figur 5 sind die beiden Positionsgruppen Pos.2 und Pos.5 zueinander redundant.

Zwei Lichtquellen sind zueinander teil-redundant, wenn sich ihre Lichtfelder zwar überlappen, die Lichtfelder aber nicht identisch sind und / oder die Lichtquellen unterschiedliche korrelierte Farbtemperaturen aufweisen. Insbesondere können die Lichtfelder sich hinsichtlich Geometrie und / oder maximaler Einzel-Beleuchtungsstärke und / oder Lichtfeld-Durchmesser unterscheiden. Entsprechend sind zwei Positionsgruppen zueinander teil-redundant, wenn sie zwar auf der beleuchteten Oberfläche dasselbe Lichtfeld erzeugen und es zu jeder Lichtquelle der einen Positionsgruppe mindestens eine teil-redundante Lichtquelle der anderen Positionsgruppe gibt, aber keine redundante.

Das Steuergerät 10 versucht zunächst, jede abgeschattete Lichtquelle durch jeweils eine nicht abgeschattete Lichtquelle zu ersetzen, wobei die ErsatzLichtquelle redundant zur abgeschatteten Lichtquelle ist. Falls n Lichtquellen abgeschattet sind, werden bei diesem Vorgehen zur Kompensation mindestens n nicht abgeschattete und redundante Lichtquellen benötigt. Eine Lichtquelle y ist zu einer Lichtquelle x redundant, wenn die Lichtachsen LAₓ und LA_{y} der beiden Lichtquellen die beleuchtete Oberfläche Ob im gleichen Punkt schneiden, das gleiche Lichtfeld Lfₓ und Lf_{y} und daher den gleichen Lichtfeld-Durchmesser und die gleiche korrelierte Farbtemperatur aufweisen. Anmerkung: Im Ausführungsbeispiel wird eine teilweise abgeschattete Lichtquelle ausgeschaltet, bis die Abschattung wieder aufgehoben ist.

Das Steuergerät 10 verwendet die Informationen, welche Lichtquelle zu welcher Positionsgruppe gehört. Zu jeder Lichtquelle x.4 der Positionsgruppe Pos.4 gibt es jeweils eine redundante Lichtquelle a.1, ..., f.1 der Positionsgruppe Pos.1, vgl. Figur 2 und Figur 3. Falls also eine Lichtquelle der Positionsgruppe Pos.4 abgeschattet ist, ermittelt das Steuergerät 10 die nicht abgeschatteten Lichtquellen der Positionsgruppe Pos.1 und umgekehrt. Nur in der Positionsgruppe Pos.1, aber nicht in den übrigen Positionsgruppen findet sich keine Lichtquelle, die zu einer abgeschatteten Lichtquelle der Positionsgruppe Pos.4 redundant ist. Falls die Lichtquellen der beiden Positionsgruppen Pos.2 und Pos.5 so wie in Figur 5 gezeigt ausgestaltet sind, so gibt es zu jeder Lichtquelle der Positionsgruppe Pos.2 eine redundante Lichtquelle der Positionsgruppe Pos.5 und umgekehrt.

Das Steuergerät 10 vermag die m redundanten Lichtquellen y.1, ..., y.m dergestalt anzusteuern, dass die m angesteuerten Lichtquellen y.1, ..., y.m zusammen eine größere maximale Einzel-Beleuchtungsstärke als zuvor erzielen. Dieser Schritt kompensiert aber nur dann die Abschattung der Lichtquelle x, wenn die Summe der vergrößerten aktuellen maximalen Einzel-Beleuchtungsstärken der m Lichtquellen y.1, ..., y.m so groß ist wie die Summe der aktuellen maximalen Einzel-Beleuchtungsstärken der m+1 Lichtquellen x und y.1, ..., y.m vor der Abschattung. Dies ist oft nicht möglich, insbesondere dann, wenn mehrere Lichtquellen gleichzeitig abgeschattet werden und / oder wenn einige der m nicht abgeschatteten redundanten Lichtquellen y.1, ..., y.m bereits vor der Abschattung die jeweils maximal mögliche Einzel-Beleuchtungsstärke erzielten und deren maximale Einzel-Beleuchtungsstärke daher nicht weiter vergrößert werden kann. Die maximal mögliche Einzel-Beleuchtungsstärke, die eine Lichtquelle erzielen kann, wird insbesondere von der maximalen Stromstärke, der durch diese Lichtquelle fließen darf, begrenzt.

Falls eine abgeschattete Lichtquelle nicht oder nicht vollständig durch mindestens eine redundante und nicht abgeschattete Lichtquelle kompensiert werden kann, sucht das Steuergerät 10 durch das folgende schrittweise Vorgehen nach mindestens einer anderen Lichtquelle, um die Abschattung zu kompensieren und hierbei die oben genannten drei Ziele mit der dort genannten Priorität einzuhalten. Falls ein Schritt zu einer vollständigen oder wenigstens ausreichenden Kompensierung der Abschattung führt, wird der nächste Schritt nicht durchgeführt.

Als ersten Schritt sucht das Steuergerät 10 nach Lichtquellen, die zu einer abgeschatteten Lichtquelle teil-redundant sind. Zwei verschiedene Positionsgruppen sind teil-redundant, wenn sie zwar zusammen dasselbe Lichtfeld erzeugen, aber nicht jede Lichtquelle der einen Positionsgruppe zu jeweils einer Lichtquelle der anderen Positionsgruppe redundant ist. Beispiele für redundante und nur teil-redundante Lichtquellen werden in Figur 6 und Figur 7 gezeigt. Die beiden Positionsgruppen Pos.2 und Pos.5 erzeugen zwar das gleiche Lichtfeld, vgl. Figur 8. Jedoch gibt es zu einer Lichtquelle der Positionsgruppe Pos.2 keine redundante Lichtquelle der Positionsgruppe Pos.5 und umgekehrt.

Falls in der Situation von Figur 6 und Figur 7 eine Lichtquelle der Positionsgruppe Pos.2 abgeschattet ist, sucht das Steuergerät 10 nach denjenigen Lichtquellen der Positionsgruppen Pos.2 oder Pos.5, die zur abgeschatteten Lichtquelle teil-redundant sind und selber nicht abgeschattet sind. Falls beispielsweise die Lichtquelle a.2 abgeschattet ist, so ermittelt das Steuergerät 10 die beiden Lichtquellen b.2 und f.2 der Positionsgruppe Pos.2 und die beiden Lichtquellen a.5 und b.5 der Positionsgruppe Pos.5. Das Steuergerät 10 sucht nach mindestens einer Lichtquelle, die selber nicht abgeschattet ist und noch nicht mit der maximalen Einzel-Beleuchtungsstärke betrieben wird. Falls das Steuergerät 10 eine solche Lichtquelle detektiert, so bewirkt das Steuergerät 10, dass die maximale Einzel-Beleuchtungsstärke dieser Lichtquelle auf die maximal mögliche Einzel-Beleuchtungsstärke vergrößert wird. Falls das Steuergerät 10 mehrere solcher Lichtquellen findet, so werden bevorzugt die maximalen Einzel-Beleuchtungsstärken aller dieser Lichtquellen vergrößert.

Der zweite Schritt wird durchgeführt, wenn die vorherigen Schritte nicht zu einer ausreichenden Kompensierung der Abschattung geführt haben. Das Steuergerät 10 sucht im zweiten Schritt nach jeder Lichtquelle y, die kumulativ folgende Eigenschaften erfüllt:
- Die Lichtquelle y gehört zu derselben Positionsgruppe wie die abgeschattete Lichtquelle x.
- Die Lichtquelle y ist nicht abgeschattet.
- Die Lichtquelle y wird aktuell nicht mit der maximal möglichen Einzel-Beleuchtungsstärke betrieben.

Falls diese Voraussetzung erfüllt ist, so bewirkt das Steuergerät 10, dass die maximale Einzel-Beleuchtungsstärke der Lichtquelle y vergrößert wird, bevorzugt bis zur maximal möglichen Einzel-Beleuchtungsstärke.

Falls auch dies nicht zu einer ausreichenden Kompensierung der Abschattung führt, so führt das Steuergerät 10 einen dritten Schritt aus und sucht im dritten Schritt nach jeder Lichtquelle y, die kumulativ folgende Eigenschaften erfüllt:
- Die Lichtquelle y gehört zu einer Positionsgruppe, die zur Positionsgruppe der abgeschatteten Lichtquelle x teil-redundant ist.
- Die Lichtquelle y ist nicht abgeschattet.
- Die Lichtquelle y wird aktuell nicht mit der maximal möglichen Einzel-Beleuchtungsstärke betrieben.

Wiederum bewirkt das Steuergerät 10, dass die maximale Einzel-Beleuchtungsstärke dieser Lichtquelle y vergrößert wird.

Falls auch der dritte Schritt nicht zu einer ausreichenden Kompensierung der Abschattung führt, wählt das Steuergerät 10 Lichtquellen aus, die aktuell nicht abgeschattet sind und zu einer weiteren Positionsgruppe gehören, beispielsweise zu einer Positionsgruppe, die benachbart zu der Positionsgruppe einer abgeschatteten Lichtquelle ist. Das Steuergerät 10 bewirkt, dass die maximale Einzel-Beleuchtungsstärke mindestens einer ausgewählten Lichtquelle vergrößert wird.

### Bezugszeichenliste

| | |
|---|---|
| 5 | Griff am Träger 8, ermöglicht es , die Beleuchtungsvorrichtung 100 im Raum zu bewegen und zu orientieren |
| 8 | Träger der Beleuchtungsvorrichtung 100, trägt die Lichtquellen a.1, ..., a.6, b.1, ..., b.6, ..., den Griff 5 und die Abstandsmesser dm, dm.1, ..., dm.6 |
| 10 | signalverarbeitendes Steuergerät (control unit), empfängt Signale von den Abstandsmessern dm, dm.2, ..., dm.6, verwendet diese Signale als das Signal der Abstandsmess-Anordnung, steuert die Lichtquellen a.1, ..., a.6, b.1, ..., b.6, ... der Beleuchtungsvorrichtung 100 an, hat wenigstens zeitweise Lesezugriff auf die Datenspeicher 11 und 12 und Schreibzugriff auf den Datenspeicher 12 |
| 11 | Datenspeicher, in dem unveränderliche Informationen über die Lichtquellen a.1, ..., a.6, b.1, ..., b.6, ... abgespeichert sind |
| 12 | Datenspeicher, in dem veränderbare Informationen über die Lichtquellen a.1, ..., a.6, b.1, ..., b.6, ... abgespeichert sind |
| 20 | Eingabeeinheit, mit deren Hilfe ein Benutzer die maximale Gesamt-Beleuchtungsstärke, den Lichtfeld-Durchmesser und die korrelierte Farbtemperatur verändern kann |
| 100 | Beleuchtungsvorrichtung, umfasst die Lichtquellen a.1, ..., a.6, b.1, ..., b.6, ..., den Träger 8, den Griff 5, die Eingabeeinheit 20, das Steuergerät 10 und die Abstandsmesser dm, dm.2, ..., dm.6, ist rotationssymmetrisch zur optischen Mittelachse MA |
| a.1, ..., a.6, b.1, ..., b.6, ... | einzelne Lichtquellen der Beleuchtungsvorrichtung 100 |
| AO | abschattendes Objekt |
| Bg.a, ..., Bg.e | Baugruppen der Beleuchtungsvorrichtung 100, umfassen jeweils elf Lichtquellen a.1, ..., a.6, b.1, ..., b.6, ... |
| dm | mittiger Abstandsmesser, misst den Abstand zwischen der Beleuchtungsvorrichtung 100 und dem beleuchteten Objekt entlang der Mittelachse MA |
| dm.x | Abstandmesser der Positionsgruppe Pos.x (x=2,...,6) |
| LA_{a.4} | optische Lichtachse der Lichtquelle a.4 |
| LA_{d.2} | optische Lichtachse der Lichtquelle d.2 |
| Lf.1 | Lichtfeld, welches die Lichtquellen der Positionsgruppe Pos.1 zusammen erzeugen |
| Lf.2 | Lichtfeld, welches die Lichtquellen der Positionsgruppe Pos.2 zusammen erzeugen |
| Lf_{a.4} | kreisförmiges Lichtfeld der Lichtquelle a.4 auf der beleuchteten Oberfläche Ob |
| Lf_{d.2} | kreisförmiges Lichtfeld der Lichtquelle d.2 auf der beleuchteten Oberfläche Ob |
| Lk_{a.4} | Lichtkegel der Lichtquelle a.4 |
| Lk_{d.2} | Lichtkegel der Lichtquelle d.2 |
| MA | optische Mittelachse der Beleuchtungsvorrichtung 100 |
| Ob | zur Beleuchtungsvorrichtung 100 zeigende und daher beleuchtete Oberfläche des Objekts Obj |
| Obj | beleuchtetes Objekt, hier: Patient auf dem Operationstisch, dessen zur Beleuchtungsvorrichtung 100 zeigende Oberfläche Ob von der Beleuchtungsvorrichtung 100 beleuchtet wird |
| Pos.1 | Positionsgruppe der Beleuchtungsvorrichtung 100, umfasst die Lichtquellen a.1, b.1, ..., f.1 |
| Pos.2, ..., Pos.6 | weitere Positionsgruppen der Beleuchtungsvorrichtung 100, umfasst die Lichtquellen a.2, ..., f.2 bzw. a.3, .., f.3 bzw.... sowie jeweils mindestens einen Abstandsmesser dm.2 bzw. dm.3 bzw.... |
| r | übereinstimmender Abstand auf der beleuchteten Oberfläche zwischen der Mittelachse MA einerseits und den Lichtachsen der Lichtquellen der Positionsgruppen Pos.2 und Pos.5 andererseits |
| r₁ | Abstand auf der beleuchteten Oberfläche Ob zwischen der Mittelachse MA einerseits und den Lichtachsen LA_{a.3} und LA_{d.3} der Lichtquellen der Positionsgruppe Pos.3 andererseits |
| r2 | Abstand auf der beleuchteten Oberfläche Ob zwischen der Mittelachse MA einerseits und den Lichtachsen LA_{a.6} und LA_{d.6} der Lichtquellen der Positionsgruppe Pos.6 andererseits |
| S | Schnittpunkt der optischen Mittelachse MA mit der beleuchteten Oberfläche Ob |
| S.1 | gemeinsamer Schnittpunkt der optischen Lichtachsen LA_{a.5} und LA_{a.2} mit der beleuchteten Oberfläche Ob |
| S.2 | gemeinsamer Schnittpunkt der optischen Lichtachsen LA_{d.5} und LA_{d.2} mit der beleuchteten Oberfläche Ob |

## Patentansprüche

1. Beleuchtungsvorrichtung (100) zur Beleuchtung einer Oberfläche (Ob),
wobei die Beleuchtungsvorrichtung (100)
- eine Lichtquellen-Menge mit mehreren Lichtquellen (a.1, a.2, ..., b.1, b.2, ...),
- eine Abstandsmess-Anordnung mit mehreren voneinander beabstandeten Abstandsmessern (dm, dm.2, dm.3, ...) und
- ein signalverarbeitendes Steuergerät (10)
umfasst,
wobei die Beleuchtungsvorrichtung (100)
- eine optische Mittelachse (MA) aufweist und
- dazu ausgestaltet ist, auf der beleuchteten Oberfläche (Ob) in einem Maximal-Bereich eine maximale Gesamt-Beleuchtungsstärke zu erzielen,
wobei jede Lichtquelle (a.1, a.2, ..., b.1, b.2, ...) der Lichtquellen-Menge jeweils
- eine Lichtachse (Lf_{a.1}, Lf_{a.2}, ...) aufweist und
- dazu ausgestaltet ist, auf der beleuchteten Oberfläche (Ob) jeweils ein Lichtfeld zu erzeugen,
wobei jede Lichtquelle (a.1, a.2, ..., b.1, b.2, ...) jeweils eine maximal mögliche Einzel-Beleuchtungsstärke des erzeugten Lichtfelds aufweist,
wobei die maximale Einzel-Beleuchtungsstärke einer Lichtquelle (a.1, a.2, ..., b.1, b.2, ...) der Lichtquellen-Menge unabhängig von der jeweiligen maximalen Einzel-Beleuchtungsstärke jeder anderen Lichtquelle auf einen Wert einstellbar ist, der zwischen Null und der maximal möglichen Einzel-Beleuchtungsstärke dieser Lichtquelle (a.1, a.2, ..., b.1, b.2, ...) liegt,
wobei das Steuergerät (10) dazu ausgestaltet ist, für jede Lichtquelle (a.1, a.2, ..., b.1, b.2, ...) der Lichtquellen-Menge jeweils vorgegebene Informationen zu erfassen,
wobei die erfassten oder erfassbaren Informationen die Informationen umfassen,
- welche maximal mögliche Einzel-Beleuchtungsstärke diese Lichtquelle (a.1, a.2, ..., b.1, b.2, ...) zu erzeugen vermag und
- wie die Lichtachse (Lf_{a.1}, Lf_{a.2}, ...) dieser Lichtquelle (a.1, a.2, ..., b.1, b.2, ...) relativ zur optischen Mittelachse (MA) der Beleuchtungsvorrichtung (100) positioniert und / oder orientiert ist,
wobei jeder Abstandsmesser (dm, dm.2, ..., dm.6) der Abstandsmess-Anordnung jeweils
- eine zur beleuchteten Oberfläche (Ob) zeigende Messrichtung aufweist und
- dazu ausgestaltet ist, ein Maß für den jeweiligen Abstand zwischen sich und einem Licht streuenden Objekt (Ob, AO) zu messen,
wobei das Steuergerät (10) dazu ausgestaltet ist, die jeweilige aktuelle Einzel-Beleuchtungsstärke jeder Lichtquelle (a.1, a.2, ..., b.1, b.2, ...) der Lichtquellen-Menge auf einen Wert einzustellen,
wobei das Steuergerät (10) weiterhin dazu ausgestaltet ist, eine Beleuchtungsstärken-Vorgabe zu erfassen, wobei die Beleuchtungsstärken-Vorgabe einen Sollwert für die maximale Gesamt-Beleuchtungsstärke vorgibt, welche die Beleuchtungsvorrichtung (100) erzeugen soll,
wobei das Steuergerät (10) weiterhin dazu ausgestaltet ist, abhängig von einem Signal der Abstandsmess-Anordnung
- zu prüfen, ob ein Abschattungs-Ereignis aufgetreten ist,
das ist ein Ereignis, dass sich mindestens ein Objekt (AO) zwischen der Beleuchtungsvorrichtung (100) und der beleuchteten Oberfläche (Ob) befindet, und
- nach der Detektion eines Abschattungs-Ereignisses wenigstens näherungsweise einen Abschattungs-Bereich zu ermitteln, das ist ein Bereich auf der beleuchteten Oberfläche (Ob), der durch das Objekt (AO) vollständig oder wenigstens teilweise abgeschattet wird, und
abhängig von dem ermittelten Abschattungs-Bereich nach mindestens einer zur Abschattungs-Kompensation geeigneten Lichtquelle der Lichtquellen-Menge zu suchen,
wobei eine zur Abschattungs-Kompensation geeignete Lichtquelle eine Lichtquelle (a.1, a.2, ..., b.1, b.2, ...) ist,
- die aktuell mit einem Wert für die maximale Einzel-Beleuchtungsstärke betrieben wird, die kleiner als die maximal mögliche Einzel-Beleuchtungsstärke dieser Lichtquelle ist,
- die überhaupt nicht oder wenigstens nicht vollständig abgeschattet ist und
- deren Lichtachse (Lf_{a.1}, Lf_{a.2}, ...) die beleuchtete Oberfläche (Ob) im ermittelten Abschattungs-Bereich schneidet,
wobei das Steuergerät (10) weiterhin dazu ausgestaltet ist, dann, wenn mindestens eine zur Abschattungs-Kompensation geeignete Lichtquelle gefunden ist,
abhängig von einer erfassten Beleuchtungsstärken-Vorgabe und von erfassten oder erfassbaren Informationen über die Lichtquellen (a.1, a.2, ..., b.1, b.2, ...) der Lichtquellen-Menge
- eine Teilmenge mit mindestens einer geeigneten Lichtquelle zu ermitteln,
- für jede Lichtquelle der ausgewählten Teilmenge jeweils einen Sollwert für die maximale Einzel-Beleuchtungsstärke dieser Lichtquelle zu berechnen,
wobei dieser Sollwert größer als der aktuelle Wert der maximalen Einzel-Beleuchtungsstärke dieser Lichtquelle ist, und
- zu bewirken, dass diese Lichtquelle den berechneten Sollwert für die maximale Einzel-Beleuchtungsstärke tatsächlich aktuell erzielt,
wobei zwei Ziele bei der Berechnung der Sollwerte für die maximalen Einzel-Beleuchtungsstärken der Lichtquellen der Teilmenge sind, dass
- die Position des Maximal-Bereichs relativ zur optischen Mittelachse (MA) unverändert bleibt und
- der tatsächliche Wert der maximalen Gesamt-Beleuchtungsstärke, die die Beleuchtungsvorrichtung (100) aktuell im Maximal-Bereich erzielt, gleich der erfassten Beleuchtungsstärken-Vorgabe ist.

2. Beleuchtungsvorrichtung (100) nach Anspruch 1,
**dadurch gekennzeichnet, dass**
die erfassbaren oder erfassten Informationen über die Lichtquellen für jede Lichtquelle (a.1, a.2, ..., b.1, b.2, ...) der Lichtquellen-Menge einen Lichtfeld-Durchmesser des von dieser Lichtquelle erzeugten Lichtfelds umfassen und
das Steuergerät (10) weiterhin dazu ausgestaltet ist, zusätzlich eine Lichtfeld-Durchmesser-Vorgabe zu erfassen, die einen Sollwert für den Gesamt-Lichtfeld-Durchmesser vorgibt, den das von der Beleuchtungsvorrichtung (100) erzeugte Lichtfeld aufweisen soll,
wobei das Steuergerät (10) weiterhin dazu ausgestaltet ist, die Sollwerte für die aktuellen maximalen Einzel-Beleuchtungsstärken der Lichtquellen der Teilmenge mit dem weiteren Ziel zu berechnen,
dass der tatsächliche Wert des Gesamt-Lichtfeld-Durchmessers des Lichtfelds, das die Beleuchtungsvorrichtung (100) erzielt, gleich der erfassten Lichtfeld-Durchmesser-Vorgabe ist.

3. Beleuchtungsvorrichtung (100) nach einem der vorhergehenden Ansprüche,
**dadurch gekennzeichnet, dass**
das Steuergerät (10) dazu ausgestaltet ist,
- nach einer Erfassung einer Beleuchtungsstärken-Vorgabe und optional einer Lichtfeld-Durchmesser-Vorgabe
für jede Lichtquelle (a.1, a.2, ..., b.1, b.2, ...) der Lichtquellen-Menge jeweils einen initialen Sollwert für die aktuelle maximale Einzel-Beleuchtungsstärke der Lichtquelle (a.1, a.2, ..., b.1, b.2, ...) zu berechnen und
- zu bewirken, dass der jeweilige tatsächliche Wert der maximalen Einzel-Beleuchtungsstärke, den eine Lichtquelle (a.1, a.2, ..., b.1, b.2, ...) der Lichtquellen-Menge erzielt, gleich dem berechneten initialen Sollwert für diese Lichtquelle (a.1, a.2, ..., b.1, b.2, ...) ist,
wobei der initiale Sollwert mindestens so lange verwendet ist, wie kein Abschattungs-Ereignis detektiert und keine weitere Beleuchtungsstärken-Vorgabe erfasst ist,
wobei das Steuergerät (10) weiterhin dazu ausgestaltet ist, nach einer Detektion eines Abschattungs-Ereignisses und dem Finden einer geeigneten Lichtquelle mindestens einmal eine Vergrößerungs-Abfolge durchzuführen,
wobei die oder jede Vergrößerungs-Abfolge die Schritte umfasst, dass das Steuergerät (10)
- eine Teilmenge mit mindestens einer zur Abschattungs-Kompensation geeigneten Lichtquelle der Lichtquellen-Menge ermittelt,
- jeweils einen Sollwert für die maximale Einzel-Beleuchtungsstärke der oder jeder Lichtquelle der ermittelten Teilmenge berechnet, wobei der Sollwert größer ist als die aktuelle maximale Einzel-Beleuchtungsstärke, und
- bewirkt, dass die jeweilige aktuelle maximale Einzel-Beleuchtungsstärke der oder jeder Lichtquelle der ermittelten Teilmenge auf den berechneten Sollwert vergrößert wird.

4. Beleuchtungsvorrichtung (100) nach Anspruch 3,
**dadurch gekennzeichnet, dass**
das Steuergerät (10) weiterhin dazu ausgestaltet ist,
bei der Durchführung mindestens einer Vergrößerungs-Abfolge, bevorzugt bei der Durchführung jeder Vergrößerungs-Abfolge,
nach den Schritten, die Teilmenge zu ermitteln, die jeweiligen Sollwerte zu berechnen und die Vergrößerung der jeweiligen Einzel-Beleuchtungsstärke zu bewirken,
- rechnerisch zu prüfen, ob die Position des Maximal-Bereichs relativ zur optischen Mittelachse (MA) trotz des Abschattungs-Ereignisses ausreichend wenig verändert ist und nunmehr der tatsächliche aktuelle Wert der maximalen Gesamt-Beleuchtungsstärke der Beleuchtungsvorrichtung (100) ausreichend genau mit der erfassten Beleuchtungsstärken-Vorgabe übereinstimmt, und
- dann, wenn dies nicht der Fall ist,
entweder erneut eine Teilmenge zu ermitteln und jeweils einen Sollwert für die maximale Einzel-Beleuchtungsstärke jeder Lichtquelle dieser ermittelten Teilmenge zu berechnen oder
festzustellen, dass die Abweichung zwischen der Beleuchtungsstärken-Vorgabe und dem aktuellen Wert der maximalen Gesamt-Beleuchtungsstärke der Beleuchtungsvorrichtung (100) und optional die Abweichung zwischen der Lichtfeld-Durchmesser-Vorgabe und dem aktuellen Wert des Lichtfeld-Durchmessers nicht weiter verringert werden kann.

5. Beleuchtungsvorrichtung (100) nach einem der vorhergehenden Ansprüche,
**dadurch gekennzeichnet, dass**
das Steuergerät (10) dazu ausgestaltet ist, nach der Detektion eines Abschattungs-Ereignisses mindestens eine Lichtquelle der Lichtquellen-Menge zu detektieren, die vollständig oder wenigstens teilweise abgeschattet ist, bevorzugt jede abgeschattete Lichtquelle, und
das Steuergerät (10) weiterhin dazu ausgestaltet ist, bei der Suche nach einer zur Abschattungs-Kompensation geeigneten Lichtquelle
- unter Verwendung von erfassten oder erfassbaren Informationen über die Lichtquellen der Lichtquellen-Menge
nach mindestens einer Lichtquelle zu suchen, die bezüglich der oder mindestens einer abgeschatteten Lichtquelle mindestens ein vorgegebenes Kompensierungs-Kriterium erfüllt,
- dann, wenn mindestens eine das oder mindestens ein Kompensierungs-Kriterium erfüllende Lichtquelle gefunden ist,
zu prüfen, welche der gefundenen Lichtquellen jeweils zur Abschattungs-Kompensation geeignet sind, und
- dann, wenn mindestens eine Lichtquelle, die das Kompensierungs-Kriterium erfüllt und die geeignet ist,
als Teilmenge eine solche Menge zu ermitteln und zu verwenden, die aus Lichtquellen besteht, die das oder mindestens ein Kompensierungs-Kriterium erfüllen und zur Abschattungs-Kompensation geeignet sind.

6. Beleuchtungsvorrichtung (100) nach Anspruch 5,
**dadurch gekennzeichnet, dass**
die Lichtquellen-Menge der Beleuchtungsvorrichtung (100)
- mindestens eine erste Lichtquelle und mindestens eine andere Lichtquelle, die zur ersten Lichtquelle redundant ist, und / oder
- mindestens eine zweite Lichtquelle und mindestens eine andere Lichtquelle, die zur zweiten Lichtquelle teil-redundant ist,
umfasst,
wobei eine andere Lichtquelle zur ersten Lichtquelle redundant ist, wenn
- die beiden Schnittpunkte der beiden Lichtachsen der beiden Lichtquellen mit einer beleuchteten ebenen Oberfläche (Ob) um nicht mehr als eine vorgegebene Toleranz voneinander beabstandet sind und
- die beiden Lichtfelder, welche diese beiden Lichtquellen erzielen, um jeweils nicht mehr als eine vorgegebene Toleranz voneinander abweichen,
wobei eine andere Lichtquelle zur zweiten Lichtquelle teil-redundant ist, wenn
- die beiden Schnittpunkte der beiden Lichtachsen der beiden Lichtquellen mit einer beleuchteten ebenen Oberfläche (Ob) um nicht mehr als eine vorgegebene Toleranz voneinander beabstandet sind oder
- die beiden Lichtfelder, welche diese beiden Lichtquellen erzielen, um jeweils nicht mehr als eine vorgegebene Toleranz voneinander abweichen oder
- der Schnittpunkt der Lichtachse der einen Lichtquelle in einem dₓ-Kreis um den Schnittpunkt der Lichtachse der anderen Lichtquelle liegt, wobei x ein vorgegebener Prozentsatz ist,
die erfassten oder erfassbaren Informationen über die Lichtquellen für jede Lichtquelle der Lichtquellen-Menge die Information umfassen, welche andere Lichtquelle oder welche anderen Lichtquellen der Lichtquellen-Menge zur Lichtquelle redundant und / oder welche teil-redundant sind,
wobei das Steuergerät (10) dazu ausgestaltet ist, ist, als das oder ein Kompensierungs-Kriterium das Kriterium zu verwenden, dass eine weitere Lichtquelle zu der oder einer abgeschatteten Lichtquelle redundant und / oder teil-redundant ist.

7. Beleuchtungsvorrichtung (100) nach Anspruch 6,
**dadurch gekennzeichnet, dass**
die Beleuchtungsvorrichtung
- mindestens eine erste Lichtquelle,
- mindestens eine zweite Lichtquelle,
- mindestens eine erste andere Lichtquelle, die zur ersten Lichtquelle redundant ist, und
- mindestens eine zweite andere Lichtquelle, die zur zweiten Lichtquelle teil-redundant und bevorzugt nicht zur ersten Lichtquelle redundant ist,
umfasst,
wobei das Steuergerät (10) dazu ausgestaltet ist, eine erste Vergrößerungs-Abfolge durchzuführen,
wobei die erste Vergrößerungs-Abfolge die Schritte umfasst,
- für jede abgeschattete Lichtquelle nach jeweils mindestens einer zur Abschattungs-Kompensation geeigneten anderen Lichtquelle zu suchen, die zur abgeschatteten Lichtquelle redundant und / oder teil-redundant ist und zusätzlich mindestens ein vorgegebenes Zusatz-Kriterium erfüllt, und
- dann, wenn mindestens eine redundante geeignete Lichtquelle gefunden ist, den tatsächlichen Wert der maximalen Einzel-Beleuchtungsstärke der oder mindestens einer redundanten geeigneten Lichtquelle zu vergrößern,
wobei das Steuergerät (10) dazu ausgestaltet ist,
- zu prüfen, ob aufgrund der Vergrößerung, die während der ersten Vergrößerungs-Abfolge durchgeführt wurde,
der Wert der tatsächlichen maximale Gesamt-Beleuchtungsstärke der Beleuchtungsvorrichtung (100) ausreichend genau mit der erfassten Beleuchtungsstärken-Vorgabe übereinstimmt, übereinstimmt,
optional zusätzlich der Wert des Lichtfeld-Durchmessers ausreichend genau mit der erfassten Lichtfeld-Durchmesser-Vorgabe, und
- dann, wenn dies nicht der Fall ist, mindestens eine zweite Vergrößerungs-Abfolge durchzuführen,
wobei die oder mindestens eine zweite Vergrößerungs-Abfolge die Schritte umfasst,
- für jede abgeschattete Lichtquelle nach jeweils einer zur Abschattungs-Kompensation geeigneten anderen Lichtquelle zu suchen,
die zur abgeschatteten Lichtquelle redundant und / oder teil-redundant ist und das oder mindestens ein vorgegebenes Zusatz-Kriterium nicht erfüllt, und
- dann, wenn mindestens eine teil-redundante geeignete Lichtquelle gefunden ist, den tatsächlichen Wert der maximalen Einzel-Beleuchtungsstärke der oder mindestens einer teil-redundanten und nicht redundanten geeigneten Lichtquelle zu vergrößern,

8. Beleuchtungsvorrichtung (100) nach Anspruch 7,
**dadurch gekennzeichnet, dass**
das oder ein bei der ersten Vergrößerungs-Abfolge angewendete Zusatz-Kriterium erfüllt ist,
wenn die andere Lichtquelle zur abgeschatteten Lichtquelle redundant ist.

9. Beleuchtungsvorrichtung (100) nach Anspruch 7 oder Anspruch 8,
**dadurch gekennzeichnet, dass**
die Lichtquellen (a.1, a.2, ..., b.1, b.2, ...) der Lichtquellen-Menge dergestalt in mindestens zwei Lichtquellen-Gruppen (Pos.1, ..., Pos.6) unterteilt sind, dass jede Lichtquelle (a.1, a.2, ..., b.1, b.2, ...) der Lichtquellen-Menge zu genau einer Lichtquellen-Gruppe (Pos.1, ..., Pos.6) gehört, und
das oder ein bei der ersten Vergrößerungs-Abfolge angewendete Zusatz-Kriterium erfüllt ist,
wenn
- die andere Lichtquelle und die abgeschattete Lichtquelle zu derselben Lichtquellen-Gruppe (Pos.1, ..., Pos.6) gehören und / oder
- die andere Lichtquelle zu einer vorgegebenen Lichtquellen-Gruppe (Pos.1, ..., Pos.6) gehört.

10. Beleuchtungsvorrichtung (100) nach einem der Ansprüche 7 bis 9,
**dadurch gekennzeichnet, dass**
das Steuergerät (10) dazu ausgestaltet ist,
- zu prüfen, ob nach der zweiten Vergrößerungs-Abfolge die Position des Maximal-Bereichs relativ zur optischen Mittelachse (MA) trotz des Abschattungs-Ereignisses ausreichend wenig verändert ist und die aktuelle maximale Gesamt-Beleuchtungsstärke der Beleuchtungsvorrichtung (100) ausreichend genau mit der erfassten Beleuchtungsstärken-Vorgabe übereinstimmt, und
- dann, wenn dies nicht der Fall ist, ist, mindestens eine weitere zur Abschattungs-Kompensation geeignete Lichtquelle der Lichtquellen-Menge auszuwählen und zu bewirken, dass deren Einzel-Beleuchtungsstärke vergrößert wird.

11. Beleuchtungsvorrichtung (100) nach einem der Ansprüche 7 bis 10,
**dadurch gekennzeichnet, dass**
die Lichtquellen (a.1, a.2, ..., b.1, b.2, ...) der Lichtquellen-Menge dergestalt in mindestens zwei Lichtquellen-Gruppen (Pos.1, ..., Pos.6) unterteilt sind, dass jede Lichtquelle (a.1, a.2, ..., b.1, b.2, ...) der Lichtquellen-Menge zu genau einer Lichtquellen-Gruppe (Pos.1, ..., Pos.6) gehört,
wobei die Lichtquellen einer Lichtquellen-Gruppe zusammen mindestens beim Ausbleiben eines Abschattungs-Ereignisses auf der beleuchteten Oberfläche (Ob) ein Lichtfeld erzeugen,
dessen maximale Beleuchtungsstärke im Schnittpunkt (S) der optischen Mittelachse (MA) mit der beleuchteten Oberfläche (Ob) oder in einem Kreis um diesen Schnittpunkt (S) herum auftritt,
wobei die erfassbaren Informationen über die Lichtquellen für jede Lichtquelle der Lichtquellen-Menge die Information umfassen, welche Lichtquelle der Lichtquellen Menge zu welcher Lichtquellen-Gruppe gehört, und
wobei das Steuergerät (10) dazu ausgestaltet ist, als das oder ein Kompensierungs-Kriterium das Kriterium zur verwenden,
dass eine weitere Lichtquelle zu derselben Lichtquellen-Gruppe gehört wie die oder eine abgeschattete Lichtquelle.

12. Beleuchtungsvorrichtung (100) nach einem der Ansprüche 7 bis 11,
**dadurch gekennzeichnet, dass**
jede Lichtquelle der Beleuchtungsvorrichtung (100) jeweils eine korrelierte Farbtemperatur aufweist und die Lichtquellen (a.1, a.2, ..., b.1, b.2, ...) der Lichtquellen-Menge insgesamt mindestens zwei verschiedene Farbtemperaturen aufweisen,
wobei die erfassten oder erfassbaren Informationen über die Lichtquellen für jede Lichtquelle der Lichtquellen-Menge jeweils deren korrelierte Farbtemperatur umfasst und
wobei das Steuergerät (10) dazu ausgestaltet ist, als das oder ein Kompensierungs-Kriterium das Kriterium zu verwenden, dass eine weitere Lichtquelle dieselbe Farbtemperatur wie die oder eine abgeschattete Lichtquelle aufweist.

13. Beleuchtungsvorrichtung (100) nach einem der vorhergehenden Ansprüche,
**dadurch gekennzeichnet, dass**
das Steuergerät (10) dazu ausgestaltet ist, automatisch
- das Ereignis zu detektieren, dass mindestens eine Lichtquelle der Lichtquellen-Menge ausgefallen ist, und
- durch eine Abschattungs-Kompensation den Ausfall der oder jeder ausgefallenen Lichtquelle zu kompensieren

14. Beleuchtungsverfahren zur Beleuchtung einer Oberfläche (Ob)
unter Verwendung einer Beleuchtungsvorrichtung (100),
wobei die Beleuchtungsvorrichtung (100)
- eine Lichtquellen-Menge mit mehreren Lichtquellen (a.1, a.2, ..., b.1, b.2, ...),
- eine Abstandsmess-Anordnung mit mehreren voneinander beabstandeten Abstandsmessern (dm, dm.2, ..., dm.6) und
- ein signalverarbeitendes Steuergerät (10)
umfasst,
wobei die Beleuchtungsvorrichtung (100) eine optische Mittelachse (MA) aufweist,
wobei jede Lichtquelle (a.1, a.2, ..., b.1, b.2, ...) der Lichtquellen-Menge jeweils
- eine Lichtachse (Lf_{a.1}, Lf_{a.2}, ...) aufweist und
- dazu ausgestaltet ist, auf der beleuchteten Oberfläche (Ob) jeweils ein Lichtfeld zu erzeugen,
wobei jede Lichtquelle (a.1, a.2, ..., b.1, b.2, ...) jeweils eine maximal mögliche Einzel-Beleuchtungsstärke des erzeugten Lichtfelds aufweist,
wobei die aktuelle maximale Einzel-Beleuchtungsstärke einer Lichtquelle (a.1, a.2, ..., b.1, b.2, ...) der Lichtquellen-Menge unabhängig von der aktuellen Einzel-Beleuchtungsstärke jeder anderen Lichtquelle auf einen Wert einstellbar ist, der zwischen Null und der maximal möglichen Einzel-Beleuchtungsstärke dieser Lichtquelle (a.1, a.2, ..., b.1, b.2, ...) liegt,
wobei jeder Abstandsmesser (dm, dm.2, ..., dm.6) der Abstandsmess-Anordnung jeweils eine zur beleuchteten Oberfläche (Ob) zeigende Messrichtung aufweist und
wobei das Verfahren die Schritte umfasst, dass
das Steuergerät (10) für jede Lichtquelle (a.1, a.2, ..., b.1, b.2, ...) der Lichtquellen-Menge jeweils vorgegebene Informationen erfasst,
wobei die erfassten Informationen die Informationen umfassen,
- welche maximal mögliche Einzel-Beleuchtungsstärke diese Lichtquelle (a.1, a.2, ..., b.1, b.2, ...) zu erzeugen vermag und
- wie die Lichtachse (Lf_{a.1}, Lf_{a.2}, ...) dieser Lichtquelle (a.1, a.2, ..., b.1, b.2, ...) relativ zur optischen Mittelachse (MA) der Beleuchtungsvorrichtung (100) positioniert und / oder orientiert ist,
das Steuergerät (10) eine Beleuchtungsstärken-Vorgabe erfasst, wobei die Beleuchtungsstärken-Vorgabe einen Sollwert für die maximale Gesamt-Beleuchtungsstärke vorgibt, welche die Beleuchtungsvorrichtung (100) erzeugen soll,
das Steuergerät (10) mindestens einmal, bevorzugt wiederholt, die jeweilige aktuelle Einzel-Beleuchtungsstärke jeder Lichtquelle (a.1, a.2, ..., b. 1, b.2, ...) der Lichtquellen-Menge auf einen Wert einstellt,
die Beleuchtungsvorrichtung (100) auf der beleuchteten Oberfläche (Ob) in einem Maximal-Bereich eine maximale Gesamt-Beleuchtungsstärke erzielt,
jeder Abstandsmesser (dm, dm.2, ..., dm.6) der Abstandsmess-Anordnung mindestens einmal, bevorzugt wiederholt, ein Maß für den jeweiligen Abstand zwischen sich und einem Licht streuenden Objekt (Ob, AO) misst und
das Steuergerät (10) mindestens einmal, bevorzugt wiederholt,
- prüft, ob ein Abschattungs-Ereignis aufgetreten ist,
das ist ein Ereignis, dass sich mindestens ein Objekt (AO) zwischen der Beleuchtungsvorrichtung (100) und der beleuchteten Oberfläche (Ob) befindet, und
- für die Prüfung ein Signal der Abstandsmess-Anordnung verwendet,
wobei das Steuergerät (10) nach der Detektion eines Abschattungs-Ereignisses die weiteren Schritte auslöst, dass
das Steuergerät (10) vollständig oder wenigstens näherungsweise einen Abschattungs-Bereich ermittelt, das ist ein Bereich auf der beleuchteten Oberfläche (Ob), der durch das Objekt (AO) vollständig oder wenigstens teilweise abgeschattet wird,
das Steuergerät (10) abhängig von dem ermittelten Abschattungs-Bereich nach mindestens einer zur Abschattungs-Kompensation geeigneten Lichtquelle der Lichtquellen-Menge sucht,
wobei eine zur Abschattungs-Kompensation geeignete Lichtquelle eine Lichtquelle (a.1, a.2, ..., b.1, b.2, ...) ist,
- die aktuell mit einem Wert für die Einzel-Beleuchtungsstärke betrieben wird, der kleiner als die maximal mögliche Einzel-Beleuchtungsstärke dieser Lichtquelle ist,
- die überhaupt nicht oder wenigstens nicht vollständig abgeschattet ist und
- deren Lichtachse (Lf_{a.1}, Lf_{a.2}, ...) die beleuchtete Oberfläche (Ob) im ermittelten Abschattungs-Bereich schneidet, und
dann, wenn das Steuergerät (10) mindestens eine zur Abschattungs-Kompensation geeignete Lichtquelle gefunden hat,
das Steuergerät (10) die Schritte durchführt,
- eine Teilmenge mit mindestens einer geeigneten Lichtquelle zu ermitteln,
- für jede Lichtquelle der ausgewählten Teilmenge jeweils einen Sollwert für die maximale Einzel-Beleuchtungsstärke dieser Lichtquelle zu berechnen, wobei dieser Sollwert größer als der aktuelle Wert der maximalen Einzel-Beleuchtungsstärke dieser Lichtquelle ist, und
- zu bewirken, dass diese Lichtquelle den berechneten Sollwert für die maximale Einzel-Beleuchtungsstärke tatsächlich aktuell erzielt,
wobei zwei Ziele bei der Berechnung der Sollwerte für die maximalen Einzel-Beleuchtungsstärken der Lichtquellen der Teilmenge sind,
- dass die Position des Maximal-Bereichs relativ zur optischen Mittelachse (MA) unverändert bleibt und
- der tatsächliche Wert der maximalen Gesamt-Beleuchtungsstärke, die die Beleuchtungsvorrichtung (100) aktuell erzielt, gleich der erfassten Beleuchtungsstärken-Vorgabe ist.
